# EUROPEAN PATENT SPECIFICATION

(11) **EP 2 582 791 B1**
(45) Date of publication and mention of the grant of the patent: **28.03.2018**
(21) Application number: 11796538.4
(22) Date of filing: 17.06.2011
(51) Int. Cl.: A61L 27/24, A61L 27/38, A61L 27/60, C12N 5/071, A61F 2/10, A61K 35/36

(54) **HAIR FOLLICLE NEOGENESIS**
NEOGENESE VON HAARFOLLIKELN
NÉOGENÈSE DE FOLLICULES PILEUX

(30) Priority: 18.06.2010 US 344258 P
(43) Date of publication of application: 24.04.2013
(73) Proprietor: The Henry M. Jackson Foundation for the Advancement of Military Medicine, Inc., Rockville, MD 20852 (US)
(72) Inventor: THANGAPAZHAM, Rajesh, Rockville, MD 20851 (US); DARLING, Thomas, N., Rockville, MD 20850 (US); LI, Shaowei, Potomac, MD 20854 (US)
(74) Representative: Dörries, Hans Ulrich
(86) International application number: PCT/US2011/040937
(87) International publication number: WO 2011/160055

(56) References cited:
- WO-A1-00/29553
- WO-A1-91/16010
- WO-A2-03/055990
- WO-A2-2006/097701
- US-A1- 2002 120 950
- US-A1- 2005 214 344
- CRISTIANE H. SQUARIZE ET AL: "Accelerated Wound Healing by mTOR Activation in Genetically Defined Mouse Models", PLOS ONE, vol. 5, no. 5, 1 May 2010 (2010-05-01), page e10643, XP055088225, ISSN: 1932-6203, DOI: 10.1371/journal.pone.0010643
- O'CALLAGHAN ET AL: "Tuberous sclerosis complex", 20080111, vol. 18, no. 1, 11 January 2008 (2008-01-11), pages 30-36, XP022412956,
- ANTHONY D. METCALFE ET AL: "Tissue engineering of replacement skin: the crossroads of biomaterials, wound healing, embryonic development, stem cells and regeneration", JOURNAL OF THE ROYAL SOCIETY INTERFACE, vol. 4, no. 14, 5 December 2006 (2006-12-05), pages 413-437, XP055088237, ISSN: 1742-5689, DOI: 10.1098/rsif.2006.0179
- RITSUKO EHAMA ET AL: "Hair Follicle Regeneration Using Grafted Rodent and Human Cells", JOURNAL OF INVESTIGATIVE DERMATOLOGY, vol. 127, no. 9, 12 April 2007 (2007-04-12), pages 2106-2115, XP055088241, ISSN: 0022-202X, DOI: 10.1038/sj.jid.5700823
- SHAOWEI LI ET AL: "Human TSC2-null fibroblast-like cells induce hair follicle neogenesis and hamartoma morphogenesis", NATURE COMMUNICATIONS, vol. 2, 8 March 2011 (2011-03-08), page 235, XP055088144, DOI: 10.1038/ncomms1236
- LI ET AL.: 'Human TSC2-null fibroblast-like cells induce hair follicle neogenesis and hamartoma morphogenesis' NATURE COMMUNICATIONS vol. 2, 08 March 2011,

## Description

### STATEMENT OF GOVERNMENT INTEREST

This invention was made in part with support from the U.S. Government. Accordingly, the Government has certain rights in this invention.

### FIELD OF THE INVENTION

The present invention relates to skin substitutes capable of inducing fully-formed human hair follicles. The present invention also relates to methods and compositions for inducing neogenesis of human hair follicles. In some embodiments, the present invention can be used for the treatment of full- or partial-thickness skin loss, wounds, burns, scars, and full- or partial-hair loss.

### BACKGROUND OF THE INVENTION

Studies of hair and skin continue to be at the forefront of regenerative medicine. Skin substitutes were among the earliest products to be developed using principles of tissue engineering, and the success of these ventures is evident in the clinical use of several commercially available products. In addition, hair restoration is one of the fastest growing areas of cosmetic therapies for both men and women.

The current clinical "gold standard" for treating major skin injuries involves the use of split-thickness skin autografts, which involves transplanting the epidermis with a portion of the dermis from one location on a patient to another. In cases where there is insufficient donor skin to cover the wounds, however, skin substitutes may be used. Skin substitutes available today have varied compositions, but generally comprise a nonliving collagen matrix and different combinations of keratinocytes and fibroblasts. For example, APLIGRAF® (Organogenesis, Inc., Canton, MA), which is reported to be the most clinically successful composite skin substitute currently available, is composed of allogeneic neonatal fibroblasts in bovine type I collagen overlaid with allogeneic neonatal keratinocytes.

However, currently available skin substitutes cannot perform all the functions of normal skin. For example, hair follicle neogenesis is not observed using any currently available skin substitute, which limits their use in patients. Hair follicles and their associated sebaceous glands are important for appearance, skin hydration, barrier formation, and protection against pathogens. In addition, hair follicles store epidermal stem cells that may be called upon during wound healing. Thus, skin with hair follicles heals more rapidly than skin without hair follicles. In addition, any stem cells that might exist in skin lacking hair follicles are located in superficial layers of the epidermis, making the cells susceptible to loss through minor trauma and damage through ultraviolet light. Thus, treatments that involve neogenesis of normal hair follicles would find much wider application for restoring normal skin function and appearance.

A variety of medicinal and surgical options are available to restore normal hair growth in skin. Medicinal options typically involve the use of pharmaceutical agents, such as minoxidil or finasteride, to stimulate existing quiescent hair follicles. Surgical options typically involve harvesting tissue comprising hair follicles from one part of the body and transplanting the follicles to a site where hair has been lost. However, neither approach triggers hair follicle neogenesis. Instead, both of these approaches require existing hair follicles in the skin, which limits their applicability in certain patients.

Current methods of hair follicle neogenesis involve grafting tissue containing inductive dermal papilla (DP) or dermal sheath (DS) cells from a donor into the epidermis of a recipient. For example, DS and DP cells have been isolated from mice and rats, combined with epithelial cells, and grafted or injected into animals to induce hair follicle neogenesis. However, human cells have proven much less robust than rodent cells in inducing hair follicles, and complicated experimental systems have been devised to facilitate human hair follicle formation. These systems include chamber assays, subcutaneous injection assays, and sandwich and flap-graft assays. (*See* Ohyama et al., Exp. Dermatol., 19:89-99 (2010).) Hair follicle-like structures were formed using chimeric constructs of murine mesenchymal cells and human epidermal cells in a chamber assay (see Ehama et al., J. Invest. Dermatol., 127:2106-15 (2007)), and DP cells from adult human scalp were shown to induce hair follicles in mouse embryonic epidermis using a flap-graft model (*see* Qiao et al., Regen. Med., 4:667-76 (2009)). A recent comparison of the chamber assay and sandwich assay showed equal utility for screening the hair follicle-inducing capabilities of human DP cells. (*See* Inoue et al., Cells Tissues Organs, 190:120-10 (2009).)

However, although these systems are highly valuable as investigative tools, they lack clinical utility because the hair follicles produced by these methods are not fully human constructs (but instead are chimeric rodent/human constructs), are not completely developed, contain hair shafts in the wrong anatomical location, do not exhibit long-term graft survival and normal hair follicle cycling, and/or do not form hair follicles that contain sebaceous glands. In addition, hair follicles produced by such methods tend to grow in variable and uncontrollable directions, resulting in unnatural looking hair. Thus, the follicles produced by such methods are not useful for human hair follicle neogenesis in skin lacking hair follicles. Moreover, although the capacity of human foreskin keratinocytes to form hair follicles has been reported (Ehama et al.), it has not been possible to generate human hair follicles using cultured adult human fibroblasts, even when dermal papilla/dermal sheath cells (which are specialized for hair induction) were used.

Thus, a need exists for methods and compositions capable of generating morphologically-correct, fully-developed human hair follicles. Such methods and compositions would be useful for treating conditions such as full- or partial-thickness skin loss, wounds, burns, scars, and hair loss. The present invention fills these needs by providing cellular compositions capable of hair neogenesis and regeneration.

### SUMMARY OF THE INVENTION

The invention is defined by the claims. The invention provides a skin substitute comprising epithelial cells and modified mesenchymal cells, wherein the modified mesenchymal cells have decreased TSC1/TSC2 function, increased mTORC1 function, and/or decreased mTORC2 function compared to wild type mesenchymal cells.

The invention also provides the defined skin substitute for use in transplanting to a patient cells capable of inducing human hair follicles.

The invention further provides modified mesenchymal cells for use in treating a condition requiring growth of hair follicles, eccrine glands, and/or sebaceous glands, wherein the modified mesenchymal cells and their use are both further defined, as is set out in the claims.

In paragraphs [012] to [024] the use of the term "embodiment", insofar as it applies to a method of medical treatment, is to be interpreted as referring to "embodiment disclosed herein".

Also disclosed herein is a method for transplanting cells capable of inducing human hair follicles. In one embodiment disclosed herein, the method comprises subdermally or intradermally delivering to a patient modified mesenchymal cells having decreased TSC1/TSC2 function, increased mTORG1 function, and/or decreased mTORC2 function compared to wild-type mesenchymal cells. In another embodiment, the method further comprises delivering epithelial cells to the patient. In yet another embodiment, the method comprises grafting to a patient a skin substitute of the invention.

In one embodiment, the patient has partial-thickness skin loss, full-thickness skin loss, a wound, a burn, a scar, or hair loss. In another embodiment, the method induces formation of eccrine glands. In yet another embodiment, the method induces formation of sebaceous glands.

In one embodiment, the TSC1/TSC2 function has been decreased, either directly, or indirectly because the function of at least one negative regulator of TSC1/TSC2 has been increased (such as by upregulating a protein that inhibits TSC1/TSC2 function), and/or the function of at least one positive regulator of TSC1/TSC2 has been decreased (such as by downregulating a protein that stimulates TSC1/TSC2 function) compared to wild type mesenchymal cells. In another embodiment, the function of mTORC1 has been increased, the function of mTORC2 has been decreased, or both, through mimetics of decreased TSC1/TSC2 function.

In one embodiment, the function of TSC1/TSC2 is decreased, the function of mTORC1 is increased and/or the function of mTORC2 is decreased by downregulating TSC1 or TSC2; upregulating an inhibitory protein that inhibits TSC1/TSC2 function or acts as a mimetic of decreased TSC1/TSC2 function; or downregulating a stimulatory protein that stimulates TSC1/TSC2 function or acts as a mimetic of increased TSC1/TSC2 function. In one embodiment, the stimulatory protein is chosen from at least one of TSC1, TSC2, CYLD, LKB1, FLCN, MEN1, NF1, PTEN, PRAS40, 4E-BP1, GSK3, and Deptor. In another embodiment, the inhibitory protein is chosen from at least one of Ras, Raf, Mek, Erk, Rsk1, PI3K, Akt1, Akt2, Akt3, Rheb, mTOR, Raptor, Rictor, mLST8, S6K1, ribosomal protein S6, SKAR, SREBP1, eIF4e, IKKbeta, Myc, Runx1, and p27. In one embodiment, TSC2 is downregulated. In another embodiment, FLCN is downregulated. In yet another embodiment, both TSC2 and FLCN are downregulated.

In one embodiment, the modified mesenchymal cells are from benign adnexal tumors. In yet another embodiment, the modified mesenchymal cells are from angiofibromas, fibrofolliculomas, fibrous papules, forehead plaques, hair follicule nevi, infundibulomas, isthmicomas, perifollicular fibromas, sebaceous nevi, organoid nevi, syringomas, shagreen patches, trichodiscomas, trichoepitheliomas, trichoblastomas, trichilemmomas, trichoadenomas, poromas, or ungual fibromas. In yet another embodiment, the modified mesenchymal cells are from tumors associated with Birt-Hogg-Dube syndrome, Brooke-Spiegler syndrome, Cowden syndrome, multiple endocrine neoplasia type 1, neurofibromatosis, or tuberous sclerosis complex.

In one embodiment, the modified mesenchymal cells are wild-type mesenchymal cells that are modified to decrease TSC1/TSC2 function, increase mTORC1 function, and/or decrease mTORC2 function. In another embodiment, the wild type mesenchymal cells are dermal fibroblasts, dermal papilla cells, dermal sheath cells, induced pluripotent stem cells, or mesenchymal stem cells. In yet another embodiment, the wild type mesenchymal cells are dermal fibroblasts.

In one embodiment, the wild-type mesenchymal cells are modified to decrease TSC1/TSC2 function by increasing function of at least one negative regulator of TSC1/TSC2 and/or decreasing function of at least one positive regulator of TSC1/TSC2. In another embodiment, in the wild-type mesenchymal cells, the function of mTORC1 has been increased, the function of mTORC2 has been decreased, or both, through mimetics of decreased TSC1/TSC2 function.

In one embodiment, in the wild-type mesenchymal cells, the TSC1/TSC2 function is decreased, the function of mTORC1 is increased and/or the function of mTORC2 is decreased by downregulating TSC1 or TSC2; upregulating an inhibitory protein that inhibits TSC1/TSC2 function or acts as a mimetic of decreased TSC1/TSC2 function; or downregulating a stimulatory protein that stimulates TSC1/TSC2 function or acts as a mimetic of increased TSC1/TSC2 function.

In one embodiment, the modification involves silencing a gene encoding a positive regulator of TSC1/TSC2 or a mimetic of increased TSC1/TSC2 function. In one embodiment, the gene silencing may be accomplished by treating the wild type mesenchymal cells with siRNA, shRNA, or RNAi directed against the target gene. In yet another embodiment, the modification involves overexpressing a gene encoding a negative regulator of TSC1/TSC2 or a mimetic of decreased TSC1/TSC2 function. In one embodiment, the overexpression may be accomplished by stably transfecting the wild type mesenchymal cells with an expression vector comprising the gene under control of a constitutively-active promoter.

In another embodiment, the mesenchymal cells may be transfected with growth factor genes or treated with growth factors that decrease TSC1/TSC2 function or act as a mimetic of decreased TSC1/TSC2 function, such as insulin, EGF, HGF, IGF and KGF. In yet another embodiment, the mesenchymal cells may be treated with recombinant proteins that decrease TSC1/TSC2 function or act as a mimetic of decreased TSC1/TSC2 function. Cells may be treated with drugs that increase expression of transcription factors such as thiaolidinediones (including rosiglitazone and pioglitazone), which are agonists of the transcription factor PPARG (peroxisome proliferator-activated receptor gamma).

In one embodiment, the mesenchymal cells are incorporated into a microsphere. In another embodiment, the microsphere is formed by mixing about 30,000 cells each of neonatal foreskin fibroblasts and neonatal foreskin keratinocytes in a 1:1 mixture of dermal papilla medium and keratinocyte serum free medium, and incubating the clusters for about four weeks. In another embodiment, the mesenchymal cells are provided with a matrix. In yet another embodiment, the matrix is a collagen matrix or a ground substance matrix. In yet another embodiment, the matrix is a type I collagen matrix. In yet another embodiment, the matrix is a rat type I collagen matrix, a bovine type I collagen matrix, or a human type I collagen matrix.

In one embodiment, the epithelial cells comprise one or more epithelial cells from different sources. In another embodiment, the epithelial cells are keratinocytes or keratinocyte-like cells. In yet another embodiment, the keratinocytes are neonatal foreskin keratinocytes.

In one embodiment, the mesenchymal cells and epithelial cells are derived from the same donor. In another embodiment, the donor is the patient. In yet another embodiment, the mesenchymal cells and epithelial cells are derived from different donors. In yet another embodiment, the donor of either the mesenchymal or epithelial cells is the patient.

### BRIEF DESCRIPTION OF THE DRAWINGS

The accompanying drawings, which are incorporated in and constitute a part of this specification, illustrate several non-limiting embodiments of the invention and together with the description, serve to explain the principles of the invention.

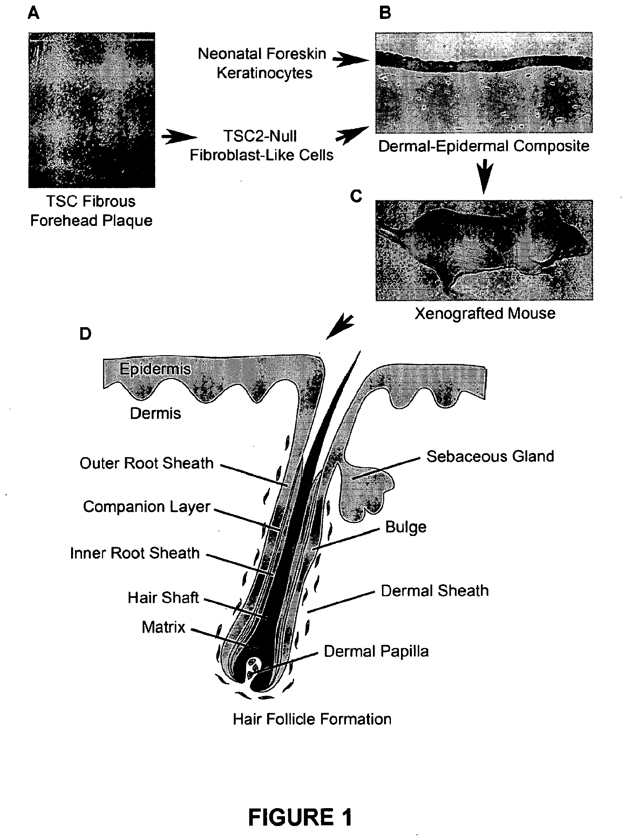

Figure 1. A) is photograph of a fibrous forehead plaque on a patient. B) is a histological stained section of a skin substitute of the invention comprising fibroblast-like cells from a TSC2-null skin hamartoma overlaid by neonatal foreskin keratinocytes. C) is a photograph of a mouse grafted with a skin substitute of the invention comprising fibroblast-like cells from a TSC2-null skin hamartoma overlaid by neonatal foreskin keratinocytes. D) is a schematic of the hair follicle microanatomy that develops in a skin substitute of the invention comprising fibroblast-like cells from a TSC2-null skin hamartoma overlaid by neonatal foreskin keratinocytes.

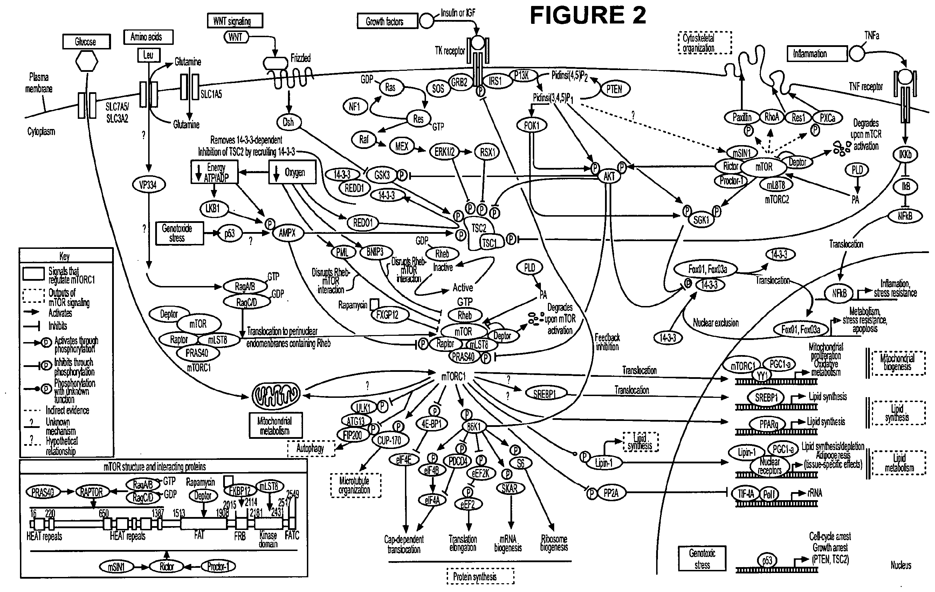

Figure 2 is a schematic representation of the mTOR (mammalian target of rapamycin) network.

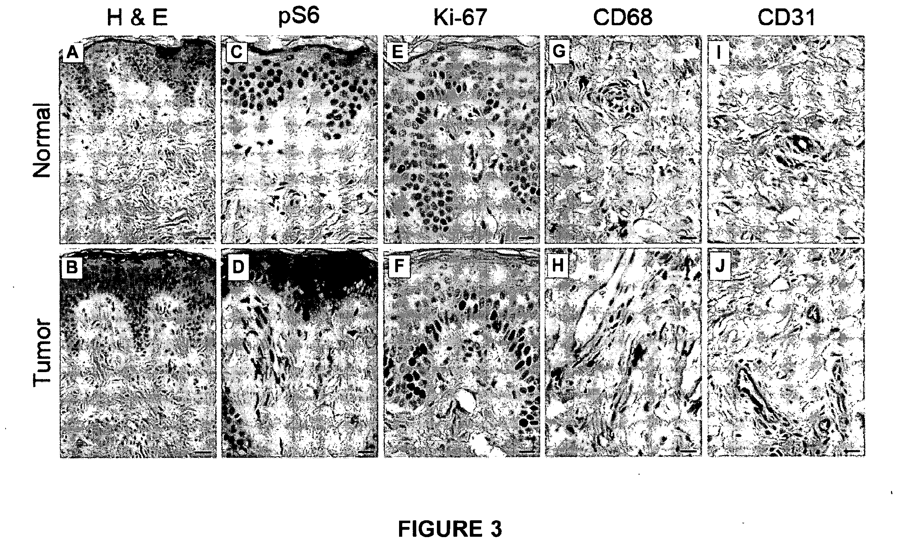

Figure 3. A) is a histological stained section of normal skin from a TSC patient. B) is a histological stained section of a forehead plaque from a TSC patient. C) is an immunohistological anti-phospho-S6 stained section of normal skin from a TSC patient. D) is an immunohistological anti-phospho-S6 stained section of a forehead plaque from a TSC patient. E) is an immunohistological anti-Ki-67 stained section of normal skin from a TSC patient. F) is an immunohistological anti-Ki-67 stained section of a forehead plaque from a TSC patient. G) is an immunohistological anti-CD68 stained section of normal skin from a TSC patient. H) is an immunohistological anti-CD68 stained section of a forehead plaque from a TSC patient. I) is an immunohistological anti-CD31 stained section of normal skin from a TSC patient. J) is an immunohistological anti-CD31 stained section of a forehead plaque from a TSC patient.

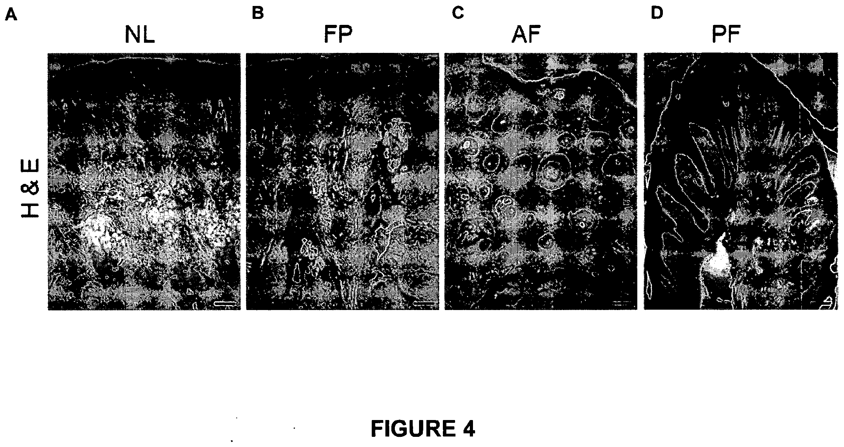

Figure 4. Histological stains of normal skin (A) and TSC skin hamartomas from a forehead plaque (B), an angiofibroma (C), and a periungual fibroma (D).

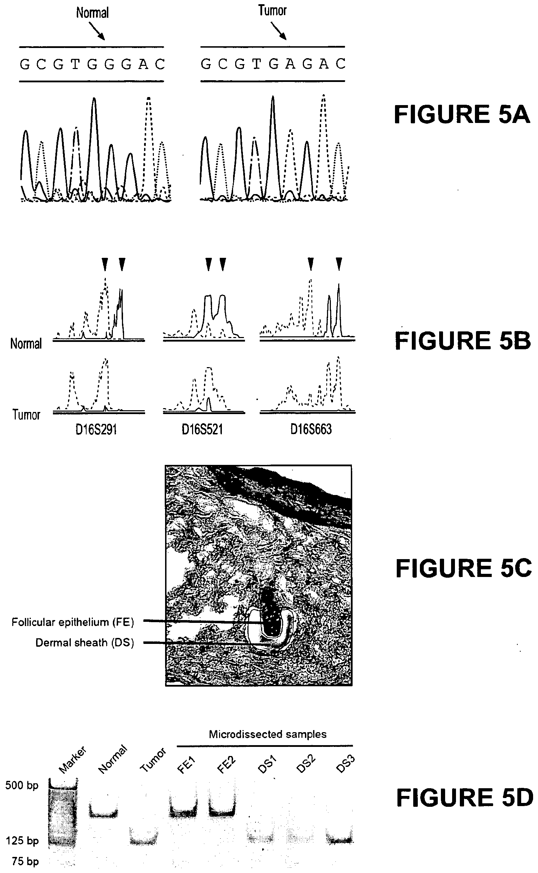

Figure 5. A) is a sequence analysis of part of the *TSC2* gene in TSC2-null fibroblast-like cells. B) is an analysis of the microsatellite nucleotide repeat polymorphisms (D16S291, D16S521, and D16S663) from normal cells and TSC2-null fibroblast-like cells. Primer pairs for D16S291 are forward primer, 5'GCAGCCTCAGTTGTGTTTCGTAATC3' and reverse primer, 5'AGTGCTGGGATTACAGGCATGAACC3'. Primer pairs for D16S521 are forward primer, 5' AGCGAGACTCCGTCTAAAAA3' and reverse primer, 5' TACAACCAAAATGCCTTACG 3'. Primer pairs for D16S663 are forward primer, 5' GTCTTTGTAGGAATGAAATCAT 3' and reverse primer, 5'ATTGCAGCAAGACTCCATCT 3'. C) is a microdissection of a tumor xenograft dermal sheath encompassing the lower portion of the follicular epithelium. D) is a 10% TBE gel showing the results of a *Bsm*A1 restriction enzyme digestion of Exon 10 of *TSC2* amplified from TSC normal fibroblasts ("normal"), TSC2-null fibroblast-like cells ("tumor"), and laser microdissected follicular epithelium ("FE") or dermal sheath ("DS") regions of tumor xenografts.

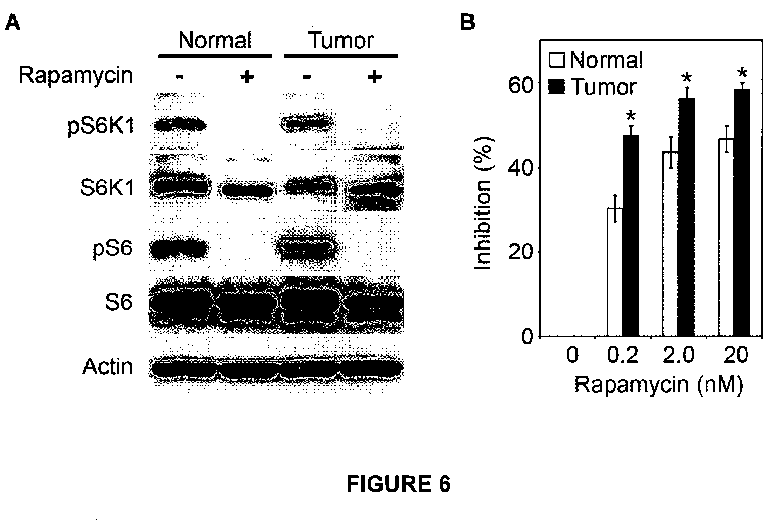

Figure 6. A) is a western blot of cell lysates from normal cells and forehead plaques from TSC patients. The blot shows levels of expression of actin (control), phosphorylated S6K1 (pS6K1), unphosphorylated S6K1, phosphorylated ribosomal protein S6 (pS6), and S6 as a function of rapamycin treatment. B) is a bar graph presenting cell proliferation data for normal cells and forehead plaque cells from TSC patients as a function of rapamycin treatment.

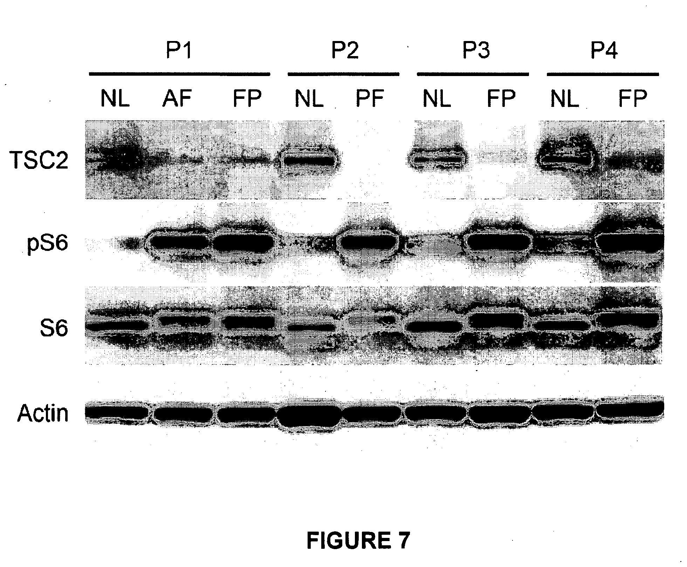

Figure 7 is a western blot of cell lysates from normal cells, forehead plaques, and angiofibromas from TSC patients. The blot shows levels of expression for actin (control), TSC2 (tuberin), phosphorylated S6 (pS6), and unphosphorylated S6.

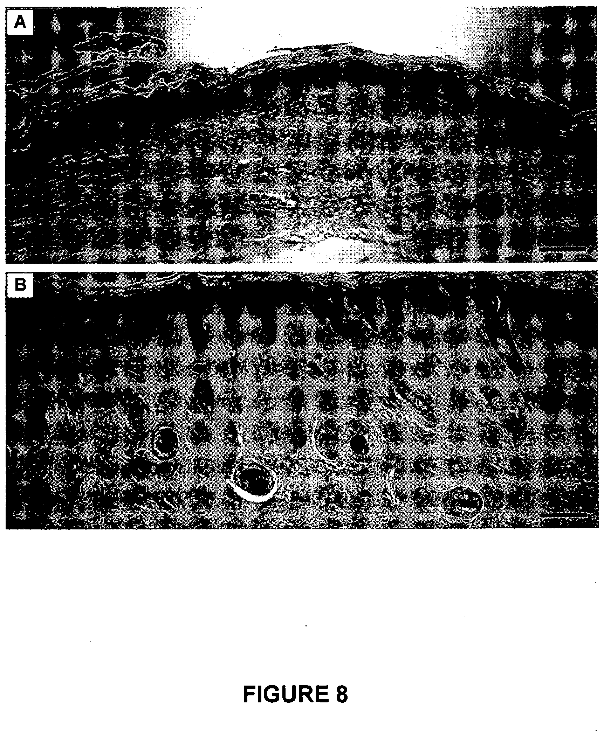

Figure 8. A) is a histological stain of grafts made with TSC normal fibroblasts and human neonatal foreskin keratinocytes. B) is a histological stain of grafts comprising TSC2-null cells from TSC skin hamartomas and human neonatal foreskin keratinocytes.

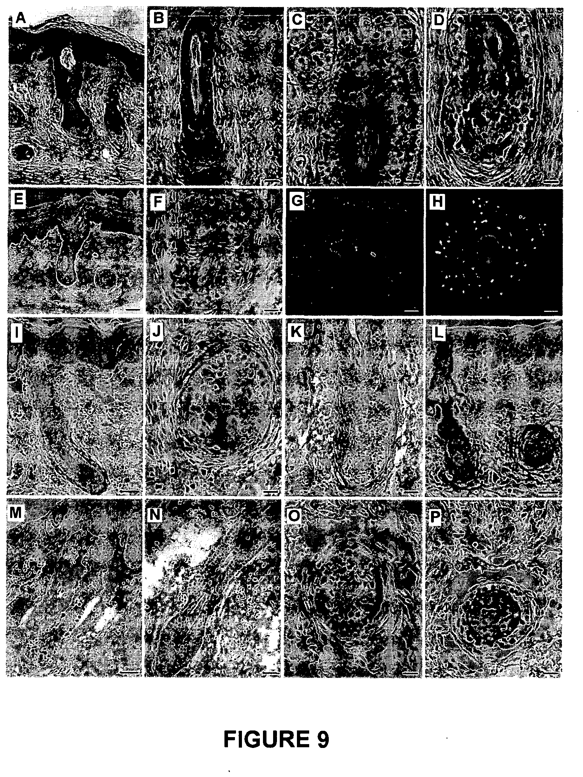

Figure 9. A) is a histological stain of an anagen hair follicle with a sebaceous gland and hair shaft from a skin substitute comprising TSC2-null cells from TSC skin hamartomas 17 weeks after grafting (scale bar = 130 µm). B) is a histological stain of a longitudinal section of a hair follicle with human hair shaft from a skin substitute comprising TSC2-null cells from TSC skin hamartomas 17 weeks after grafting (scale bar = 130 µm). C) is a histological stain of a cross section of an anagen hair follicle showing an outer root sheath, inner root sheath, hair shaft, and sebaceous gland from a skin substitute comprising TSC2-null cells from TSC skin hamartomas 17 weeks after grafting (scale bar = 35 µm). D) is a histological stain of a hair bulb with a dermal papilla, lower dermal sheath, matrix with mitotic figure, and inner and outer root sheath from a skin substitute comprising TSC2-null cells from TSC skin hamartomas 17 weeks after grafting (scale bar = 35 µm). E) and F) are immunohistological anti-COX IV antibody-stained epithelial cells and dermal cells from a skin substitute comprising TSC2-null cells from TSC skin hamartomas 17 weeks after grafting (scale bars: Figure 9E = 130 µm, Figure 9F = 35 µm). G) and H) are an *in situ* hybridization of epidermal cells including hair follicle epithelium from a skin substitute comprising TSC2-null cells from TSC skin hamartomas 17 weeks after grafting showing a fluorescent probe for the human Y chromosome (red) hybridized with nuclei (blue) (scale bars: Figure 9G = 130 µm, Figure 9H = 20 µm). I) and J) are immunohistological anti-nestin antibody-stained sections. Stained cells are from the dermal papilla and lower dermal sheath region of a skin substitute comprising TSC2-null cells from TSC skin hamartomas 17 weeks after grafting (scale bars: Figure 8I = 65 µm, Figure 8J = 35 µm). K) is an immunohistological anti-versican antibody-stained section. Stained cells are from the dermal papilla and lower dermal sheath region of an anagen hair follicle from a skin substitute comprising TSC2-null cells from TSC skin hamartomas 17 weeks after grafting (scale bar = 65 µm). L) is an alkaline phosphatase-stained section that shows enzyme activity from the dermal papilla and lower sheath region of the hair follicle from a skin substitute comprising TSC2-null cells from TSC skin hamartomas 17 weeks after grafting (scale bar = 65 µm). M) is an immunohistological anti-Ki-67 antibody-stained section. Stained cells are from the basal layer of the epidermis and hair follicle matrix from a skin substitute comprising TSC2-null cells from TSC skin hamartomas 17 weeks after grafting (scale bar = 65 µm). N) and O) are immunohistological anti-Keratin 15 antibody stained sections. Stained cells are the basal layer of the outer root sheath below the follicular infundibulum from a skin substitute comprising TSC2-null cells from TSC skin hamartomas 17 weeks after grafting (scale bar = 65 µm). P) is an immunohistological anti-Keratin 75 antibody-stained section. Stained cells are the hair follicle companion layer from a skin substitute comprising TSC2-null cells from TSC skin hamartomas 17 weeks after grafting (scale bar = 65 µm).

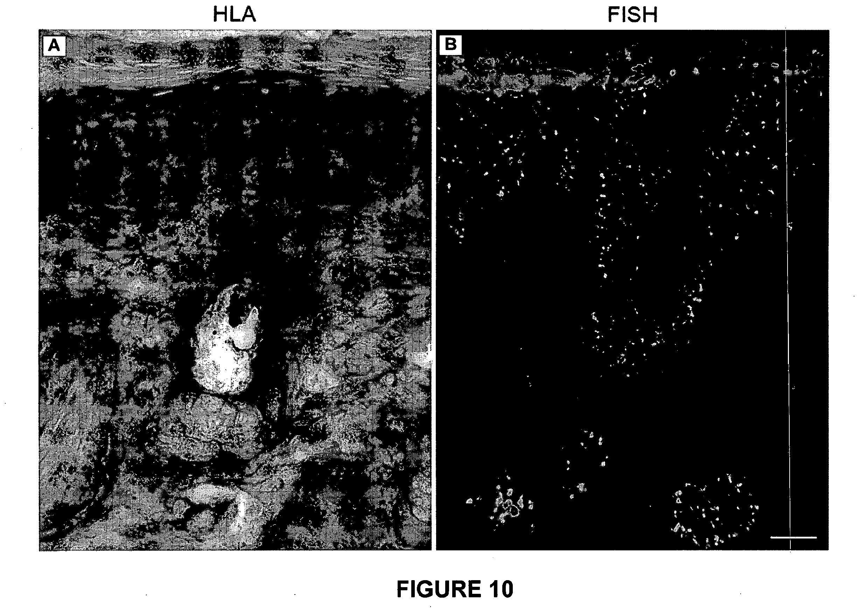

Figure 10. A) is an immunohistological anti-HLA antibody-stained section. Stained cells are the dermis, epidermis, and hair follicles of a skin substitute comprising TSC2-null fibroblast-like cells from an angiofibroma and neonatal foreskin keratinocytes (scale bar = 65 µm). B) is an *in situ* hybridization of a fluorescent probe to the human Y chromosome that hybridizes to nuclei of the epidermis and follicular epithelium when added to a frozen section of a graft comprising neonatal foreskin keratinocytes and TSC2-null fibroblast-like cells from an angiofibroma (scale bar = 65 µm).

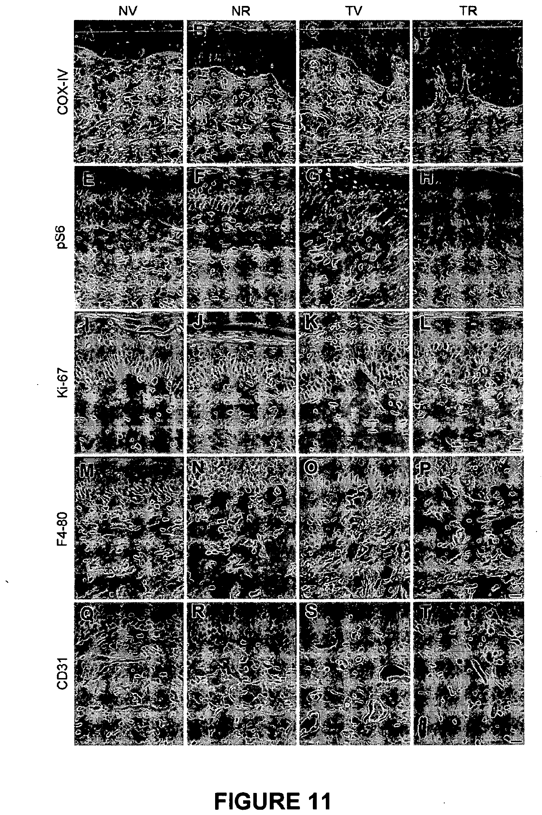

Figure 11. A) is an immunohistochemical anti-COX IV antibody-stained section of a xenograft containing normal fibroblasts from a TSC patient. The xenograft was taken from a mouse treated with vehicle (scale bar = 35 µm). B) is an immunohistochemical anti-COX IV antibody stained section of a xenograft containing normal fibroblasts from a TSC patient. The xenograft was taken from a mouse treated with rapamycin (scale bar = 35 µm). C) is an immunohistochemical anti-COX IV antibody stained section of a xenograft containing TSC2-null fibroblasts from a TSC patient. The xenograft was taken from a mouse treated with vehicle (scale bar = 35 µm). D) is an immunohistochemical anti-COX IV antibody stained section of a xenograft containing TSC2-null fibroblasts from a TSC patient. The xenograft was taken from a mouse treated with rapamycin (scale bar = 35 µm). E) is an immunohistochemical anti-pS6 antibody stained section of a xenograft containing normal fibroblasts from a TSC patient. The xenograft was taken from a mouse treated with vehicle (scale bar = 35 µm). F) is an immunohistochemical anti-pS6 antibody stained section of a xenograft containing normal fibroblasts from a TSC patient. The xenograft was taken from a mouse treated with rapamycin (scale bar = 35 µm). G) is an immunohistochemical anti-pS6 antibody stained section of a xenograft containing TSC2-null fibroblasts from a TSC patient. The xenograft was taken from a mouse treated with vehicle (scale bar = 35 µm). H) is an immunohistochemical anti-pS6 antibody stained section of a xenograft containing TSC2-null fibroblasts from a TSC patient. The xenograft was taken from a mouse treated with rapamycin (scale bar = 35 µm). I) is an immunohistochemical anti-Ki-67 antibody stained section of a xenograft containing normal fibroblasts from a TSC patient. The xenograft was taken from a mouse treated with vehicle (scale bar = 35 µm). J) is an immunohistochemical anti-Ki-67 antibody stained section of a xenograft containing normal fibroblasts from a TSC patient. The xenograft was taken from a mouse treated with rapamycin (Scale bar = 35 µm). K) is an immunohistochemical anti-Ki-67 antibody stained section of a xenograft containing TSC2-null fibroblasts from a TSC patient. The xenograft was taken from a mouse treated with vehicle (scale bar = 35 µm). L) is an immunohistochemical anti-Ki-67 antibody stained section of a xenograft containing TSC2-null fibroblasts from a TSC patient. The xenograft was taken from a mouse treated with rapamycin (scale bar = 35 µm). M) is an immunohistochemical anti-F4 80 antibody stained section of a xenograft containing normal fibroblasts from a TSC patient. The xenograft was taken from a mouse treated with vehicle (scale bar = 35 µm). N) is an immunohistochemical anti-F4 80 antibody stained section of a xenograft containing normal fibroblasts from a TSC patient. The xenograft was taken from a mouse treated with rapamycin (scale bar = 35 µm). O) is an immunohistochemical anti-F4 80 antibody stained section of a xenograft containing TSC2-null fibroblasts from a TSC patient. The xenograft was taken from a mouse treated with vehicle (scale bar = 35 µm). P) is an immunohistochemical anti-F4 80 antibody stained section of a xenograft containing TSC2-null fibroblasts from a TSC patient. The xenograft was taken from a mouse treated with rapamycin (scale bar = 35 µm). Q) is an immunohistochemical anti-CD31 antibody stained section of a xenograft containing normal fibroblasts from a TSC patient. The xenograft was taken from a mouse treated with vehicle (scale bar = 35 µm). R) is an immunohistochemical anti-CD31 antibody stained section of a xenograft containing normal fibroblasts from a TSC patient. The xenograft was taken from a mouse treated with rapamycin (scale bar = 35 µm). S) is an immunohistochemical anti-CD31 antibody stained section of a xenograft containing TSC2-null fibroblasts from a TSC patient. The xenograft was taken from a mouse treated with vehicle (scale bar = 35 µm). T) is an immunohistochemical anti-CD31 stained section of a xenograft containing TSC2-null fibroblasts from a TSC patient. The xenograft was taken from a mouse treated with rapamycin (scale bar = 35 µm).

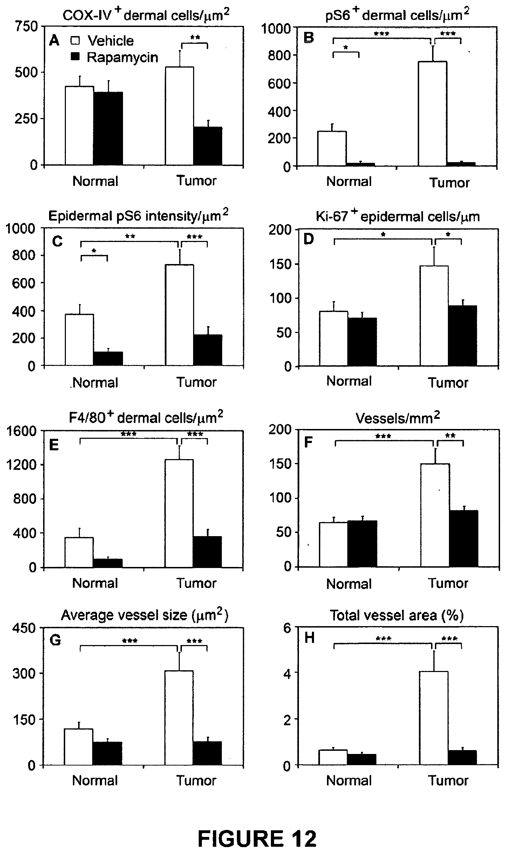

Figure 12. A) is a bar graph showing the average number of dermal cells reactive with human-specific anti-COX-IV antibody relative to the total dermal area of xenografts containing TSC2-null or normal fibroblasts from a TSC patient. The xenografts were taken mice treated with or without rapamycin, as indicated. Results are mean ± SE (** = p<0.01). B) is a bar graph showing the average number of dermal cells reactive with anti-pS6 antibody relative to the total dermal area of xenografts containing TSC2-null or normal fibroblasts from a TSC patient. The xenografts were taken from each group of mice treated with or without rapamycin, as indicated. Results are mean ± SE (** = p<0.01, *** = p<0.001). C) is a bar graph showing the average intensity of positive staining for anti-pS6 antibody in the epidermis quantified as fluorescence intensity relative to the total epidermal area of xenografts containing TSC2-null or normal fibroblasts from a TSC patient. The xenografts were taken from mice treated with or without rapamycin, as indicated. Results are mean ± SE (** = p<0.01, *** = p<0.001). D) is a bar graph showing the average numbers of non-follicular epidermal cells reactive with anti-Ki-67 antibody relative to epidermal length of xenografts containing TSC2-null or normal fibroblasts from a TSC patient. The xenografts were taken from mice treated with or without rapamycin, as indicated. Results are mean ± SE (* = p<0.05). E) is a bar graph showing the average numbers of dermal cells reactive with anti-F4/80 antibody relative to the dermal area of xenografts containing TSC2-null or normal fibroblasts from a TSC patient. The xenografts were taken from mice treated with or without rapamycin, as indicated. Results are mean ± SE (*** = p<0.001). F) is a bar graph showing the average number and area of anti-CD31 positive blood vessels expressed as the number of vessels per unit dermal area of xenografts containing TSC2-null or normal fibroblasts from a TSC patient. The xenografts were taken from mice treated with or without rapamycin, as indicated. Results are mean ± SE (** = p<0.01, *** = p<0.001). G) is a bar graph showing the average number and area of anti-CD31 positive blood vessels expressed as the average cross-sectional area of each vessel of xenografts containing TSC2-null or normal fibroblasts from a TSC patient. The xenografts were taken from mice treated with or without rapamycin, as indicated. Results are mean ± SE (*** = p<0.001). H) is a bar graph showing the average number and area of anti-CD31 positive blood vessels expressed as the ratio of vessel area to dermal area within xenografts containing TSC2-null or normal fibroblasts from a TSC patient. The xenografts were taken from mice treated with or without rapamycin, as indicated. Results are mean ± SE (*** = p<0.001).

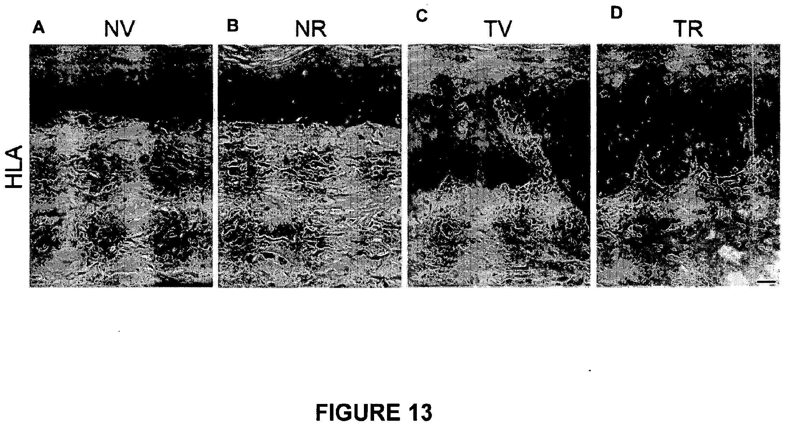

Figure 13. A) is a HLA immunochemical stained section of a xenograft containing normal fibroblasts from a TSC patient. The xenograft was taken from a mouse treated with vehicle. B) is a HLA immunochemical stained section of a xenograft containing normal fibroblasts from a TSC patient. The xenograft was taken from a mouse treated with rapamycin. C) is a HLA immunochemical stained section of a xenograft containing TSC2-null fibroblasts from a TSC patient. The xenograft was taken from a mouse treated with vehicle. D) is a HLA immunochemical stained section of a xenograft containing TSC2-null fibroblasts from a TSC patient. The xenograft was taken from a mouse treated with rapamycin.

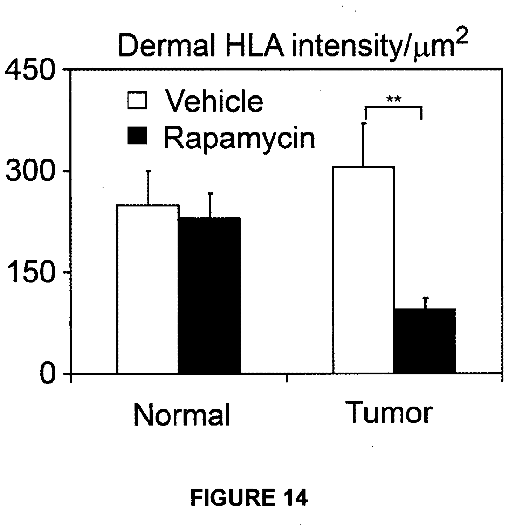

Figure 14 is a bar graph quantifying HLA-positivity as a parameter of fluorescence intensity in the dermis of xenografts relative to the dermal area for xenografts comprising normal fibroblasts or TSC2-null fibroblasts from a TSC patient. The xenografts were taken from mice treated with or without rapamycin, as indicated (** = p<0.01).

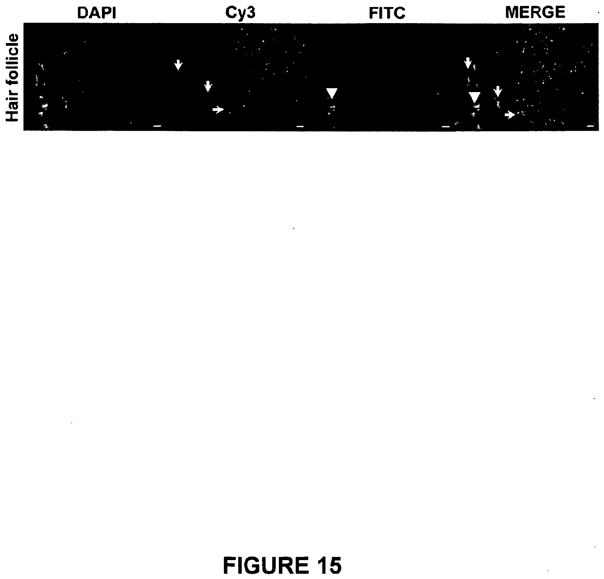

Figure 15 is an *in situ* hybridization of a skin substitute comprising TSC2-null cells from TSC skin hamartomas 17 weeks after grafting. DAPI stain shows nuclei of cells in blue. Cy3 is a human-specific centromeric probe in red with Cy3-labeled cells of the lower dermal sheath marked with a horizontal arrow and adjacent Cy3-lableled dermal fibroblasts marked with vertical arrows. FITC is a mouse-specific centromeric probe in green with FITC-labeled endothelial cells marked with an arrowhead. Merge is the combination of these three images.

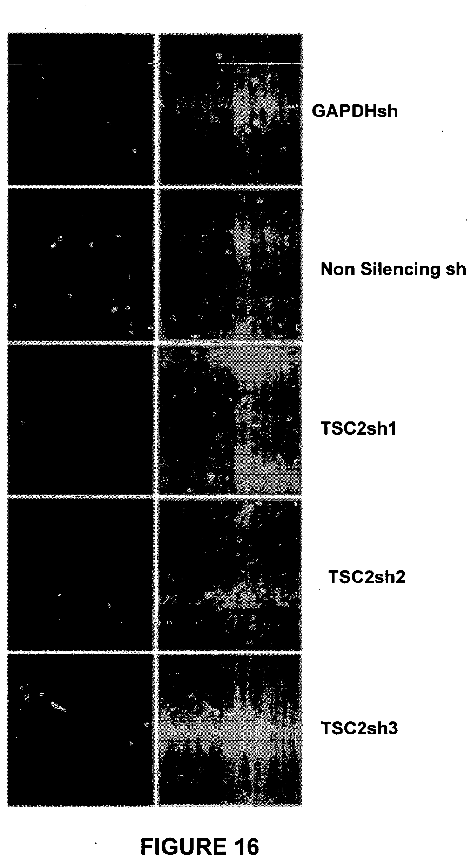

Figure 16 shows fluorescence microscopic images (panels on the left) and phase-contrast microscopic images (panels on the right) of neonatal foreskin fibroblasts stably transduced with shRNA vectors. Cells were transduced with GAPDHsh control, non-target shRNA control (Non Silencing sh), or TSC2 knock-down vector (TSC2sh1, TSC2sh2, TSC2sh3).

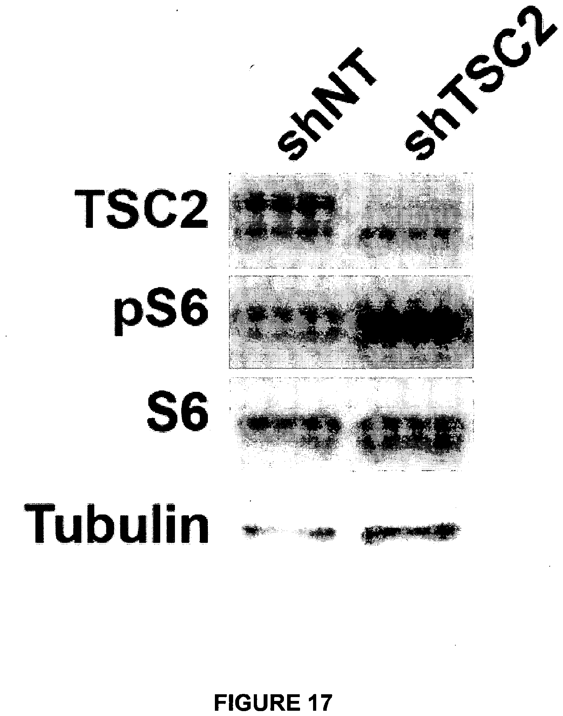

Figure 17 is a western blot of cell lysates from foreskin fibroblast cells transduced with non-target shRNA control (shNT), or TSC2-knockdown vector (shTSC2) showing levels of TSC2 (TSC2, top band), phosphorylated ribosomal protein S6 (pS6), total ribosomal protein S6 (S6), and tubulin loading control.

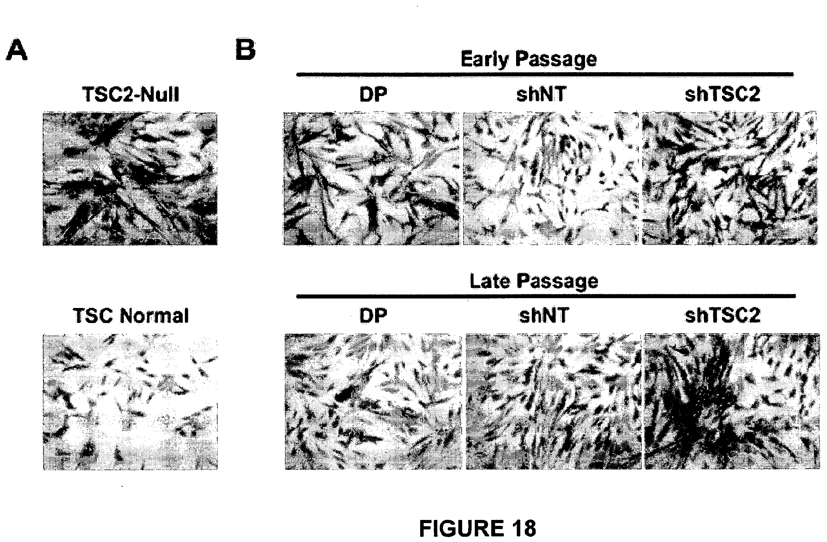

Figure 18. A) is a stain of alkaline phosphatase activity (AP) staining (blue) in monolayer cultures of TSC2-null cells from a TSC patient skin tumor at passage 4 (P=4) and normal fibroblasts (P=4). B) is a stain of AP activity (blue) in monolayer cultures of normal human dermal papilla cells and modified mesenchymal cells (neonatal foreskin fibroblasts transduced with shTSC2) at early passage (P=4) and late passage (P=7). DP = normal human dermal papilla cells; shNT = foreskin fibroblasts transduced with control shRNA; shTSC2 = foreskin fibroblasts transduced with TSC2 shRNA.

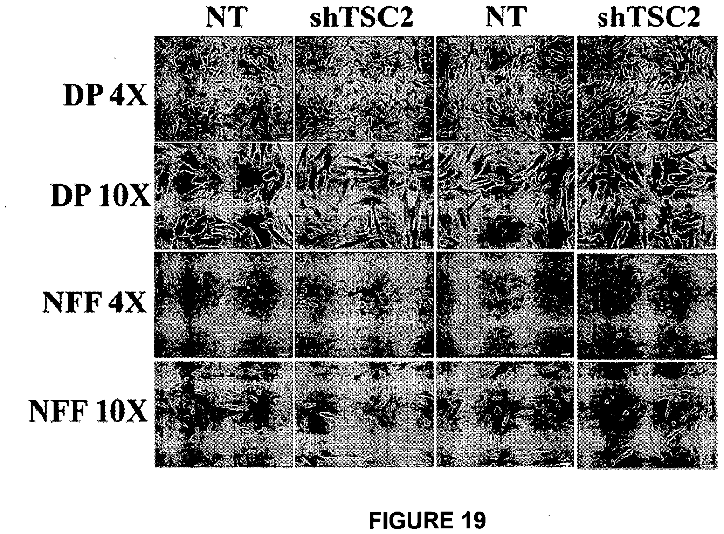

Figure 19 is a stain of AP activity (blue) in monolayer cultures of dermal papilla cells (DP) or neonatal foreskin fibroblasts (NFF) stably transduced with lentiviral particles with knockdown of TSC2 (shTSC2) compared to non-template control (NT). NT = control shRNA; shTSC2 = TSC2 shRNA; DP = dermal papilla cells; NFF = neonatal foreskin fibroblast cells; 4X and 10X indicate magnification levels.

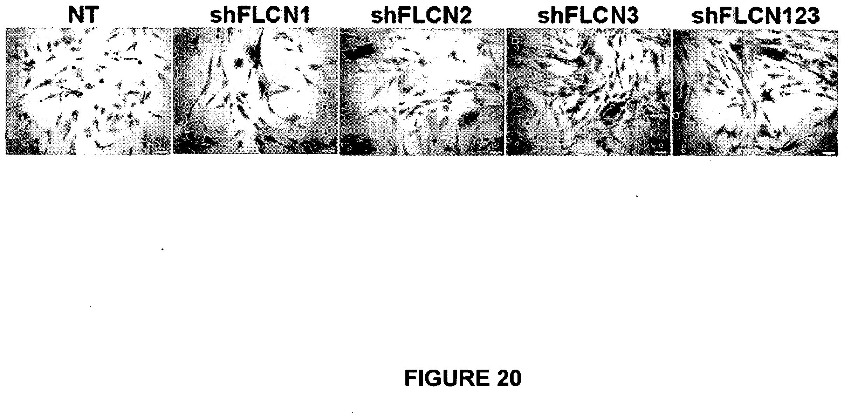

Figure 20 is a stain of AP activity (blue) in monolayer cultures (P=3) of neonatal foreskin fibroblasts stably transduced with lentiviral particles with either a non-targeting sequence (NT) as control and three different sequences (FLCN1, FLCN2, and FLCN3) targeted to silence the FLCN gene. NT = control shRNA; shFLCN1, shFLCN2, and scFLCN3 = three different FLCN shRNA; shFLCN123 = combination of all three FLCN silencing sequences.

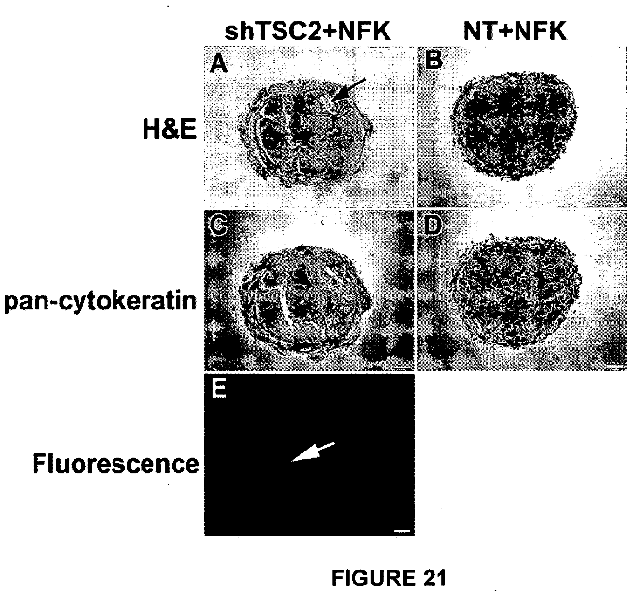

Figure 21 shows the results of an *in vitro* hanging drop culture hair follicle assay A) is a cluster composed of neonatal foreskin fibroblasts transduced with TSC2 knockdown vector (shTSC2) and neonatal foreskin keratinocytes (NFK) in a 1:1 ratio, stained with hematoxylin and eosin (H&E). B) is a cluster composed of neonatal foreskin fibroblasts transduced with non-targeting control vector (NT) and NFK in a 1:1 ratio, stained with H&E. C) is a cluster composed of neonatal foreskin fibroblasts transduced with TSC2 knockdown vector (shTSC2) and NFK in a 1:1 ratio, stained with anti-pan cytokeratin antibody. D) is a cluster composed of neonatal foreskin fibroblasts transduced with non-targeting control vector (NT) and NFK in a 1:1 ratio, stained with anti-pan-cytokeratin antibody. E) is a cluster composed of neonatal foreskin fibroblasts transduced with TSC2 knockdown vector (shTSC2) and NFK in a 1:1 ratio, stained with anti-pan-cytokeratin antibody and visualized using fluorescence microscopy. Autofluorescence of a hair-fiber-like structure is marked with an arrow.

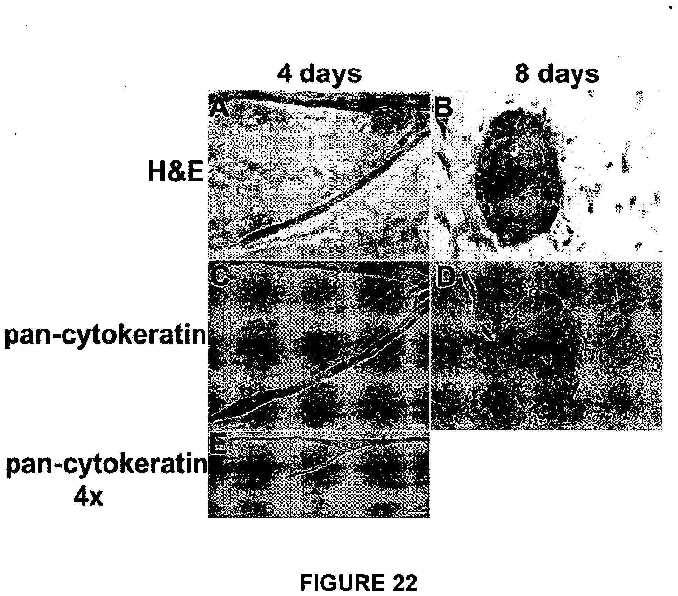

Figure 22 shows *in vitro* hair formation of hair follicle-like structures in dermal-epidermal composites (skin substitutes). A) shows hematoxylin and eosin (H&E) analysis of the skin equivalents four days after bringing the composite to the air-liquid interface. It is composed of neonatal foreskin fibroblasts transduced and stably expressing TSC2 knockdown vector (1 mg/mL of rat tail collagen type 1 in 10% FBS/DMEM, and overlaid with 1 x 10⁶ keratinocytes. Image was taken with a 10X objective. B) is a skin substitute stained with H&E eight days after bringing the composite to the air-liquid interface. Image was taken with a 10X objective. C) is a skin substitute stained with anti-pan-cytokeratin antibody four days after bringing the composite to the air-liquid interface. Image was taken with a 10X objective. D) is a skin substitute stained with anti-pan-cytokeratin antibody eight days after bringing the composite to the air-liquid interface. Image was taken with a 10X objective. E) is a skin substitute stained with anti-pan-cytokeratin antibody four days after bringing the composite to the air-liquid interface. Image was taken with a 4X objective.

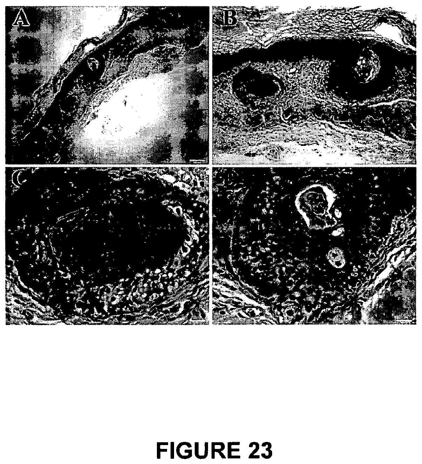

Figure 23 shows hematoxylin and eosin stained sections of dermal-epidermal composites, sampled 10 weeks after grafting. Composites were composed of dermal papilla cells transduced with shRNA to TSC2, type I collagen, and normal neonatal foreskin keratinocytes. A) is a low-power (40x) image of the graft containing multiple hair follicles. B) is a medium power (100X) image of a hair follicle infundibulum (right side) and proximal (suprabulbar) hair follicle (left side). C) is a higher-power (400x) image of the proximal hair follicle, showing outer and inner root sheaths and pigmented hair shaft cortex. D) is a high-power image of a hair follicle infundibulum with early sebaceous gland development and pigmented hair fiber.

### DETAILED DESCRIPTION OF THE INVENTION

### I. DEFINITIONS

As used herein, the singular forms "a" "an" and "the" are intended to include the plural forms as well, unless the context clearly indicates otherwise. Furthermore, to the extent that the terms "including," "includes," "having," "has," "with," or variants thereof are used in either the detailed description and/or the claims, such terms are intended to be inclusive in a manner similar to the term "comprising."

As used herein, the terms "about" and "approximately" mean within an acceptable error range for the particular value as determined by one of ordinary skill in the art, which will depend in part on how the value is measured or determined, *i.e*., the limitations of the measurement system. For example, "about" can mean from 1 to 1.5 standard deviation(s) or from 1 to 2 standard deviations, per the practice in the art. Alternatively, "about" can mean a range of up to and including 20%, 10%, 5%, or 1% of a given value. Alternatively, particularly with respect to biological systems or processes, the term can mean up to and including an order of magnitude, up to and including 5-fold, and up to and including 2-fold, of a value. Where particular values are described in the application and claims, unless otherwise stated the term "about" meaning within an acceptable error range for the particular value should be assumed.

As used herein, the term "apocrine gland" refers to glands in the skin that have a coiled, tubular excretory portion with widely dilated lumen, lined by cuboidal epithelial cells with eosinophilic cytoplasm and apical snouts, and an outer discontinuous layer of myoepithelial cells resting on a prominent basement membrane.

As used herein, the term "composition" refers to a mixture that contains a therapeutically active component(s) and a carrier, such as a pharmaceutically acceptable carrier or excipient that is conventional in the art and which is suitable for administration to a subject for therapeutic purposes. The therapeutically active component may include the mesenchymal cells of the invention. In other embodiments, term "composition" refers to the skin substitutes of the invention, which are described in further detail below. The compositions of the invention may further comprise a matrix, which is defined below.

As used herein, the term "dermal papilla" refers to the follicular dermal papilla, *i.e*., the mesenchymal cell condensation at the base of the hair follicle.

As used herein, the term "decreased TSC1/TSC2 function" refers to a downregulation in the level, function, activity and/or effect of the TSC1/TSC2 complex and can be produced by downregulation of either TSC1 or TSC2, or by changes in function of upstream regulators of TSC1 or TSC2.

Additionally, the function of the TSC1/TSC2 complex can be mimicked by other proteins that affect its downstream targets, mTORC1 and mTORC2. Therefore upregulating a protein that acts as a mimetic of decreased TSC1/TSC2 function and/or downregulating a protein that acts as a mimetic of increased TSC1/TSC2 function will lead to increased mTORC1 function, decreased mTORC2 function, or both, and such changes compared to wild-type mesenchymal cells are desired in one embodiment of this invention.

Decreased TSC1/TSC2 function, increased mTORC1 function, and/or decreased mTORC2 function can occur by: (1) downregulating of TSC1 and/or TSC2; (2) upregulating an inhibitory protein that inhibits TSC1/TSC2 function or acts as a mimetic of decreased TSC1/TSC2 function; or (3) downregulating a stimulatory protein that stimulates TSC1/TSC2 function or acts as a mimetic of increased TSC1/TSC2 function. Mimetics of increased or decreased function include other molecules that affect mTORC1 or mTORC2 activity that are downstream of TSC1/TSC2 in the mTOR signaling network.

One embodiment of this invention includes decreasing function of at least one stimulatory protein (*e.g*., LKB1, NF1, PTEN, CYLD, FLCN, PRAS40, 4E-BP1, GSK3, and MEN1) and/or increasing function of at least one inhibitory protein (*e.g*., Ras, Raf, Mek, Erk, Rsk1, Pl3K, Akt1, Akt2, Akt3, Rheb, mTOR, Raptor, Rictor, mLST8, S6K1, ribosomal protein S6, SKAR, SREBP1, elF4e, IKKbeta, Myc, Runx1, and p27). All of these modifications are encompassed by the term "decreased TSC1/TSC2 function, increased mTORC1 function, and/or decreased mTORC2 function."

As used herein, the term "eccrine glands" refers to sweat glands in the skin. Eccrine glands consist of two anatomical portions: (1) the secretory coil, located in the deep dermis at the junction with the subcutaneous tissue and composed of clear pyramidal cells and dark-stained cells, surrounded by a single outer discontinuous layer of myoepithelial cells resting on a well-defined basement membrane; and (2) the excretory part composed of a straight intradermal portion and an intraepidermal spiral portion (acrosyringium), and a double layer of small cuboidal cells with no underlying myoepithelial layer.

As used herein, the term "endothelial cell" refers to the specialized cells that line the inner walls of blood vessels.

As used herein, the term "epidermal cell" refers to cells derived from the epidermis of the skin. Epidermal cells are one type of epithelial cells. Examples of epidermal cells include, but are not limited to keratinocytes, melanocytes, Langerhans cells, and Merkel cells.

As used herein, the term "epithelial cell" refers to cells that line the outside (skin), mucous membranes, and the inside cavities and lumina of the body. In particular embodiments, the term "epithelial cell" refers to stratified squamous epithelial cells. Most epithelial cells exhibit an apical-basal polarization of cellular components. Epithelial cells are typically classified by shape and by their specialization. For example, squamous epithelial cells are thin and have an irregular flattened shape mainly defined by the nucleus. Squamous cells typically line surfaces of body cavities, such as the esophagus. Specialized squamous epithelia line blood vessels (endothelial cells) and the heart (mesothelial cells). Cuboidal epithelial cells are cube-shaped and usually have their nucleus in the center. Cuboidal epithelial cells are typically found in secretive or absorptive tissue, e.g., kidney tubules, glandular ducts, and the pancreatic exocrine gland. Columnar epithelial cells are longer than they are wide and the elongated nucleus is usually near the base of the cell. These cells also have tiny projections, called microvilli, which increase the surface area of the cells. Columnar epithelial cells typically form the lining of the stomach and intestines, as well as sensory organs.

As used herein, the term "gene" refers to nucleic acid coding sequences necessary for the production of a polypeptide or precursor. The polypeptide can be encoded by a full length coding sequence or by any portion of the coding sequence so long as the desired functional properties (e.g., enzymatic activity, ligand binding, signal transduction, etc.) of the polypeptide are retained. The term also encompasses the coding region of a structural gene and the sequences located adjacent to the coding region on both the 5' and 3' ends for a distance of about 1 kb on either end, such that the term "gene" corresponds to the length of the full-length mRNA. The sequences that are located 5' of the coding region and which are present on the mRNA are referred to as 5' untranslated sequences. The sequences that are located 3' or downstream of the coding region and that are present on the mRNA are referred to as 3' untranslated sequences. These sequences are referred to as "flanking" sequences or regions. The 5' flanking region may contain regulatory sequences such as promoters and enhancers that control or influence the transcription of the gene. The 3' flanking region may contain sequences that direct the termination of transcription, post-transcriptional cleavage and polyadenylation.

The term "gene" encompasses both cDNA and genomic forms of a gene. A genomic form or clone of a gene may contain the coding region interrupted with non-coding sequences termed "introns" or "intervening regions" or "intervening sequences." Introns are segments of a gene that are transcribed into nuclear RNA (hnRNA), and may contain regulatory elements such as enhancers. Introns are removed or "spliced out" from the nuclear or primary transcript and, therefore, are absent in the messenger RNA (mRNA) transcript. The mRNA functions during translation to specify the sequence or order of amino acids in a nascent polypeptide.

As used herein, the terms "gene knockdown" and "gene silencing" refer to any technique by which the expression of one or more genes is reduced. Such gene knockdown techniques include, but are not limited to, mutating genomic DNA to reduce or eliminate gene transcription or translation, creation of targeted double-strand breaks using a zinc finger nuclease, and treating genomic DNA with a reagent, such as an antisense oligonucleotide. As used herein, the term "antisense nucleotide" refers to a nucleic acid molecule that is substantially identical (or substantially complementary) to a portion of a target RNA or DNA. Antisense nucleotides include, but are not limited to, short complementary double stranded RNA oligonucleotides (dsRNA) such as small interfering RNA (siRNA), short interfering hairpin RNA (shRNA), micro RNA (miRNA), or interfering RNA (RNAi). As used herein, the term "amount sufficient to inhibit expression" refers to a concentration or amount of the antisense oligonucleotide that is sufficient to reduce levels or stability of mRNA or protein produced from a target gene. As used herein, "inhibiting expression" refers to the absence or observable decrease in the level of protein and/or mRNA product from a target gene. The inhibition may be either transient or permanent, depending on the application. The reduction may be at least a 10%, 20%, 30%, 40%, 50%, 60%, 70%, 80%, 90%, 95%, 99%, or 100% reduction.

The invention encompasses variations in antisense oligonucleotides. As used herein, and taking into consideration the substitution of uracil for thymine when comparing RNA and DNA sequences, the terms "substantially identical" and "substantially complementary" as applied to antisense oligonucleotides means that the nucleotide sequence of one strand of the antisense oligonucleotide is at least about 80%, 85%, 90%, 95%, 96%, 97%, 98%, 99%, or 100% identical to 20 or more contiguous nucleotides of the target gene, and which hybridize to the target gene under stringent conditions (defined below). However, 100% sequence identity between the antisense oligonucleotide and the target gene is not required to practice the present invention; the invention can tolerate sequence variations that might be expected due to gene manipulation or synthesis, genetic mutation, strain polymorphism, or evolutionary divergence. Thus the antisense oligonucleotides may comprise a mismatch with the target gene of at least 1, 2, or more nucleotides. The term "20 or more nucleotides" means a portion of the target gene that is at least about 20, 21, 22, 23, 24, 25, 26, 27, 28, 29, 30, 40, 50, 75, 100, 150, 200, 300, 400, 500, 1000, 1500, or 2000 consecutive bases, up to the coding sequence of the target gene or the full length of the target gene.

The terms "gene knockdown" and "gene silencing" also refer to any technique by which the function of a protein expressed by one or more genes is reduced. Such gene knockdown techniques include, but are not limited to, mutating genomic DNA to reduce or eliminate protein function, and treating cells with a reagent that interferes with or inhibits protein function. As used herein, the term "amount sufficient to inhibit function" refers to a concentration or amount of reagent that is sufficient to reduce levels of protein function in a cell. As used herein, "inhibiting function" refers to the absence or observable decrease in the level of protein function in a cell.

As used herein, the term "hair follicle" refers to a tubular infolding of the epidermis from which a hair may grow. A hair follicle may contain a hair shaft in the correct anatomical location, exhibit long-term graft survival, normal hair follicle cycling, and sebaceous glands.

As used herein, the term "hair regeneration" refers to the stimulation of existing quiescent hair follicles to enter the anagen phase of hair growth. The term also refers to stimulation of hair formation from hair follicle remnants or components of hair follicles (e.g., implantation of microdissected dermal papilla and follicular epithelium, or hair growth after plucking), rather than starting with intact quiescent hair follicles.

As used herein, the term "hair neogenesis" refers to the stimulation of *de novo* hair follicle growth where no hair follicle previously existed in skin with no preexisting hair follicles, or in skin with fewer than the desired number of hair follicles.

As used herein, the term "keratinocyte" refers to epithelial cells in the epidermis of the skin (including cells in the follicular epithelium) that undergo cell division and stratification from basal cells in contact with the epidermal basement membrane into squamous cells. Keratinocytes express keratin.

As used herein, the term "keratinocyte-like cell" refers to cells that express keratin and have the ability to form a stratified squamous epithelium or follicular epithelium. Keratinocyte-like cells may be derived from skin cells or other organs such as bone marrow or trachea, or from cells with stem-cell features (including embryonic stem cells) or that induce pluripotent stem cells.

As used herein, the terms "matrix" and "ground substance" refer to any natural or synthetic extracellular matrix-like composition capable of forming a hydrated gel-like cellular support. Cells may be deposited within or on matrices and ground substances. Matrices and ground substances may comprise one or more fibrous proteins having both structural and adhesive functions. Such proteins include, but are not limited to elastin, fibronectin, laminin, and collagens I, II, III, IV, V, VI, VII, VIII, IX X, XI, and XII. Alternatively, or in addition, matrices and ground substances may comprise proteoglycan molecules comprising polysaccharide chains covalently linked to proteins. Such proteoglycans include, but are not limited to, hyaluronan-, heparin sulfate-, chondroitin-, keratin sulfate-, and dermatin sulfate-linked proteins.

As used herein, the term "mesenchymal cell" refers to multipotent cells with the capacity or potential capacity to induce hair follicle formation similar to cells of the dermal papilla and connective tissue sheath from hair follicles. Mesenchymal cells are usually considered mesodermal connective tissue cells that express vimentin, but cells with the desired attributes may also be neural crest derived. Mesenchymal cells may be isolated from one or more of the following sources: patient skin or mucosa for autologous cells; donor skin or mucosa for allogeneic cells; normal skin or mucosa; skin with an adnexal tumor; and other tissues (e.g. fat, bone marrow). Mesenchymal cells include, but are not limited to, fibroblasts, dermal papilla cells, dermal sheath cells, onychofibroblasts (fibroblasts from nail unit), dental pulp cells, periodontal ligament cells, neural crest cells, adnexal tumor cells, induced pluripotent stem cells, and mesenchymal stem cells from bone marrow, umbilical cord blood, umbilical cord, fat, and other organs.

As used herein, the terms "morphologically correct" and "fully developed" refers to hair follicles that have a normal configuration with an epithelial filament coming out of the distal end of the follicle and dermal papilla sitting at the base of the follicle. The follicles also have cells proliferating at the base of the follicle, and have concentric layers of outer and inner root sheath, cuticle and cortex. The follicles exhibit normal differentiation of the outer root sheath, and have hair shafts and sebaceous glands. The hairs go through normal cycles, and contain an epithelial stem cell component.

As used herein, the term "mutation" refers to any change in the coding sequence of a gene. Mutations include missense mutations, frameshift mutations (*i.e.,* insertions and deletions), splice site mutations, nonsense mutations (*i.e.,* premature termination codons), and deletion of the gene itself.

As used herein, the term "nucleotide sequence encoding a gene product," or variations thereof such as "gene encoding," means a nucleic acid sequence comprising the coding region of a gene or the nucleic acid sequence that encodes a gene product (*i.e.,* a polypeptide). The coding region may be present in cDNA, genomic DNA, or RNA form. When present in a DNA form, the nucleotide sequence may be single-stranded (*i.e.,* the sense strand) or double-stranded. Suitable control elements such as enhancers/promoters, splice junctions, polyadenylation signals, etc. may be placed in close proximity to the coding region of the gene if needed to permit proper initiation of transcription and/or correct processing of the primary RNA transcript. Alternatively, the coding region may contain endogenous enhancers/promoters, splice junctions, intervening sequences, polyadenylation signals, etc. or a combination of both endogenous and exogenous control elements.

As used herein, the term "pharmaceutically acceptable carrier" refers to a non-toxic solid, semisolid, or liquid filler, diluents, encapsulating material, formulation auxiliary, or excipient of any conventional type. A pharmaceutically acceptable carrier is non-toxic to recipients at the dosages and concentrations employed, and is compatible with other ingredients of the formulation.

As used herein, the terms "polynucleotide," "nucleotide," "nucleic acid," "nucleic acid molecule," "nucleic acid sequence," "polynucleotide sequence," and "nucleotide sequence," are used interchangeably to refer to polymeric forms of nucleotides of any length. The polynucleotides can comprise deoxyribonucleotides, ribonucleotides, deoxyribonucleosides, ribonucleosides, substituted and alpha-anomeric forms thereof, peptide nucleic acids (PNA), locked nucleic acids (LNA), phosphorothioate, methylphosphonate, and/or naturally occurring and non-naturally occurring analogs or derivatives thereof. The terms also include naturally and non-naturally occurring variants of wild type sequences. Variants may include insertions, additions, deletions, or substitutions that are at least about 80%, 85%, 90%, 95%, 96%, 97%, 98%, or 99% identical to a wild type sequence. In some embodiments, less than 10% of the amino acid residues are altered in the protein products of the variants. In other embodiments, less than 5% of the amino acid residues are altered. Insertional and deletional variants include polynucleotides that are 90%, 95%, 105%, or 110% of the length of the corresponding wild type sequence.

As used herein, the term "sebaceous gland" refers to hair follicle-dependent glands that originate as a budding of sebaceous glands primordium. Sebaceous glands consist of multiple lobules of rounded cells (sebocytes), filled with lipid-containing vacuoles, and rimmed by a single layer of small, dark germinative cells. The lobules converge on a short duct, which empties the lipid content of degenerated sebocytes into the hair follicle.

As used herein, the terms "skin substitute," "skin equivalent," "dermal-epidermal composite," and "skin graft" refer to any product used for the purpose of damaged skin replacement, fully or partially, temporarily or permanently, and possessing some similarities with human skin, both anatomically or functionally. In the context of the present invention, these terms refer to an *in vitro* derived culture of mesenchymal cells having an upregulated TSC1/TSC2 network in combination with an *in vitro* derived culture of epithelial cells. Skin substitutes include, but are not limited to, bioengineered skin equivalents, tissue-engineered skin, tissue-engineered skin constructs, biological skin substitutes, bioengineered skin substitutes, skin substitute bioconstructs, living skin replacements, dermal-epidermal composites and bioengineered alternative tissue.

As used herein, the term "stringent hybridization conditions" refers to conditions under which a polynucleotide will hybridize to a target sequence, but to a minimal number of other sequences. In general, stringent hybridization conditions include low concentrations (<0.15M) of salts with inorganic cations such as Na²⁺ or K²⁺ (*i.e.,* low ionic strength), temperatures higher than 20°C-25°C below the Tm of the hybridized complex (*i.e.,* the temperature at which 50% of the oligonucleotides complementary to the target sequence hybridize to the target sequence at equilibrium), and the presence of denaturants such as formamide, dimethylformamide, dimethyl sulfoxide, or the detergent sodium dodecyl sulfate (SDS). The Tm value may be calculated by the equation: Tm=81.5+0.41 (% G+C), when a nucleic acid is in aqueous solution at 1 M NaCl (*See e.g.,* Anderson and Young, Quantitative Filter Hybridization, in Nucleic Acid Hybridization [1985]). Exemplary stringent hybridization conditions include, but are not limited to, hybridization in 4 x sodium chloride/sodium citrate (SSC) at about 65-70°C, hybridization in 4 x SSC plus 50% formamide at about 42-50°C followed by one or more washes in 1 x SSC at about 65-70°C, hybridization in 400 mM NaCl, 40 mM PIPES pH 6.4, 1 mM EDTA, 60°C hybridization for 12-16 hours followed by washing at 60°C with 0.1% SDS and 0.1% SSC for about 15-60 minutes, and hybridization at 42°C in a solution consisting of 5 x SSPE (43.8 g/l NaCl, 6.9 g/l NaH₂PO₄H₂ and 1.85 g/l EDTA, pH adjusted to 7.4 with NaOH), 0.5% SDS, 5 x Denhardt's reagent and 100 µg/ml denatured salmon sperm DNA followed by washing in a solution comprising 0.1 x SSPE, 1.0% SDS at 42°C.

As used herein, the term "transfection" refers to the introduction of foreign DNA into eukaryotic cells. Transfection may be accomplished by a variety of means known to the art including calcium phosphate-DNA co-precipitation, DEAE-dextran-mediated transfection, polybrene-mediated transfection, electroporation, microinjection, liposome fusion, lipofection, protoplast fusion, retroviral infection, and biolistics. Transfection also includes the introduction of foreign DNA accomplished by replication-incompetent retroviral vectors, which may also be referred to as transduction or viral transduction. The term "stable transfection" or "stably transfected" refers to the introduction and integration of foreign DNA into the genome of the transfected cell. The term "transient transfection" or "transiently transfected" refers to the introduction of foreign DNA into a cell in which the foreign DNA fails to integrate into the genome of the transfected cell. The foreign DNA persists in the nucleus of the transiently transfected cell for several days. During this time the foreign DNA is subject to the regulatory controls that govern the expression of endogenous genes in the chromosomes.

As used herein, the term "treatment," refers to any administration or application of remedies for a condition in a mammal, including a human, to obtain a desired pharmacological and/or physiological effect. Treatments include inhibiting the condition, arresting its development, or relieving the condition, for example, by restoring or repairing a lost, missing, or defective function, or stimulating an inefficient process.

As used herein, the term "trichogenic" refers to the ability of a cell to induce a hair follicle and/or to promote hair follicle morphogenesis, *i.e*., folliculogenesis.

As used herein, the terms "wild type" and "normal" refer to a gene, gene product, or signaling network that has the characteristics of that gene, gene product, or signaling network in a naturally occurring source. A wild-type gene, gene product, or signaling network is that which is most frequently observed in a population and is thus arbitrarily designed the "normal" or "wild-type" form. In contrast, the terms "modified," "mutant," and "variant" refer to a gene, gene product, or signaling network that displays modifications in sequence and or functional properties (*i.e.,* altered characteristics) when compared to the corresponding wild-type version. It is noted that naturally-occurring mutants can be isolated from a naturally occurring source, and are identified by the fact that they have altered characteristics when compared to the corresponding wild-type gene, gene product, or signaling network.

### II. SKIN SUBSTITUTES OF THE INVENTION

It has been surprisingly found that cells harvested from benign adnexal tumors are trichogenic. Specifically, mesenchymal cells exhibiting an upregulated mTORC1/TSC1/TSC2 signaling network are capable of inducing hair follicles. Such follicles are complete according to the criteria proposed by Chuong et al., "Defining hair follicles in the age of stem cell bioengineering," J. Invest. Dermatol., 127:2098-100 (2007). The follicles have a normal configuration with an epithelial filament coming out of the distal end of the follicle and dermal papilla sitting at the base of the follicle. The follicles have cells proliferating at the base of the follicle, and have concentric layers of outer and inner root sheath, cuticle and cortex. The follicles exhibit normal differentiation of the outer root sheath, and have hair shafts and sebaceous glands. The hairs go through normal cycles, and contain an epithelial stem cell component.

Accordingly, the invention provides cellular compositions capable of hair neogenesis. In one embodiment, the invention provides a skin substitute comprising epithelial cells and modified mesenchymal cells, wherein the modified mesenchymal cells have decreased TSC1/TSC2 function, increased mTORC1 function, and/or decreased mTORC2 function compared to wild type mesenchymal cells. Another embodiment of the invention provides modified mesenchymal cells, wherein the modified mesenchymal cells have decreased TSC1/TSC2 function, increased mTORC1 function, and/or decreased mTORC2 function compared to wild type cells and the modified mesenchymal cells are capable of interacting with the patient's own epithelial cells.

In one embodiment, the compositions comprise trichogenic mesenchymal cells isolated from benign adnexal tumors, which can be considered "modified" with respect to wild-type cells. Alternatively, the trichogenic cells may be artificially created by decreasing TSC1/TSC2 function, increasing mTORC1 function, and/or decreasing mTORC2 function in normal or wild-type mesenchymal cells. In the mesenchymal cells of the invention, the function of TSC1/TSC2 may be decreased, the function of mTORC1 increased, and/or the function of mTORC2 decreased by: downregulating TSC1 or TSC2; upregulating an inhibitory protein that inhibits TSC1/TSC2 function or acts as a mimetic of decreased TSC1/TSC2 function; or downregulating a stimulatory protein that stimulates TSC1/TSC2 function or acts as a mimetic of increased TSC1/TSC2 function compared to normal cells.

### A. GENERAL CHARACTERISTICS OF MAMMALIAN SKIN

Mammalian skin contains two primary layers: an outer layer called the epidermis and an inner layer called the dermis. The epidermis primarily contains keratinocytes that are formed in the deeper layers of the epidermis by mitosis and then migrate up to the surface, where they are eventually shed. The dermis contains a variety of structures including hair follicles, sebaceous glands, sweat glands, apocrine glands, nerves, lymphatic vessels, and blood vessels.

Hair follicle morphogenesis takes place mostly *in utero* during embryogenesis. Hair follicle formation begins with the appearance of epidermal placodes, which mark the location of the new hair follicle. Mesenchymal cells (*i.e.,* inductive multipotent cells) then begin to aggregate in the dermis below the epidermal placodes. The mesenchymal aggregates signal to the keratinocytes in the overlaying placodes, which then begin growing downward into the dermis. When the epidermal keratinocytes reach the mesenchymal aggregates, the cells undergo a series of differentiation and proliferation processes, eventually forming a mature hair follicle.

Mature hair follicles contain four main parts: the dermal papilla (DP), dermal sheath (DS), follicular epithelium, and hair shaft (Figure 1D). The DP is located at the base, or bulb, of the hair follicle adjacent to the hair matrix that produces the hair shaft. The DS is made up of connective tissue and envelops the hair follicle. The follicular epithelium includes the outer root sheath and the inner root sheath. The hair shaft is a proteinaceous structure that extends from the base of the follicle through the epidermis to the exterior of the skin.

The hair follicle is a dynamic miniorgan that repeatedly cycles through periods of growth (anagen), regression (catagen), and quiescence (telogen). The lower portion of the hair follicle regresses or regrows, regenerating in each cycle through complicated interactions between the dermal mesenchymal cells and epidermal cells. The permanent portion of the lower hair follicle above the continuously remodeled part is referred to as the "bulge" because it protrudes slightly from the follicle. The bulge contains multipotent cells capable of forming the follicle, sebaceous gland, and epidermis. As individuals age, the anagen and catagen phases of the hair follicle cycle become shorter, and hair follicles experience a more rapid shift to the telogen phase. As a result, normal hairs are gradually replaced by finer vellus hairs, and in some individuals, the cells may lose their trichogenic properties entirely.

### B. SKIN CELLS FOR USE IN THE INVENTION

The skin substitutes of the invention comprise either (1) modified mesenchymal cells, or (2) modified mesenchymal cells and epithelial cells. In the embodiments wherein the skin substitutes comprise modified mesenchymal cells and no epithelial cells, the mesenchymal cells interact with the patient's epithelial cells to produce a hair follicle. In the embodiments wherein the skin substitute comprises modified mesenchymal cells and epithelial cells, the modified mesenchymal and epithelial cells supplied in the skin substitute interact, with or without the patient's epithelial cells, to produce a hair follicle.

### 1. MESENCHYMAL CELLS

Generally, any source of mesenchymal cells (*i.e.,* inductive multipotent cells) are useful in the present invention. Accordingly, the present invention is not limited to the use of any particular source of cells with the capacity or potential capacity of inducing hair follicle formation. Indeed, the present invention contemplates the use of a variety of cell lines and sources that can induce hair follicles. Mesenchymal cells are usually considered mesodermal connective tissue cells that express vimentin, but vimentin-expressing cells with these attributes may also be neural crest derived. Sources of cells include the inductive multipotent cells of the dermal papilla and connective tissue sheath from hair follicles. Mesenchymal cells may be isolated from one or more of the following sources: patient skin or mucosa for autologous cells; donor skin or mucosa for allogeneic cells; normal skin; skin with an adnexal tumor; and other tissues (*e.g*., fat, bone marrow, etc.). Examples of mesenchymal cells include fibroblasts, dermal papilla cells, dermal sheath cells, onychofibroblasts (fibroblasts from nail unit), dental pulp cells, periodontal ligament cells, neural crest cells, adnexal tumor cells, induced pluripotent stem cells, and mesenchymal stem cells from bone marrow, umbilical cord blood, umbilical cord, fat, and other organs.

### 2. EPITHELIAL CELLS

Generally, any source of epithelial cells or cell line that can stratify into squamous epithelia are useful in the present invention. Accordingly, the present invention is not limited to the use of any particular source of cells that are capable of differentiating into squamous epithelia. Indeed, the use of a variety of cell lines and sources that can differentiate into stratified squamous epithelial is contemplated. Sources of cells include primary and immortalized keratinocytes, keratinocyte-like cells, and cells with the capacity to be differentiated into keratinocyte-like cells, obtained from humans and cavaderic donors (Auger et al., In Vitro Cell. Dev. Biol.--Animal 36:96-103; and U.S. Pat. Nos. 5,968,546 and 5,693,332), neonatal foreskin (Asbill et al., Pharm. Research 17(9):1092-97 (2000); Meana et al., Burns 24:621-30 (1998); and U.S. Pat. Nos. 4,485,096; 6,039,760; and 5,536,656), and immortalized keratinocytes cell lines such as NM1 cells (Baden, In Vitro Cell. Dev. Biol. 23(3):205-213 (1987)), HaCaT cells (Boucamp et al., J. cell. Boil. 106:761-771 (1988)); and NIKS cells (Cell line BC-1-Ep/SL; U.S. Pat. No. 5,989,837; ATCC CRL-12191).

Epithelial cells may also be obtained from: patient skin or mucosa (autologous), donor skin or mucosa (allogeneic), epidermal cell lines, epidermal cells derived from stem cells, primary or passaged epidermal cells, trachea, and cells derived from blood mononuclear cells or circulating stem cells. Subpopulations of epithelial cells from these sources may also be used, for example by enriching the number of cells with stem-cell properties. Epithelial cells express keratin or can be induced to express keratin, and have the capacity of forming a stratified squamous epithelium and/or follicular epithelium.

In some embodiments, the epithelial cells are from two different sources. For example, the invention may be practiced using immortalized keratinocytes together with autologous keratinocytes. The relative proportion of autologous cells to immortalized cells may be 1:99, 5:95, 10:90, 20:80, 30:70, 40:60, 50:50, 60:40, 70:30, 80:20, or 90:10. In this way, the number of autologous keratinocytes may be reduced. The immortalized keratinocytes may be enhanced to promote skin healing, for example by genetically modifying the cells to express growth factors or angiogenic factors. The immortalized keratinocytes may be modified so that they can be targeted for elimination at any point following engraftment. Specifically, in one embodiment disclosed and/or claimed herein, so called "suicide genes" may be used and the cells can be genetically modified so that they die in response to a drug treatment. (See Vogler et al., An Improved Bicistronic CD20/tCD34 Vector for Efficient Purification and In Vivo Depletion of Gene-Modified T Cells for Adoptive Immunotherapy., Mol Ther. doi:10.1038 (May 11, 2010) (advanced online epublication); and Scaife et al., Novel Application of Lentiviral Vectors Towards Treatment of Graft-Versus-Host Disease, Expert Opin Biol Ther. 2009 Jun;9(6):749-61.)

### 3. ISOLATING CELLS

Mesenchymal and epithelial cells may be isolated from skin or mucosa samples or skin tumors using any suitable techniques. For example, mesenchymal cells may be isolated by migration of cells from tissue explants. An example of such a method is described in further detail in Example 3. Alternatively, cells may be dissociated from skin or mucosa samples or skin tumors to isolate mesenchymal and epithelial cells. Examples of such a method are described in further detail in Example 3. In addition, epithelial cells may be isolated by inducing multipotent stem cells to differentiate into epithelial cells. An example of such a method is described in further detail in Example 7.

Isolated cells may be grown in any suitable medium known to those skilled in the art. Exemplary media are discussed in detail in Example 6. The samples may be enriched for hair inductive cells based on any technique known to those skilled in the art. For example, cells may be selected based on the presence of suitable cell markers, such as CD133, CD10, or nestin, as discussed in greater detail in Example 5. Alternatively, growth factors such as BMP2, 4, 5, or 6, Wnt-3a, Wnt-10b, insulin, FGF2, KGF, etc. may be added to maintain and enrich the hair inductive cells, including dermal papilla cells, as discussed in greater detail in Example 5. Cells may also be enriched for their ability to differentiate into hair follicles using the cell adhesion and cell sorting methods discussed in greater detail in Example 8.

### C. THE TSC1/TSC2 AND MTOR SIGNALING NETWORK

The modified mesenchymal cells used in the skin substitutes of the invention have either naturally occurring modifications to the TSC1/TSC2 and mTOR signaling network, or are engineered to create a modification of the TSC1/TSC2 and mTOR signaling network. Therefore, the term "modified" encompasses both naturally occurring and engineered changes to this network compared to wild-type cells.

The *TSC1* and *TSC2* genes are tumor-suppressor genes. *TSC1* is located on chromosome 9q34 and encodes a 140 kDa protein called hamartin, while *TSC2* is located on chromosome 16p13.3 and encodes a 200 kDa protein called tuberin. Hamartin (also called TSC1) and tuberin (also called TSC2) associate to form a heterodimeric protein complex called the TSC1/TSC2 complex. The TSC1/TSC2 complex acts as a central hub, linking a network of signaling networks into what is referred to herein as the TSC1/TSC2 and mTOR signaling network.

The TSC1/TSC2 complex is believed to exert its effect in the invention by inhibiting function of mTORC1, which is part of the mTOR (mammalian target of rapamycin) network. It is also believed to stimulate function of mTORC2. The mTOR network is centrally involved in growth regulation and proliferation control. Figure 2 presents a schematic overview of the mTOR network. mTOR is a member of the phosphoinositide-3-kinase-related (PI3K-related) family of kinases. Two structurally and functionally distinct mTOR-containing complexes have been identified in mammalian cells: mTORC1 and mTORC2.

The TSC1/TSC2 protein complex functions upstream of both mTORC1 and mTORC2. The TSC1/TSC2 complex exhibits a GTPase activating protein (GAP) function through the TSC2 protein, which inactivates the small G-protein Rheb (Ras homolog enriched in brain), thereby negatively regulating mTORC1. In contrast, the TSC1/TSC2 complex positively regulates mTORC2.

The TSC1/TSC2 complex and the individual TSC1 and TSC2 proteins also interact with a number of other signaling networks. For example, the TSC1/TSC2 complex interacts with components of InR (insulin-like receptor) signaling, including InR, PTEN (phosphate and tensin homologue deleted on chromosome 10), Akt (protein Kinase B), and S6K1 (70 kDa ribosomal protein S6 kinase). In addition, TSC1 interacts with the proteins DOCK7, ezrin/radixin/moesin, FIP200, IKKbeta, Melted, Merlin, NADE(p75NTR), NF-L, PIk1 and TBC7. TSC2 interacts with 14-3-3 (isoforms beta, epsilon, gamma, eta, sigma, tau, and zeta), Akt, AMPK, CaM, CRB3/PATJ, cyclin A, cyclins D1, D2, D3, Dsh, ERalpha, Erk, FoxO1, HERC1, HPV16 E6, HSCP-70, HSP70-1, MK2, NEK1, p27KIP1, Pam, PC1, PP2Ac, Rabaptin-5, Rheb, RxRalpha/VDR and SMAD2/3. The proteins axin, Cdk1, cyclin B1, GADD34, GSK3, mTOR and RSK1 have been shown to co-immunoprecipitate with both TSC1 and TSC2. The kinases Cdk1 and IKKbeta phosphorylate hamartin; Erk, Akt, MK2, AMPK and RSK1 phosphorylate TSC2; and GSK3 phosphorylates both TSC1 and TSC2. Accordingly, these various proteins and their associated signaling networks, are considered part of the TSC1/TSC2 and mTOR signaling network.

Of these, TSC1, TSC2, FLCN, MEN1, and PTEN are shared among the different syndromes discussed below that have an inherited predisposition to skin adnexal tumors.

### 1. METHODS FOR DEGREASING FUNCTION OF TSC1/TSC2 AND DOWNREGULATING MIMETICS OF DECREASED TSC1/TSC2 FUNCTION

The function of TSC1/TSC2 may be decreased by any method known to those skilled in the art, as may the function of mTORC1 be increased and/or the function of mTORC2 be decreased. This may be carried out by directly downregulating TSC1 or TSC2 and/or by downregulating a stimulatory protein that stimulates TSC1/TSC2 function or acts as a mimetic of increased TSC1/TSC2 function (*e.g*., CYLD, LKB1, FLCN, MEN1, NF1, PTEN, PRAS40, 4E-BP1, GSK3, or Deptor).

For example, cells may be treated with short complementary double stranded RNA oligonucleotides (dsRNA) such as small interfering RNA (siRNA), short interfering hairpin RNA (shRNA), micro RNA (miRNA) or interfering RNA (RNAi) directed to the gene encoding the stimulatory protein. One skilled in the art may use any standard procedure to knockdown gene expression in normal mesenchymal cells. For example, lentiviral particles may be used to deliver custom cloned short hairpin RNA (shRNA) to the mesenchymal cells. A detailed description of such a method is provided in Examples 2 and 4.

Alternatively, or in addition, gene-therapy based methods may be used to downregulate TSC1, TSC2, and/or stimulatory proteins that stimulate TSC1/TSC2 function. For example, zinc finger proteins suchs as zinc finger nucleases may be used to generate targeted double-strand breaks in the TSC1 or TSC2 genes, or in the genes encoding stimulatory proteins that stimulate TSC1/TSC2 function. Through the process of non-homologous end joining, such double strand breaks create a functional knockout of the targeted gene(s).

The function of TSC1, TSC2 and/or TSC1/TSC2 stimulatory proteins may also be inhibited by mutating the gene encoding these proteins to eliminate protein function. The function of TSC1 or TSC2 may also be regulated indirectly by decreasing the expression of certain interacting proteins. For example, knocking down the expression of polycystin-1, a protein that represses mTORC1 by protecting TSC2 from Akt phosphorylation (Dere R. et al., "Carboxy terminal tail of polycystin-1 regulates localization of TSC2 to repress mTOR," PLoS One, 5(2):e9239 (2010)), is expected to decrease TSC2 function. Another approach is to knock down the expression of proteins regulating upstream or downstream interacting proteins. For example, the loss of TSC1/TSC2 function results in activation of mTORC1, and knocking down Deptor is expected to increase mTORC1 function. Alternatively, cells may be treated with chemicals or molecules that decrease the function of the stimulatory protein. For example, TSC2 is activated by AMPK, and drugs that inhibit AMPK, such as compound C or sunitinib (Laderoute K.R. et al., "SU11248 (sunitinib) directly inhibits the activity of mammalian 5'-AMP-activated protein kinase (AMPK)," Cancer Biol Ther., 10(1) (2010)) may be able to decrease TSC2 function.

### 2. METHODS FOR UPREGULATING AN INHIBITORY PROTEIN THAT INHIBITS TSC1/TSC2 FUNCTION OR ACTS AS A MIMETIC OF DECREASED TSC1/TSC2 FUNCTION

The function of proteins that inhibit TSC1/TSC2 function or act as a mimetic of decreased TSC1/TSC2 function may be increased by any method known to those skilled in the art, as may the function of mTORC1 be increased and/or the function of mTORC2 be decreased.. (*See, e.g*., Ortiz-Urda, S. et al., Injection of Genetically Engineered Fibroblasts Corrects Regenerated Human Epidermolysis Bullosa Skin Tissue, The Journal of Clinical Investigation 111 (2): 251-255 (2003).) For example, the function of inhibitory proteins may be increased by knocking in strong promoters to drive expression of the genes encoding the inhibitory proteins. A detailed description of such a method is provided in Example 4.

Alternatively, cells may be treated with dsRNA directed to genes whose products suppress the expression or function of the inhibitory proteins. The function of inhibitory proteins may also be increased by mutating the genes encoding the inhibitory proteins to render the protein constitutively active. Alternatively, the inhibitory protein may be delivered directly to the cells, as described in detail in Example 4.

### 3. BENIGN ADNEXAL TUMORS

As discussed above, the modifications to the TSC1/TSC2 and mTOR signaling network may be naturally occurring. These naturally occurring modifications may be present in a benign adnexal tumor. Therefore, any benign adnexal tumor is believed to provide an adequate source for modified mesenchymal cells according to the invention.

Benign adnexal tumors are non-malignant skin neoplasms that exhibit morphological differentiation towards one of the different types of adnexal epithelium present in normal skin: (1) the pilosebaceous unit (*i.e.,* the hair shaft, the hair follicle, and the sebaceous gland); (2) the eccrine sweat glands; and (3) the apocrine sweat glands. Benign adnexal tumors are usually multilobulated, have symmetric and smooth borders, and have uniform collections of epithelial cells, usually with no tumor necrosis or ulceration. There is usually no atypia (*i.e.,* cellular abnormalities), and mitotic activity is generally minimal. Dense fibrotic stromal reaction occurs frequently in these tumors.

Examples of benign adnexal tumors include, but are not limited to, angiofibromas, apocrine/eccrine nevus, basaloid epidermal proliferations, basaloid follicular hamartoma, chondroid syringoma, cylindroma, desmoplastic trichilemmoma, desmoplastic trichoepithelioma, fibrofolliculoma, fibrous papules, folliculosebaceous cystic hamartoma, forehead plaques (Figure 1A), hair follicle nevi, hidroacanthoma simplex, hidradenoma, hidradenoma papilliferum, hidrocystoma, infundibulomas, intraepidermal poroma, isthmicomas, nevus sebaceous of Jadassohn, nodular hidradenoma, organoid nevi overlying dermal mesenchymal lesions, papillary eccrine adenoma, perifollicular fibromas, pilar tumor, pilar sheath acanthoma, pilomatricoma, poroma, proliferative pilomatricoma, proliferating trichilemmal cyst, sebaceous hyperplasia, sebaceoma, sebaceous adenoma, sebaceous epithelioma, sebaceous hyperplasia, sebaceous nevi, sebaceous trichofolliculoma, sebaceous tumors, shagreen patches, spiradenoma, steatocystoma, stubulopapillary hidradenoma, syringocystadenoma papilliferum, syringofibradenoma, syringofibroadenoma, syringoma, trichilemmal cyst, trichilemmoma, trichoadenoma, trichoblastoma, trichoblastic fibroma, trichodiscoma, trichoepitheliomas, trichofolliculoma, tubular apocrine adenoma, tubulopapillary hidradenoma, and ungual fibromas.

Of these conditions, angiofibromas, fibrous forehead plaques, fibrofolliculoma, trichodiscoma, and perifollicular fibroma are the most similar to each other. These share histological and immunohistological features. In addition, ungual fibroma and shagreen patch share TSC1/TSC2 abnormalities.

Benign adnexal tumors also include, but are not limited to, the tumors associated with Birt-Hogg-Dubé syndrome, Brooke-Spiegler syndrome, Cowden syndrome (CS), familial basaloid follicular hamartoma syndrome, multiple endocrine neoplasia type 1 (MEN1), neurofibromatosis (NF1), Peutz-Jeghers syndrome (PJS), and tuberous sclerosis complex (TSC).

Benign adnexal tumors consist of multiple cell types and show disorganized and excessive cell growth with a tendency to accentuate one skin structure. For example, the tumors observed in CS, which are called trichilemmomas, exhibit a thickened epithelium resembling the outer sheath of the hair follicle. The tumors found in TSC, which are called angiofibromas, appear to be hyperplasias of the papillary and/or periadnexal dermis. The tumors found in NF1, which are called neurofibromas, show exaggerated amounts of neural and fibrous tissue. Finally, the tumors found in PJS, which are called lentigines, show melanocytic hyperplasia.

Most benign adnexal tumor syndromes are caused by mutations in genes that signal through the TSC1/TSC2 complex. For example, mutations in either *TSC1* or *TSC2* cause tuberous sclerosis (TSC), a multisystem autosomal dominant disorder. Linkage analysis suggests that for familial TSC, approximately half of the mutations causing the disorder occur in *TSC1,* while the other half occur in *TSC2.* In contrast, for sporadic TSC, mutations in *TSC2* are about five times more common than mutations in *TSC1.* Patients with *TSC2* mutations seem to be more severely affected than patients with mutations in the *TSC1* gene. The mutation spectra of the TSC genes are very heterogeneous, and no hotspots of mutations have been found.

TSC affects about 1 in 6000 live births and is characterized by seizures, cognitive dysfunction, and the development of tumor-like growths in the kidneys, heart, skin, lungs, and brain. The skin lesions develop in early childhood in nearly all patients and include angiofibromas, periungual fibromas, calcified retinal hamartomas, cortical tubers, renal angiomyolipomas, hypomelanotic macules, forehead fibrous plaques, facial angiofibromas, and shagreen patches. The severity of TSC and its impact on quality of life is extremely variable. The greatest source of morbidity is the brain tumors (cortical tubers), which cause seizures in 80-90% of affected individuals and behavioral abnormalities (mostly autism) in over half of affected individuals.

The TSC1/TSC2 complex also plays a role in benign adnexal tumor syndromes caused by mutations in other genes. For example, Peutz-Jeghers syndrome (PJS) is caused by a mutation in the *LKB1* tumor suppressor gene, and is characterized by hamartoma polyps in the intestine and hyperpigmented macules on the lips and oral mucosa. The LKB1 protein is a serine/threonine kinase that phosphorylates and activates adenosine monophosphate-activated protein kinase (AMPK), which in turn phosphorylates and activates TSC2. PJS affects about 1 in 120,000 births.

Similarly, Cowden syndrome (CS), Bannayan-Riley-Ruvalcaba syndrome (BRRS), Proteus syndrome (PS), and Lhermitte-Duclos disease (LDD) are all autosomal dominant hamartoma syndromes and all involve mutations in the *PTEN* tumor suppressor gene. Loss of PTEN activity increases Akt activity, which downregulates TSC2. CS occurs in about 1 in 200,000 people. BRRS is a rare overgrowth syndrome that manifests as hamartomatous polyposis. PS affects about 100-200 people worldwide, and causes skin overgrowth and atypical bone development accompanied by tumors over half the body. Finally, LDD affects approximately 200 people worldwide, and manifests as hamartomas in the cerebellum.

In addition, neurofibromatosis type 1 (NF1) is caused by mutations in the *NF1* gene, and is characterized by the development of benign neurofibromas and malignant peripheral nerve sheath tumors. *NF1* encodes neurofibromin, which functions as a Ras-GTPase-activating protein. Ras has many functions in the cell, one of which is to inhibit the TSC1/TSC2 complex. NF1 occurs in about 1 in 3000 patients, and affected individuals can exhibit cognitive deficits, bone deformations, and hamartomatous lesions of the iris. Neurofibrosarcomas (malignant schwannomas) develop in 3% to 15% of affected individuals, most often associated with deep neurofibromas.

As a further example, 90% of autosomal dominant polycystic kidney disease (ADPKD) is caused by mutations in the *PKD1* gene. TSC1 is required for localization of PC1, and is believed to play a synergistic role in ADPKD. ADPKD is characterized by the presence of multiple cysts in both kidneys, and occurs in about 1 in 400 to 1 in 1,000 individuals worldwide. ADPKD is also associated with end-stage renal disease.

Thus, the mTORC1/TSC1/TSC2 signaling network provides a common link among several different benign adnexal tumor syndromes.

### III. METHODS OF MAKING COMPOSITIONS OF THE INVENTION

Compositions disclosed and/or claimed herein include both skin substitutes and preparations for injection.

### A. SKIN SUBSTITUTES

The skin substitutes of the invention contain different cell types than prior art skin substitutes, yet may be prepared by any methods known to those in the art (Figure 1 B). For example, Greenberg S et al., "In vivo transplantation of engineered human skin," Methods Mol Biol., 289:425-30 (2005) discloses methods for creating *in vitro* skin substitutes. In addition, Shevchenko RV et al., "A review of tissue-engineered skin bioconstructs available for skin reconstruction," J R Soc Interface, 7(43):229-58 (2010) provides a review of various approaches that may be used for preparing skin substitutes. Exemplary methods are also provided in Example 9.

In one embodiment, the compositions comprising trichogenic cells are provided in the form of a skin substitute. In some embodiments, the skin substitutes are formed by combining the trichogenic cells (or trichogenic cells with fibroblasts, endothelial cells, and/or other supportive mesenchymal cells) with a ground substance or matrix, and then overlaying the construct with epithelial cells. Prior to grafting, the epithelial cells may be induced to partially or fully form a stratified squamous epithelium and cornified layer by exposing the surface of the substitute to air.

In another embodiment, the trichogenic cells may be cultured before combining with a matrix. In another embodiment, the cell-matrix mixture is cultured before combining with the epithelial cells. In another embodiment, the trichogenic cells are grown on or below, rather than being incorporated into, the ground substance or matrix, and this is overlaid with epithelial cells.

In another embodiment, the trichogenic cells are first made into microspheres before being incorporated or inserted into, or laid on, the ground substance/matrix/scaffold, and this is overlaid with epithelial cells. The microspheres may be composed of trichogenic cells with or without epithelial cells and with or without matrix. If the microsphere has a matrix, it may be the same or different in composition from that of dermal scaffold. The ground substance/matrix/scaffold into which the microspheres are placed may be with or without added fibroblasts, endothelial cells, and/or other supportive mesenchymal cells. The spacing of the microspheres may be random or at intervals replicating the spacing of hair follicles in normal human skin.

In another embodiment disclosed and/or claimed herein, the trichogenic cells (or trichogenic cells with fibroblasts or other supportive mesenchymal cells) are used in a dermal construct that is made separately from the epidermal construct, and the two are grafted sequentially to the patient. As an alternative to using an epidermal construct, the epithelial cells may be sprayed onto the grafted dermal construct, using an aerosol of cells in media or in fibrin glue.

Compounds that may be used for the ground substance/matrix/scaffold include collagens, elastin, laminin, fibrin, hyaluronan or hyaluronic acid, fibronectin, chitosan, cellulose, silk fibroin, and alginates. These compounds may be human, rat, porcine, or bovine; from crustaceons or fungi (chitosan) or plants or algae (cellulose); or proteins expressed as recombinant forms in bacteria or other organisms. These compounds may also be modified or combined, such as hair keratin-collagen sponge, hyaluronan coupled with fibronectin functional domains, poly(lactic-co-glycolic acid)/chitosan hybrid nanofibrous membrane, polycaprolactone (PCL) collagen nanofibrous membrane, silk fibroin and alginate, polyvinyl alcohol/chitosan/fibroin blended sponge, tegaderm-nanofibre construct, bacterial cellulose, ICX-SKN skin graft replacement (InterCytex, Cambridge, England), collagen-glycosaminoglycan-chitosan, composite nano-titanium oxide-chitosan, Collatamp® (EUSAPharma, Langhorne, PA), deacetylated chitin or plant cellulose transfer membranes. The scaffold may also be human, porcine, or bovine acellular dermis, tendon, or submucosa, that can be lyophilized, cross-linked, meshed, or combined with any of the above compounds. It may be complex mixtures such as Matrigel™ (BD Biosciences) or extracellular matrix derived from fibroblasts or other cells. The matrix, ground substance, or scaffold may also consist of or incorporate synthetic materials, including silicone, polysiloxane, polyglycolic acid, polylactic acid, nylon, PolyActive™ matrix (OctoPlus, Cambridge, MA) (polyethylene oxide terephthalate and polybutylene terephthalate), and biodegradable polyurethane microfibers

The skin substitute may be supplied sealed in a heavy gauge polyethylene bag with a 10% CO₂/air atmosphere and agarose nutrient medium, ready for single use. The skin substitute may be kept in the sealed bag at 68°F-73°F (20°C-23°C) until use. The skin substitute may be supplied as a circular disk, for example, approximately 75 mm in diameter and 0.75 mm thick. The agarose shipping medium may contain agarose, L-glutamine, hydrocortisone, human recombinant insulin, ethanolamine, O-phosphorylethanolamine, adenine, selenious acid, DMEM powder, HAM's F-12 powder, sodium bicarbonate, calcium chloride, and water for injection. The skin substitute may optionally be stored on a plastic tray or in a cell culture dish within the bag. The skin substitute may be packaged with an epidermal (dull, matte finish) layer facing up and a dermal (glossy) layer facing down, resting on a polycarbonate membrane.

### B. PREPARATIONS FOR INJECTION OR IMPLANTATION

Disclosed and/or claimed herein are preparations for injection or implantation. These preparations may be prepared by any methods known to those in the art. Exemplary methods are provided in Example 9. In one embodiment, the mesenchymal cells are presented in a buffer suitable for injection, such as a sterile saline solution, phosphate buffered saline, Dulbecco's modified Eagle's medium (DMEM), Hank's balanced salt solution, Plasmalyte A, or RPMI. In one embodiment, the mesenchymal cells are incorporated into microspheres. In another embodiment, the mesenchymal cells are provided with a matrix or ground substance. The matrix may be natural polymers such as methylcellulose, collagen, chitosan, hyaluronic acid, gelatin, alginate, fibrin, fibronectin, or agarose. The matrix may be complex mixtures such as Matrigel™ or synthetic polymers. In another embodiment disclosed and/or claimed herein, the mesenchymal cells are combined with epithelial cells with or without matrix or ground substance before their use in method of injection or implantation.

The compositions comprising trichogenic cells may be subdermally or intradermally injected or implanted at a site where hair growth is desired without further culture. Cells prepared by dissociation methods as described in Example 3B may be resuspended in buffer and injected directly or first incorporated into microspheres prior to injection or implantation. The cells in culture medium can be stored on ice for 24 or more hours or frozen in liquid nitrogen for long-term storage. For cryopreservation, cells are placed in a solution of 10% DMSO, 70% DMEM and 20% fetal bovine serum. Cells are placed in cryovials at a concentration of 0.1-10 million cells per ml and frozen in a control-rate freezer and stored at -180°C until the day of injection or implantation. Viability of all thawed cells may be verified to be more than 85% before use.

Compositions comprising trichogenic cells may be injected or implanted into recipient skin or wound. Compositions may also be injected or implanted into grafts (split-thickness grafts or skin substitutes including dermal-epidermal composites and dermal constructs combined with epidermal constructs or cell spraying) before application to the patient or following grafting. The compositions comprising trichogenic cells may be cultured before injection or implantation.

### IV. METHODS OF ADMINISTERING THE SKIN SUBSTITUTES OF THE INVENTION

Disclosed herein are at least two modes of administering the skin substitutes of the invention. The skin substitutes of the invention may be grafted onto a patient (Figure 1C) or they may be injected into a patient. As such, the invention provides skin substitutes and/or modified mesenchymal cells for use in a method for transplanting cells to a patient that are capable of inducing human hair follicles in the patient.

### A. PATIENTS BENEFITTING FROM TREATMENT WITH THE INVENTION

The compositions disclosed and/or claimed herein are useful for treating patients with full-thickness or partial-thickness skin loss, devitalized skin, wounds, ulcers, chemical or thermal burns, scars, and full or partial losses or abnormalities of hair, sebaceous glands, or eccrine glands that may be congenital or acquired. Skin injuries are grouped into three categories: epidermal, partial-thickness, and full-thickness. Epidermal injuries do not require specific surgical treatment, as only the epidermis is affected and this regenerates rapidly without scarring. Partial-thickness wounds affect the epidermis and the dermis. Such wounds generally heal by epithelialization from the margins of the wound, where basal keratinocytes from the wound edge, hair follicle, or sweat glands migrate to cover the damaged area. Full-thickness injuries are characterized by the complete destruction of epithelial-regenerative elements. This type of injury heals by contraction, with epithelialization from only the edge of the wound. Partial-thickness injuries and full-thickness injuries often require skin grafting.

The compositions disclosed and/or claimed herein may also be used to treat surgical wounds. For example, the removal of large skin lesions, such as giant nevi (moles), leaves wounds that cannot heal on their own, and are too large for autologous split-thickness skin grafts. The compositions disclosed and/or claimed herein will be useful for treating such lesions.

The most common form of hair loss is a progressive hair thinning condition called androgenic alopecia. Hair loss can occur on any part of the body and can arise from any number of factors. For example, traction alopecia is most commonly found in people who pull on their hair with excessive force into ponytails or cornrows. Alopecia areata is an autoimmune disorder that can result in hair loss in just one location (alopecia areata monolocularis), or can result in the loss of every hair on the entire body (alopecia areata universalis). Hypothyroidism, tumors, and skin outgrowths (such as cysts) also induce localized baldness. Hair loss can also be caused by chemotherapy, radiation therapy, childbirth, major surgery, poisoning, mycotic infections, and severe stress. In addition, iron deficiency is a common cause of hair thinning. In many cases of hair loss, the hair follicles have stopped cycling and have entered a quiescent stage. In other cases, the hair follicles are lost completely, or never formed in the first place.

The compositions and methods disclosed and/or claimed herein are useful for treating any condition requiring growth of hair follicles. The method may also induce eccrine glands. The method may further induce sebaceous glands.

### B. ADMINISTRATION OF SKIN SUBSTITUTES

The method may comprise grafting to a patient the skin substitute of the invention. The skin substitutes of the invention may be administered by any suitable technique known to those skilled in the art.

### 1. PREPARATION OF THE GRAFT SITE

The graft site may be prepared by any technique known to those skilled in the art. An exemplary technique is provided in Example 12. The graft site may be injured skin (for example, partial- or full-thickness chemical or thermal burns, denuded skin, or devitalized skin), a wound bed with partial or complete absence of skin (for example, a site where the skin was avulsed or ulcerated), a surgical wound (for example, following excision of benign or malignant skin growths), or skin with any congenital (for example, aplasia cutis congenita) or acquired (for example, skin scarred by any cause) reduction, abnormality, or absence of hair follicles, sebaceous glands, and/or eccrine glands. The graft site may be washed with water, an antibiotic wash, or an alcohol solution (such as an alcohol swab). A desired pattern of hair may be drawn on the graft site with a surgical marker. A local anesthetic may be administered to the patient. In cases requiring further anesthetics, a gaseous, intravenous, or nerve block anesthetic may be administered to the patient.

The existing skin tissue, devitalized tissue, eschar, wound or ulcer edges, or scar tissue may be removed using standard techniques in the art. When possible, any skin infections or deteriorating conditions should be resolved prior to application of the graft. Antimicrobial, antifungal, and antiviral agents, administered topically or systemically, may be used during a period of time (such as a week) prior to and following administration of the skin substitute to reduce the risk of infection.

Skin substitutes may be applied to a clean, debrided skin surface after thoroughly irrigating the wound with a non-cytotoxic solution. Debridement may extend to healthy, viable, bleeding tissue. Prior to application, hemostasis may be achieved. Prior to debridement the wound may be thoroughly cleansed with sterile saline to remove loose debris and necrotic tissue. Using tissue nippers, a surgical blade, or curette, hyperkeratotic and/or necrotic tissue and debris may be removed from the wound surface. Ulcer margins may be debrided to have a saucer effect. After debridement, the wound may be cleansed thoroughly with sterile saline solution and gently dried with gauze. Oozing or bleeding resulting from debridement or revision of wound edges may be stopped through the use of gentle pressure, or if necessary ligation of vessels, electrocautery, chemical cautery, or laser. Heavy exudation may displace a skin substitute and reduce adherence. Exudation may be minimized by appropriate clinical treatment. For example, sterile air at room temperature or up to 42°C may be blown over the wound until the wound is sticky. If exudation persists, the skin substitute may be made permeable to exudate by perforating the skin substitute to allow for drainage.

### 2. APPLICATION OF THE SKIN SUBSTITUTE

A variety of clinical techniques may be used for applying the skin substitute to the patient. Skin substitutes may be applied in the outpatient clinic or in a surgical suite depending on the size of the defect being repaired, pain level, and the need for general anesthesia. Exemplary techniques are described in Examples 11 and 12. Before applying the skin substitute, the practitioner can review the expiration date of the skin substitute, check the pH, and visually observe and smell the skin substitute to ensure that there are no contaminants, such as bacterial contaminants or particulate matter. The skin substitute may be stored in a polyethylene bag at controlled temperature 68°F-73°F (20°C-23°C) until immediately prior to use.

The practitioner may cut open the sealed polyethylene bag, and if the skin substitute is provided in a cell culture dish or plastic tray, it may be transferred to the sterile field with aseptic technique. If present, a tray or cell culture dish lid may be lifted off, and the practitioner may note the epidermal and dermal layer orientation of the skin substitute. Using a sterile atraumatic instrument, a practitioner may gently dislodge approximately 12.7 mm (0.5 inch) of the skin substitute away from the wall of the tray or cell culture dish. When lifting the skin substitute, a practitioner may be careful not to perforate or lift any membrane beneath the skin substitute, which, if present, should remain in the tray.

With sterile gloved hands, a practitioner may insert one index finger under the released section of the skin substitute and use the other index finger to grasp the skin substitute in a second spot along the edge of the device. Holding the skin substitute in two places, the practitioner may lift the entire skin substitute out of the tray or cell culture dish using a smooth, even motion. If excessive folding occurs, the skin substitute can be floated (epidermal surface up) onto warm sterile saline solution in a sterile tray.

The skin substitute may be placed so that the dermal layer (the glossy layer closest to the medium) is in direct contact with the site for the skin substitute.

Using a saline moistened cotton applicator, the practitioner may smooth the skin substitute onto the site so there are no air bubbles or wrinkled edges. If the skin substitute is larger than the site for application, the excess skin substitute may be trimmed away to prevent it from adhering to the dressing. If the skin substitute is smaller than the site for application, multiple skin substitutes may be applied adjacent to each other until the defect is filled.

The skin substitute may be secured with any appropriate clinical dressing. Sutures or samples are not required but may be used in some instances to anchor the graft to the graft bed. Dressings may be used to assure contact of the skin substitute to the site for application and to prevent movement. Therapeutic compression may be applied to the graft site. In some cases it may be necessary to immobilize the grafted limb to minimize shearing forces between the skin substitute and the application site. Dressings may be changed once a week or more frequently if necessary.

Additional applications of skin substitutes may be necessary in certain instances. Prior to additional applications, non-adherent remnants of a prior skin graft or skin substitute should be gently removed. Healing tissue or adherent skin substitutes may be left in place. The site may be cleansed with a non-cytotoxic solution prior to additional applications of skin substitute. An additional skin substitute may be applied to the areas where the prior skin substitute is not adherent.

### C. INJECTION OF TRICHOGENIC CELLS

The trichogenic cells of the invention may be injected by any suitable method known to those skilled in the art. An exemplary method is described in Example 11. In one embodiment disclosed herein, the method comprises subdermally or intradermally delivering to a patient modified mesenchymal cells having decreased TSC1/TSC2 function, increased mTORC1 function, and/or decreased mTORC2 function compared to wild type mesenchymal cells. In another embodiment disclosed herein, the method further comprises delivering epithelial cells to the patient. Cells may be delivered as a suspension or as microspheres. When injecting a suspension, each injection site may deliver 50-2,000 cells. When injecting microspheres, each injection site may deliver one or more microspheres.

### 1. PREPARATION OF THE GRAFT SITE

The graft site may be washed with water, an antibiotic wash, or an alcohol solution (such as an alcohol swab). A desired pattern of hair may be drawn on the graft site with a surgical marker, either in an outline fashion or a pixilated fashion showing each injection site. Paper templates or templates of other material may also be applied to the injection site showing the pattern for injection, or injections may be delivered at the correct spacing by using robotics or a device with multiple injection ports in a grid. A local anesthetic may be administered to the patient. In cases requiring further anesthetics, a gaseous, intravenous, or nerve block anesthetic may be administered to the patient.

### 2. INJECTION METHODS, DOSAGE, AND FREQUENCY OF ADMINISTRATION

The injections may be administered according to techniques known in the art for subdermal or intradermal injections. A concentration of 1,000 to 20,000 cells/ml may be used in the injection. A volume of 0.05 to 0.1 ml may be injected at each injection site using a 1-3 ml syringe with a 14-30 gauge needle. In such embodiments, the skin is pulled taut, and the needle is inserted bevel up at a 5° to 30° angle with the skin. The cells are then injected slowly with gentle pressure, the needle is removed, and gentle pressure is applied to prevent leakage and promote absorption.

Injections may be repeated over a period of time, either for patient comfort or because additional hair follicles may be produced after repeated administration. In such a case, the administrations may be spaced a week apart, two weeks, three weeks, a month, two months, three months, or six months apart.

Several of the foregoing embodiments are illustrated in the non-limiting examples set forth below. However, other embodiments of the invention will be apparent to those skilled in the art from consideration of the specification and practice of the invention disclosed herein. It is intended that the specification and examples be considered as exemplary only.

### V. EXAMPLES

### EXAMPLE 1: PREPARATION AND EVALUATION OF SKIN SUBSTITUTE FROM TSC PATIENTS

TSC skin hamartomas, including fibrous forehead plaques, angiofibromas, and periungual fibromas, contain dermal and/or perifollicular fibroblast-like cells and variable changes in the epithelium. Patients diagnosed with TSC were enrolled in an Institutional Review Board-approved protocol, 00-H-0051 at the National Heart, Lung, and Blood Institute, NIH. Samples of angiofibromas, periungual fibromas, fibrous plaques, and normal-appearing skin from TSC patients were obtained and bisected, with one portion used for routine pathology and the other used for frozen sections or cell culture.

### A. HISTOLOGICAL AND IMMUNOHISTOCHEMICAL COMPARISON OF NORMAL TISSUE SAMPLES AND TUMOR TISSUE SAMPLES

The histological (Figures 3A and 3B) and immunohistochemical differences between normal and tumorous patient samples were characterized as a baseline for comparison. Briefly, paraffin sections of the samples were deparaffinized and treated for antigen retrieval by boiling in 10 mM sodium citrate buffer (pH 6.0) for 20 minutes. Frozen sections were fixed in acetone at -20°C for 10 minutes. Sections were stained for cellular markers using specific antibodies and VECTASTAIN ABC kit with Vector® Red or DAB substrate (Vector Laboratories, Burlingame, CA) according to manufacturer's procedures (except for Ki-67 staining, which used ABC-horse radish peroxidase staining with DAB peroxidase brown substrate). Relative intensity of positive staining was quantified using an Olympus BX40 light microscope (Olympus, Melville, NY) and Openlab 4.0 software (Improvision, Lexington, MA).

Vessels in paraffin-embedded patient samples were stained with a rabbit polyclonal antibody to CD31 (Abcam Inc, Cambridge, MA). The number and area of positive vessels were measured and normalized by total area. Cell proliferation in the samples was detected by immunostaining using a rabbit monoclonal antibody against Ki-67 (Thermo Scientific, Fermont, CA). Ki-67 positive cells were counted using an Olympus BX40 light microscope (Olympus, Melville, NY), and normalized by the length of the epidermis. Tumor-associated macrophages were detected in the samples by immunohistochemical staining with rat anti-mouse F4/80 antibody (Abcam). Macrophage content in the dermis was measured using a 20X objective by counting three random fields of each section and normalizing by area. CD68-positive mononuclear phagocytes were identified by immunohistochemical staining using an ABC kit (vector) and an anti-CD68 antibody (M0814, DakoCytomation). Finally, elevated mTORC1 function was determined by immunostaining for phosphorylated ribosomal protein S6 (anti-pS6, 2211, Cell Signaling).

The fibrous plaques showed larger cells, altered collagen structure, and increased vessels compared to normal skin (Figure 3A and 3B). Fibroblast-like cells from TSC fibrous plaques, like angiofibromas and ungual fibromas, also showed increased immunoreactivity for phosphorylated ribosomal protein S6 compared to normal fibroblasts (Figures 3C and 3D), indicating that fibrous plaques exhibit increased mTOR function compared to normal skin. The epidermis of the fibrous plaques also exhibited greater immunoreactivity for pS6 (Figures 3C and 3D), as well as greater proliferation (Figures 3E and 3F) than normal skin. This may be caused by paracrine factors released by the TSC2-null cells. These changes in hamartoma fibroblast-like cells and epidermal cells were accompanied by dramatic increases in two additional cellular constituents: CD68-positive mononuclear phagocytes (Figures 3G and 3H), and CD31-positive blood vessels (Figures 3I and 3J). The studies were done with tissue samples.

Taken together, these experiments revealed that the forehead plaques, like angiofibromas and ungual fibromas, all contain increased mTORC1 function, CD31-positive vessels, CD68-positive mononuclear phagocytes, and proliferating (*i.e.,* Ki-67-positive) epidermal cells compared to TSC normal-appearing skin.

### B. ANALYSIS OF HAIR FOLLICLES IN TUMOR TISSUE SAMPLES AND NORMAL TISSUE SAMPLES

Compared to those in normal skin, hair follicles in fibrous plaques and angiofibromas appeared variably enlarged, elongated, or greater in number, whereas periungual fibromas had a thickened epidermis but no hair follicles (Figures 4A-4D). This observation is based on tissue sections stained with hematoxylin and eosin. Hair follicles in the angiofibromas were variably hypertrophied, elongated or immature. The ungual fibromas did not have follicular structures.

### C. ANALYSIS OF TSC2 AND MTORC1 FUNCTION IN TUMOR CELLS

Fibroblasts were isolated from the angiofibromas, periungual fibromas, forehead plaques, and normal-appearing skin biopsies by cutting the biopsies into small pieces and plating them on 35 mm culture dishes in 1 ml DMEM with 10% FBS, penicillin (100 U/ml) and streptomycin (100 µg/ml) to cover the tissue. The medium was changed twice a week until the cells migrated to cover the dishes. The cells were then harvested for sub-culture.

The isolated cells were analyzed for TSC2 expression by PCR, restriction digestion, and sequence verification. Briefly, DNA isolated from the cultured cells was used for amplification of exon 10 of TSC2 using AmpliTaq gold DNA polymerase (Applied Biosystems) in magnesium-containing buffer supplied by the manufacturer. Thermocycling was performed as follows: denaturation at 95°C for 30 seconds, annealing at 59°C for 1 minute, and extension at 72°C for 1 minute, followed by cycling 34 times and a final extension at 72°C for 1 minute. The PCR-amplified DNA products were separated by electrophoresis and purified by QIAquick Gel Extraction kit (QIAGEN). Purified DNA was sequenced by USU BIC Genomic Division-DNA Sequencing Service using a 3130xl Genetic Analyzer with ABI PRISM BigDye Terminator v3.1 Cycle Sequencing Kits BigDye® Terminator v3.1 Cycle Sequencing Kit (Applied Biosystems).

The PCR primers for sequencing were: 5'TGGTGTCCTATGAGATCGTCC3' and 5'AGGAGCCGTTCGATGTT3'. Sequencing of TSC2-null cells from fibrous forehead plaques revealed a nonsense mutation in the *TSC2* gene. Specifically, a G at position 1074 in exon 10 was mutated to an A, which converted the normal UGG codon for tryptophan into the stop codon UGA (Figure 5A). Cells also showed a loss of heterozygosity at three microsatellite markers flanking the *TSC2* gene (Figure 5B), rendering the cells homo- or hemizygous for the point mutation in exon 10. The point mutation introduced a new restriction site for *Bsm*A1 cleavage of PCR-amplified tumor DNA (Figure 5C). Normal patient fibroblasts did not contain the mutation (Figure 5D).

The PCR-amplified DNA products were also analyzed by restriction enzyme digestion. The PCR primers were used to amplify exon 10 for enzyme digestion analysis were: 5'AAGCAGCTCTGACCCTGTGT3' and 5'GGCCCAAGGTACCATCTTCT3'. To confirm the presence of the G→A point mutation introducing the *Bsm*A1 restriction site, 2 µl of PCR-amplified DNA was mixed with 10X Buffer 4 (NEB), 2 µl of *Bsm*A1 (NEB), and 12 µl of water. The mixtures were incubated at 55°C overnight and samples of digested and undigested PCR products were separated by electrophoresis at 100 volts in 10% TBE gels. Cleavage was determined based on the migration patterns of the bands in the gel.

The cells were also analyzed for hyperphosphorylation of ribosomal protein S6 under conditions of serum starvation. Cells (5 X 10⁵ cells in DMEM with 10% FBS) are seeded in 60-mm dishes. The next day, the medium was replaced by serum-free DMEM. After incubation at 37 °C for another 24 hours, cells were lysed in protein extraction buffer (20 mM Tris, pH 7.5, 150mM NaCl, 1% Nonidet P-40, 20 mM NaF, 2.5 mM Na₂P₄O₇, 1mM β-glycerophosphate, 1 mM benzamidine, 10 mM p-nitrophenyl phosphate, 1 mM phenylmethylsulfonyl fluoride). Samples comprising equivalent amounts of total protein were separated in 10% (w/v) polyacrylamide gels and transferred to 0.45-µm Invitrolon™ PVDF membranes (Invitrogen Corporation) before immunoblotting using anti-phospho-S6 ribosomal protein (Ser 235/236) or anti-S6 ribosomal protein primary antibodies (Cell Signaling), horseradish peroxidase-conjugated anti-rabbit secondary antibodies (GE Healthcare, UK), and SuperSignal West Pico chemiluminescence detection kit (Pierce Chemical, Rockford, IL). Band intensity was measured using Kodak Capture DC 290 imaging system (Eastman Kodak Co, Rochester, NY).

The cells were also treated with rapamycin, which inhibits mTORC1. Specifically, TSC skin tumor cells or normal-appearing fibroblasts were plated in a 96-well plate (2000 cells per well) in DMEM containing 10% FBS. The next day, the medium was changed to 10% FBS/DMEM with or without rapamycin at 0.2 nM, 2 nM, or 20 nM for 3 days. The cell numbers were then assessed using an MTT cell proliferation assay kit (CELLTITER® Non-Radioactive Cell Proliferation Assay (Promega, Madison, WI)). These experiments revealed that rapamycin blocked mTORC1 activation (Figure 6A), (mTORC1 activation was measured by phosphorylation of the downstream molecule, S6, as described above) and decreased the *in vitro* proliferation of TSC2-null fibroblasts to a greater extent than paired samples of fibroblasts from patient normal-appearing skin (Figure 6B). Since rapamycin is a specific inhibitor of mTORC1, these results confirm that the phosphorylation of S6 is due to activation of mTORC1 in the TSC2-null cells.

In summary, these results showed that some samples exhibited dramatically decreased expression of TSC2 and corresponding constitutive activation of mTORG1. In fibroblast-like cells grown from 3 of 4 fibrous plaques, 3 of 65 angiofibromas, and 8 of 41 periungual fibromas, TSC2 protein expression was undetectable or barely detectable, and mTORC1 was constitutively active (Figure 7). To obtain samples that were pure or highly enriched for TSC2-null cells, cultured fibroblast-like cells were screened for loss of TSC2 expression and mTORC1 activation. These cells were used in the xenografts described below.

### D. XENOGRAFT MODELS FOR TSC SKIN HAMARTOMAS

An extensively used system of *in vitro* constructed dermal-epidermal composites was adapted for grafting. Keratinocytes with accompanying melanocytes were isolated from foreskins of unidentified normal neonates by treating with dispase (Becton Dickinson Labware, Bedford, MA) at 4°C overnight. The epidermal sheet was separated from dermal sheets and subsequently digested with 0.05% trypsin-0.53 mM EDTA (Invitrogen, Gaithersburg, MD) at 37°C for 20 min. The cells were collected and plated on tissue culture dishes in keratinocyte serum-free media (Invitrogen) supplemented with bovine pituitary extract and recombinant epidermal growth factor.

Skin substitutes were created by mixing TSC2-null skin tumor cells or TSC2 normal fibroblasts harvested from female patients with 1 mg/ml of rat tail collagen type 1 (BD Biosciences, Bedford, MA) in 10% FBS/DMEM. The mixture was placed into 6-well Transwell plates (Corning Inc., Corning, NY) at a density of 0.5 x 10⁶ cells per well. The cell mixture was cultured for 3 days, and then the cultured keratinocytes were added at a density of 1 x 10⁶ cells per well. The constructs were then submerged in a 3:1 mixture of DMEM and Ham's F12 (GIBO/Invitrogen, Grand Island, NY) containing 0.1 % FBS, and cultured for 2 days. After culture, the keratinocytes were brought to the air-liquid interface, by removing some of the liquid, and cultured in DMEM and Ham's F12 (1:1) containing 1% FBS for another 2 days before grafting.

Mice were grafted in a surgery room using 6-8 week old female Cr:NIH(S)-nu/nu mice (FCRDC, Frederick, MD). Mice were anesthetized using inhalant anesthesia with a mixture of O₂ and isoflurane (2-4%). The grafting area on the back of the mouse was estimated, and skin was removed using curved scissors after washing with povidine and 70% ethanol. Skin substitutes were placed on the graft bed in correct anatomical orientation, covered with sterile petroleum jelly gauze, and secured with bandages. The mice were then transferred to sterile cages after reawakening. The bandages were changed at week 2 and removed after 4 weeks.

In mice sacrificed 8 to 17 weeks after grafting, grafts containing TSC normal fibroblasts formed skin without hair follicles (Figure 8A). Grafts containing TSC2-null cells from certain TSC skin tumors formed hair follicles (Figure 8B, Table 1), suggesting that TSC skin tumor cells induced follicular neogenesis in the foreskin keratinocytes.

**Table 1. Follicle formation in tumor grafts using cells from different patients and tumor grafts in mice treated with or without rapamycin**

| **i. Tumor Grafts** | | | | | |
|---|---|---|---|---|---|
| **Patient**# | **Patent age (years)** | **Tumor** | **Location** | **Graft duration (weeks)** | **Grafts with hair follicles/ total HLA-positive grafts** |
| 1 | 38 | fibrous plaque | left forehead | 17 | 0/4 |
| 1 | 38 | angiofibroma | left alar groove | 17 | 0/4 |
| 2 | 51 | periungual fibroma | right 4^{th} toe | 17 | 0/3 |
| 3 | 31 | fibrous plaque | right supraclavicular | 17 | 1/5 |
| 4 | 20 | fibrous plaque | central forehead | 8 | 3/5 |
| 4 | 20 | fibrous plaque | central forehead | 17 | 2/3 |
| 5 | 31 | angiofibroma | right alar groove | 17 | 3/5 |

| **ii. Grafted mice treated with or without rapamycin** | | | | | |
|---|---|---|---|---|---|
| **Sample** | **Treatment** | **Grafts with hair follicles/ total HLA-positive grafts** | **Follicular density (follicles/mm)** | **Follicular area/ dermal area (%)** | **Hair follicle diameter (µm)** |
| Patient 4 fibrous plaque | Rapamycin | 7/15 | 2.35 ± 0.09 | 9.3 ± 0.3 | 99.8 ± 6.0 |
| | Vehicle | 7/12 | 2.16 ± 0.36 | 11.7 ± 2.5 | 116 ± 14 |

Hair follicles in the grafts were appropriately spaced and anatomically complete. A hair shaft, sebaceous glands, concentric layers of inner and outer root sheath surrounded by a dermal sheath, and hair bulb with dermal papilla, hair matrix, and cortex were all present (Figure 9A-9D). The hair follicles mimicked the region from which they were obtained. For example, as in facial skin, more follicles were in catagen (regressing) and telogen (resting) than anagen (growing), which is more typical of scalp follicles (Figure 8B). In addition, hair shafts were not visible from the skin surface of grafts of cells harvested from the forehead or nose (Figure 1C). These results suggest that the invention may produce optimal results if the source of the mesenchymal cells mirrors their ultimate destination (*i.e.,* mesenchymal cells from the scalp are used to treat a balding scalp, while mesenchymal cells from the arm are used to treat a burn on the arm).

The hair shafts lacked the regularly spaced air pockets of murine hair, consistent with their being of human origin. Immunohistochemistry with an anti-human COX IV antibody was performed to confirm the species of origin of the follicles. Briefly, paraffin sections of the xenografts were deparaffinized and treated for antigen retrieval as discussed above for the patient tissue samples. Sections were then stained according to manufacturer's instructions with an anti-COX-IV 3E11 antibody (Cell Signaling technology, Danvers, MA), which does not recognize mouse COX IV. Immunoreactivity was observed in the follicles, epithelium, and dermis of xenografts (Figures 9E and 9F), but not in mouse skin (data not shown). Similar results were obtained using a pan-human HLA class I monoclonal antibody (Figure 10A), which stained interfollicular epidermis more intensely than follicular epithelium, as expected in normal skin.

Fluorescence *in situ* hybridization using a probe for the human Y chromosome was performed to distinguish between the human foreskin keratinocytes (which are of male origin) and the TSC2-null cells from female patients. Briefly, Y chromosome FISH was carried out using CEP Y (DY21) chromosome spectrum orange probe (Vysis, Downers IL60515) according to the manufacturer's protocol. The probe hybridized to nuclei in the epidermis and the follicular epithelium, but not to the nuclei of dermal cells (Figures 9G and 9H) or flanking normal mouse skin (not shown). These results show that the foreskin keratinocytes were induced to differentiate into several of the cellular components that compose normal hair follicles, confirming *de novo* hair follicle induction.

The normality of the induced hair follicles was further confirmed by immunohistochemistry using markers of specific compartments of fully developed human hair follicles. Briefly, paraffin sections of the xenografts were deparaffinized and treated for antigen retrieval as discussed above. Sections were then stained with anti-human nestin antibodies (AB5922, Millipore), anti-human versican antibodies (PA1-1748A, Thermo Scientific, Rockford, IL), anti-Ki-67 antibodies (RM-9106, Thermo Scientific), anti-human keratin 15 antibodies (PCK-153P, Covance), and anti-cytokeratin 75 antibodies (GP-K6hf, Progen Biotechnik GmbH).

Cells in the region of the dermal papilla and lower dermal sheath showed normal staining for nestin (Figures 9I and 9J) and versican (Figure 9K). Immunoreactivity for Ki-67 was concentrated in the region of the hair matrix (Figure 9M), typical of active anagen phase proliferation with robust hair shaft formation. Keratin 15, a marker for hair follicle stem cells located in the bulge region, was localized in the basal layer of the outer root sheath (Figures 9N and 9O), as observed in human angiofibromas. Finally, immunoreactivity for keratin 75, a marker for the companion layer, was present in a single layer of cells between the inner and outer root sheaths (Figure 9P), as observed in normal human hair. Thus, by both morphological and immunohistochemical criteria, fully developed human hair was present in the xenografted skin.

Sections were also analyzed for alkaline phosphatase activity. Briefly, frozen sections were fixed in acetone for 10 minutes, then washed in 1X PBS with 0.1 % Tween 20. Sections were incubated for 15 minutes in a humid chamber at room temperature with the pre-equilibration buffer (100 mM NaCl, 50 mM NgCl₂, 100 mM Tris-HCl, pH 9.5, 0.1% Tween-20). Developing solution (BM Purple AP substrate, Roche, Indianapolis, IN) was applied to the tissue for 2 hours in a dark humid chamber. The reaction was then stopped with 20 mM EDTA in PBS, and sections were mounted with VectaMount™ AQ Aqueous Mounting Medium (Vector). Cells in the region of the dermal papilla and lower dermal sheath showed normal alkaline phosphatase activity (Figure 9I). This indicates that the grafted TSC2-null cells exhibit alkaline phosphatase activity in the proper location as expected for dermal sheath/dermal papilla cells.

The genetic identity of the cells in the xenografts was investigated to determine the presence of TSC2-null cells in the dermal papilla/lower dermal sheath regions of the induced follicles. Briefly, sections of xenografts were microdissected and DNA extracted for restriction enzyme analysis, as discussed above. These studies revealed mutant DNA in cells from the region of the dermal papilla/lower dermal sheath, but not in follicular epithelium (Figures 5C and 5D). The presence of TSC2-null cells in this region and in the interfollicular dermis (data not shown), indicated that TSC tumor fibroblast-like cells are multipotent progenitor cells that can exhibit features of dermal fibroblasts or dermal papilla/dermal sheath cells.

The xenograft model was also used to determine whether the TSC2-null cells were able to induce the cytological and biochemical alterations observed above for tumor tissue samples. Briefly, mice grafted with TSC tumor cells (n=27) or TSC normal fibroblasts (n=27) either received rapamycin (2 mg/kg) (n=29) or an equal volume of vehicle (0.9% NaCl, 5% polyethylene glycol, and 5% Tween-80) (n=25) by intraperitoneal injection on alternate days for 12 weeks beginning at week 5 after grafting. Mice were sacrificed 24 hours after the last injection, the grafts were harvested, and one half of each graft was prepared for paraffin embedding, while the other half was prepared for frozen sections. Paraffin sections were stained for blood vessels (CD31) (Figures 11Q-11T), phosphorylation of ribosomal protein S6 (pS6) (Figures 11E-11H), and persistence of human cells (COX-IV) (Figures 11A-11D). Frozen sections were stained for cell proliferation (Ki-67) (Figures 11I-11L), and tumor associated macrophages (F4/80) (Figures 11M-11P).

There were no gross differences in size or appearance between tumor and normal grafts in mice treated with or without rapamycin. For mice treated with vehicle, the numbers of COX IV-positive cells in the dermis of the tumor grafts was similar to those in normal grafts (Figures 11A, 11C, and 12A). However, tumor grafts treated with vehicle contained greater numbers of dermal and epidermal cells immunoreactive for pS6 than normal grafts (Figures 11E, 11G, 12B, and 12C). In addition, the epidermis of tumor grafts treated with vehicle had greater numbers of Ki-67-positive cells than normal grafts (Figures 11I, 11K, and 12D). Tumor grafts treated with vehicle also contained increased numbers of CD68-positive mononuclear phagocytes and increased CD31-positive vessel density, size, and total vessel area (Figures 11Q and 11S) compared to normal grafts. Qualitatively similar changes were observed using the TSC2-null cells from the other patient fibrous plaques, angiofibromas, and periungual fibroma, compared to normal grafts constructed from TSC normal fibroblasts. Because the tumor grafts and normal grafts were both generated using the same neonatal foreskin keratinocytes, these results show that TSC2-null cells are sufficient to induce the hamartomatous features of TSC skin tumors.

Rapamycin treatment decreased the number of human dermal cells in tumor xenografts, as determined by staining with human anti-HLA class I (Figure 13C,D) or COX-IV antibodies (Figure 11C,D), but tumor cells persisted throughout treatment. Rapamycin had no significant effects on cell number in normal xenografts (Figures 13 and 14). Persistence of TSC2-null cells at the end of treatment was confirmed by the presence of mutant DNA in both microdissected dermis of tumor xenografts and in fibroblasts grown from tumor xenografts following harvesting (data not shown). TSC2-null cells persisted despite *in vivo* penetration of rapamycin, as shown by loss of pS6 immunoreactivity in dermal and epidermal cells (Figures 11E and 11G). Rapamycin treatment decreased the number of Ki-67 positive epidermal cells, mononuclear phagocytes, and vessel density, size, and total area in tumor grafts (Figures 11 and 12). These results suggest that the decreased redness and size of TSC skin lesions observed in patients taking rapamycin may result from both anti-tumor cell effects and anti-angiogenic effects. The antiangiogenic effects of rapamycin may be due to direct inhibition of vascular endothelium and/or indirect effects such as decreased release of angiogenic factors by TSC2-null cells or decreased recruitment of pro-angiogenic mononuclear cells. Rapamycin did not influence the percentage of grafts with hair follicles, hair follicle density, or hair follicle diameter (Table 1). The lack of effect on hair follicle parameters may indicate that induction of follicles is mTORC1-independent, or that rapamycin was ineffective after follicular neogenesis had commenced.

Fluorescence *in situ* hybridization using probes specific for human or mouse DNA was performed to distinguish human from mouse cells in the xenografts using TSC2-null cells and human keratinocytes (Figure 15). Four µg frozen sections were air-dried before incubating in 2X SSC buffer at 37°C for 20 min. Following sequential dehydration in ethanol, sections were treated with 10 mM HCl plus 0.006% pepsin at 37°C for 2.5 min and washed twice in PBS before dehydrating and air drying. Sections were denatured in 70% formamide, 2X SSC at 70°C for 2 min and dehydrated before hybridizing overnight with probe mixture (10.5 µL of hybridization buffer and 2 µL of probe) at 37°C. The sample was washed twice at 37°C with 2X SSC/50% formamide and counterstained by applying 10 µL of DAPI (Vector Laboratories) on each target area. The probes used were Conc. Human Pan Centromeric Paint 1695-Cy3-02 (cat# SFP3339) and Conc. Mouse Pan Centromeric Paint-FITC 1697-MF-02 (cat# MF-02) (Openbiosystem). DAPI stain showed nuclei of cells comprising the hair follicle bulb, including the follicular epithelium and dermal papilla/dermal sheath cells and, at the left lower corner, vascular endothelial cells. The Cy3 human-specific centromeric probe marked cells in the hair follicle bulb, including cells of the lower dermal sheath (horizontal arrow) and adjacent dermal fibroblasts (vertical arrows). The FITC mouse-specific centromeric probe labeled endothelial cells (arrowhead). A merged image showed that cells of the follicular epithelium, dermal sheath, and dermal papilla were of human origin (arrows). These results demonstrate that the hair follicles were of human origin, both for the epidermal and the dermal (dermal papilla and dermal sheath) components.

### E. SUMMARY AND CONCLUSIONS

This example discloses skin substitutes capable of follicular neogenesis. This example also discloses the development of a xenograft model for skin hamartomas in tuberous sclerosis complex (TSC). TSC2-null fibroblast-like cells grown from human TSC skin hamartomas, but not fibroblasts from patient normal-appearing skin, stimulated histological changes mimicking TSC hamartomas and induced normal human foreskin keratinocytes to form hair follicles. Follicles were periodically spaced, correctly oriented, and complete with sebaceous glands, hair shafts, inner and outer root sheaths, and expressed markers of the companion layer and bulge region of stem cells. TSC2-null cells surrounding the lower portion of the hair follicle (*i.e*., the hair bulb) expressed markers of the dermal sheath and dermal papilla, including the stem-cell marker nestin. Tumor xenografts recapitulated features of TSC skin hamartomas including increased mTORC1 function, angiogenesis, and proliferation of overlying epidermal cells. Treatment with rapamycin, an mTORC1 inhibitor, normalized these parameters and reduced the number of tumor cells, but did not alter hair follicle size or density.

These studies indicate that the disordered tissue architecture of hamartomas results from cells with inductive capabilities that are normally found during fetal tissue development. Thus, this example shows that TSC2-null fibroblast-like cells are the inciting cells for TSC skin hamartomas, simulating angiogenesis, and are capable of inducing follicular neogenesis. The expression of stem-cell markers and the preservation of hair-inducing ability by these cells suggest that loss of TSC2 function alters differentiation of a multipotent progenitor cell in the dermis. In mice, loss of TSC2 in radial glia increases a progenitor pool and decreases neurons, whereas deletion of TSC1 in hematopoietic stem cells increases granulocyte-monocyte progenitors and decreases megakaryocyte-erythrocyte progenitors. TSC2-null cells from angiofibromas and fibrous plaques are tools for exploring follicular morphogenesis and regeneration. The fact that TSC skin tumors usually arise postnatally suggests the possibility of creating or enhancing follicle-inducing cells by using agents impacting the TSC1/TSC2 network and/or signaling networks involved in the genesis of other follicular hamartomas. This study of hamartomas provides insights into tissue organization and maturation.

### EXAMPLE 2: TSC2 AND FLCN KNOCKDOWN STUDIES

To mimic the loss of TSC2 expression observed in cells with proven trichogenic capabilities, shRNA was used to knock down TSC2 expression in cultured fibroblasts and dermal papilla cells. In addition, since patients with Birt-Hogg Dube syndrome have a loss of FLCN function that leads to the formation of skin hamartomas similar to TSC skin hamartomas, shRNA was also used to knock down FLCN expression in cultured fibroblasts and dermal papilla cells. As discussed below, TSC2 and FLCN knockdowns enhanced the trichogenic properties of cells.

### A. GENE KNOCKDOWNS

Wild-type mesenchymal cells (*i.e*., dermal fibroblasts and dermal papilla cells) were modified to decrease TSC1/TSC2 function and increase mTORC1 function by knocking down expression of TSC2 using shRNA to TSC2. In addition, wild-type mesenchymal cells were modified to mimic loss of TSC1/TSC2 function by decreasing expression of FLCN using shRNA to FLCN. Commercially available lentiviral particles carrying the pGIPZ-lentiviral shRNAmir vector containing a hairpin sequence targeting TSC2 (Open Biosystems) were used to knockdown TSC2 expression. Commercially available lentiviral particles carrying the pGIPZ-lentiviral shRNAmir vector contianing a hairpin seqeunce targeting FLCN (Open Biosystems) were used ot knockdown FLCN expression. Neonatal foreskin fibroblasts or human dermal papilla cells were transduced by the lentiviral particles followed by puromycin selection (2µg/ml) starting 48 hrs post transduction. The cells stably expressing shRNA (as determined by GFP-expression) were pooled and maintained in puromycin. A pGIPZ lentivirus containing a non-targeting shRNA control (shNT, NT) with no homology to known mammalian genes was used as the negative control for the knockdown experiments.

Figure 16 shows the successful expression of GFP in all of the stably transfected foreskin fibroblasts from a TSC2 knockdown experiment. All cells remained permanently transduced for at least 11 passages. Western blot analysis confirmed that the transductions resulted in a more than 90% knockdown of TSC2 (Figure 17, top band). The same results were observed in dermal papilla cells transduced with TSC2 knock-down vectors (data not shown). In addition, the FLCN knock-down particles transduced 100% of fibroblasts (data not shown). Thus, virtually all cells were transduced with the vectors used for knocking down the genes of interest, and expression of the target genes was substantially reduced.

### B. EFFECT OF TSC2 KNOCKDOWN ON MTORC1 SIGNALING

Western blot analysis was performed on the stably transfected TSC2 knockdown foreskin fibroblasts to determine whether TSC2 expression was decreased sufficiently to observe activation of signaling through mTORC1. Figure 17 illustrates that TSC2 knockdown was accompanied by overactive mTORC1 signaling, as indicated by the hyperphosphorylation of ribosomal protein S6 (pS6) under serum-starved conditions. Total S6 was unchanged, and a tubulin control confirmed that comparable amounts of protein had been loaded in the different lanes. Similar results were obtained in duplicate transductions (data not shown). Thus, TSC2 expression was successfully knocked-down in a way that activated signaling through mTORC1.

These results demonstrate that wild-type mesenchymal cells may be modified to decrease TSC1/TSC2 function and increase mTORC1 function by decreasing TSC2 expression or by decreasing expression of a mimetic of TSC1/TSC2 function.

### C. ANALYSIS OF TRICHOGENESIS

Cells that induce the formation of hair follicles (trichogenic cells) express alkaline phosphatase. Alkaline phosphatase is a marker for dermal papilla cells, and dermal papilla cells with higher alkaline phosphatase activity have greater capacity for inducing hair follicles *in vivo.* Accordingly, alkaline phosphatase activity was measured in the cultured transduced cells to determine whether knockdown of TSC2 or FLCN expression resulted in increased numbers of trichogenic cells. As shown in Figure 18, human dermal papilla cells have high alkaline phosphatase activity during early passage, which rapidly decreases with subsequent passage. In contrast, transducing normal human fibroblasts with shTSC2, but not with non-targeting vector (shNT), increased alkaline phosphatase activity and this increase was maintained for several passages. Overall, alkaline phosphatase activity was higher in TSC2-null cells than TSC normal fibroblasts, indicating trichogenic activity in the TSC2-knockdown cells. Similar results were obtained when TSC2 was knocked down in human dermal papilla cells (Figure 19) and when FLCN was knocked down in dermal fibroblasts (Figure 20). Thus, cells with knockdown of TSC2 or FLCN showed increased cellular activity of alkaline phosphatase, a marker for trichogenic dermal papilla cells.

### D. ANALYSIS OF HAIR FOLLICLE NEOGENESIS IN HANGING BALL ASSAY

An *in vitro* hair follicle assay was used to determine the effect of knockdown of TSC2 on hair follicle organization and structure formation in hanging drop cell cultures. Briefly, hanging drop cultures of 30,000 cells were made from modified mesenchymal cells (neonatal foreskin fibroblasts (NFF) with knockdown of TSC2 (shTSC2) or non-template (NT) control) were combined with neonatal foreskin keratinocytes (NFK) (30,000 cells each per cluster) in 10 µl of a 1:1 mixture of dermal papilla medium and keratinocyte serum free medium. The clusters were incubated for 4 weeks as hanging drops in an incubator. The hanging drop cultures were analyzed with hematoxylin and eosin, and immunohistochemistry was performed with anti-pan-cytokeratin antibody to selectively identify keratinocytes.

Figure 21 compares the structures formed in hanging drop cultures using keratinocytes and fibroblasts transduced with TSC2-knockdown shRNA or non-template (NT) control. Clusters with TSC2-knockdown cells tended to show greater organization, with keratinocytes surrounding the fibroblasts (Figures 21A and 21C), whereas NT controls tended to remain disorganized (Figures 21B and 21D). Hair-fiber-like structures were observed in TSC2-knockdown cultures. In these clusters, refractile fiber-like structures formed that auto-fluoresced with the same green color as normal human hair (Figure 21E). These clusters with TSC2-knockdown cells may be implanted into skin or incorporated into grafts for hair follicle formation, as discussed below.

### E. ANALYSIS OF HAIR FOLLICLE NEOGENESIS IN DERMAL-EPIDERMAL COMPOSITE GRAFTS

Dermal-epidermal composites were generated using neonatal foreskin fibroblast (NFF) or dermal papilla cells transduced and stably expressing TSC2 knockdown vector. The cells were mixed with 1 mg/mL of rat tail collagen type 1 in 10% FBS/DMEM, and added to 6-well transwell plates at a density of 0.5 X 10⁶ cells per well. The dermal constructs were grown in 10% FBS/DMEM for 3 days. Five 30,000 cell hanging drop microspheres of NFF transduced with shTSC2 were placed gently on the dermal constructs and overlaid with 1 X 10⁶ keratinocytes. The dermal-epidermal composites were incubated for 4 days submerged in a mixture of DMEM and Ham's F12 (3:1) containing 0.1% FBS, after which the composites were brought to the air-liquid interface and the skin equivalents were fixed in 10% formalin after growing for either 4 or 8 days in DMEM and Ham's F12 (1:1) containing 1% FBS. The skin equivalents were then analyzed by hematoxylin and eosin (H&E), and immunohistochemistry was performed with anti-pan-cytokeratin antibody.

As shown in Figure 22, the dermal-epidermal composites composed of normal human keratinocytes and fibroblasts in a collagen gel formed a stratified squamous epithelium overlying the dermal equivalent, and the dermal epidermal junction was fairly straight without invaginations of keratinocytes. Using fibroblasts with knockdown of TSC2, however, tubular invaginations of keratinocytes formed by 4 days (Figure 22A), and by 8 days these invaginations had enlarged into multicellular tubes with a peripheral rim of pallisading keratinocytes (Figure 22B), similar in appearance to a developing hair follicle. Immunohistochemistry revealed that these structures invaginated into the dermal equivalent (Figures 21C-E), demonstrating that they were epithelial cells. Thus, knockdown of TSC2 promotes *in vitro* formation of hair-follicle-like structures in dermal-epidermal composites.

### F. ANALYSIS OF HAIR FOLLICLE NEOGENESIS IN MICE GRAFTED WITH DERMAL-EPIDERMAL COMPOSITES

Mouse grafting experiments were performed to determine if knockdown of TSC2 promotes formation of hair follicles *in vivo.* Briefly, neonatal foreskin fibroblast and dermal papilla were transduced and selected for either TSC2 knockdown vector or nontargeting vector as discussed above. The cells were mixed with 1 mg/ml of rat tail collagen type 1 (BD Biosciences, Bedford, MA) in 10% FBS/DMEM, and added to 6-well transwell plates (Corning Incorporated, Corning, NY) at a density of 0.5 X 10⁶ cells per well. The dermal constructs were grown in 10% FBS/DMEM for 3 days and overlaid with 1 X 10⁶ keratinocytes . The dermal-epidermal composites were incubated for 2 days submerged in a mixture of DMEM and Ham's F12 (3:1) (GIBCO/Invitrogen, Grand Island, NY) containing 0.1% FBS, after which the composites were brought to the air-liquid interface and grown for another 2 days in DMEM and Ham's F12 (1:1) containing 1% FBS before grafting.

Female 6-8 week old Cr:NIH(S)-nu/nu mice (FCRDC, Frederick, MD) were anesthetized with a mixture of O₂ and isoflurane (2-4%). The grafting area on the back of the mouse was carefully estimated, and skin was removed using curved scissors. Composites were placed on the graft bed in correct anatomical orientation, covered with sterile petroleum jelly gauze, and secured with bandages. The bandages were changed at 2 weeks and removed after 4 weeks. In total, 39 mice were grafted (6 mice - neonatal foreskin fibroblast with non-targeting control shRNA; 14 mice - neonatal foreskin fibroblast with TSC2 shRNA; 6 mice - dermal papilla with non-targeting control shRNA; and 13 mice - dermal papilla with TSC2 shRNA). In 6 mice sampled 10 weeks after grafting, shTSC2 fibroblasts induced hair-follicle-like structures in one of three mice sampled, and shTSC2 dermal papilla cells induced hair follicles in one of three mice sampled (Figure 23). Results are pending for the other mice at the time of filing.

### G. CONCLUSIONS

The results presented in this Example using lentiviral transduction of shRNA provides a proof-of-concept that loss of TSC2 or FLCN enhances the trichogenic capacities of fibroblasts.

### EXAMPLE 3: ISOLATION OF MESENCHYMAL CELLS FROM ADNEXAL TUMORS OR NORMAL HUMAN SKIN

Mesenchymal cells may be isolated from one or more of the following sources: patient skin or mucosa for autologous cells; donor skin or mucosa for allogeneic cells; normal skin or mucosa; skin with an adnexal tumor; and other tissues (e.g. fat, bone marrow, etc.). Fibroblasts may be isolated by enzyme digestion if the sample size is sufficiently large (*i.e.,* greater than or equal to 1 cm³).

### A. CELL MIGRATION METHOD

Cells may be isolated from skin samples or skin tumors using a cell migration method. To isolate mesenchymal cells by cell migration from explants, skin samples are cut into small pieces and transferred into 35 or 100 mm sterile dishes containing 1 or 5 mL of 10% FBS/DMEM or mesenchymal stem cell growth medium (MSCGM; Lonza Group Ltd, Switzerland). The plates are incubated in a 5% CO₂ incubator at 37°C. The medium is changed twice a week until a substantial number of mesenchymal cells are observed. The cells migrating out of tissue fragments are regularly monitored using an inverted microscope. Mesenchymal cells are subcultured when they occupy most of the dish surface between explants (approximately 2-3 weeks after start of the culture). The cells are harvested for sub-culture and the small tissue pieces are transferred to fresh dishes for isolating more cells, repeating the transfer of explants more than 10 times until cells no longer migrate from the tissue. Cells from each transfer are stored in liquid nitrogen at early passage.

### B. Two ALTERNATIVE CELL DISSOCIATION METHODS

Cell dissociation from skin samples or skin tumors may be used to isolate mesenchymal cells. According to this method, the skin sample (1 X 1 cm) is treated overnight in 60 mm dishes with 3 ml of dispase at 4°C. Alternatively, samples may be treated with 0.25% trypsin for 30 minutes at room temperature. The dermis is separated from the epidermal sheet and cut into small pieces. The sample is incubated in a 50 ml centrifuge tube with 10 ml of enzyme solution (HEPES containing Richter's improved MEM insulin medium (RPMI), supplemented with 1 mM sodium pyruvate, 2.75 mg/mL bacterial collagenase, 1.25 mg/mL hyaluronidase, and 0.1 mg/mL DNase I) at room temperature for 3 h. After incubation, the tissue is mechanically dissociated by pipetting up and down 10 times. The cell suspension is filtered through a sterile nylon mesh to remove tissue fragments and centrifuged at 400 x g for 10 min at room temperature. The supernatant is discarded, and the cell pellet is resuspended in 10 ml of medium (such as mesenchymal stem cell growth medium or DMEM plus 10%FBS) and transferred into a 75-cm² culture dish. The cells are cultured in a 5% CO₂ incubator at 37°C, and the medium is changed 24 h later to remove nonadherent material.

An alternative approach for cell dissociation from skin samples or skin tumors is to wash dermis three times in PBS, mince into small pieces (2-3 mm³) and digest in PBS (calcium and magnesium free) solution containing Clostridium histolyticum collagenase (CHC) extract (Worthington Biochemical Corp., Lakewood, NJ) in 4 ml/g tissue at 37°C under gentle shaking conditions (50-55 rpm). After the incubation, the digest is filtered through an open filter chamber (NPBI, Emmer-Compascuum, The Netherlands), and the filter is rinsed twice with 10 ml culture medium. The wet tissue weight is measured before and after digestion to calculate the tissue digestion efficiency. The cell suspension is centrifuged at 250 × g for 10 min, the supernatant is aspirated, and cells are resuspended in cell culture medium. Using a counting chamber, the cell concentration is determined three times in three independently taken samples (isolation cell yield), and viability is assessed by trypan blue (Sigma) exclusion. Cells are seeded in culture at a density of 500 cells/mm² or 1000 cells/mm² (5 × 10⁴ or 10 × 10⁴ cells/cm² ) in three separate flasks. After 24 hours, the percentage of attached cells is assessed using an inverted microscope connected to a video camera with frame grabber image-printer.

### C. ISOLATION OF DERMAL PAPILLA (DP)/DERMAL SHEATH (DS) MESENCHYMAL CELLS

Dermal papilla cells may be isolated from samples of human scalp by microdissection, followed by treatment with collagenase for 30 minutes with mild agitation at 37°C. The enrichment for dermal papilla cells may be confirmed using toluidine blue staining, or by examining the cells for intranuclear rodlets. Cells may be grown in a 1:1 mixture of Chang medium and keratinocyte-conditioned medium, changed every 2-3 days.

Dermal sheath cells may be isolated from normal adult human skin by dicing human skin samples and then enzymatically dissociating the samples with collagenase. Enzymatic dissociation yields more cells in a shorter time period than using skin explants. Moreover, cell viability and proliferation are sufficient to populate dermal equivalents for autologous grafting. Dermal sheath cells may be identified by incubating the cells with FITC labeled anti-CD10 antibody for 30 min. at 4°C, followed by cell sorting.

DP and DS cells may also be isolated by rinsing normal human scalp tissue (1 X 1 cm) in Hanks buffer three times, each for 10 min, cutting into strips about 0.3-0.5 cm in width, and cutting off at the interface of dermis and subcutaneous fat. The subcutaneous tissue is incubated with 3-5 ml of 0.5% dispase (Sigma Chemical Co. St. Louis, MO) at 4°C for 16-18 h. The hair follicles are pulled out from cutaneous fat. The epithelia are extruded out from the dermal sheaths by applying gentle pressure with the tip of a pair of microforceps. Then the dermal sheaths are incubated in 0.2% collagenase D (Boehringer Mannheim, Germany) in Engle's minimum essential medium (MEM) (ICN Biomedicals, Inc., Aurora, OH, USA) containing 10% FBS at 37°C for 6-8 hours until the stalk of dermal papilla is digested under microscope control. When the fibrous sheaths are digested entirely and the papilla just begins to be digested, the enzyme digestion is stopped. Hanks is added and the suspension is centrifuged for 5 minutes at 2000 rpm, which is repeated three times. The pellet is resuspended and centrifuged at low-speed at 200 rpm for 5 minutes and repeated three times leaving DS cells in the supernatant for culture. Dermal papillae are completely isolated out from residue with low-speed centrifugation. The final dermal papilla pellet is resuspended without any isolated cells, and transferred into a 25 ml flask containing medium for explant culture in MEM medium with 10% FBS. The cultures are incubated for 5 days, and the medium is changed twice weekly.

The isolated dermal papilla and dermal sheath cells may then be incubated in any suitable medium to test for induction or maintenance of dermal papilla and dermal sheath markers.

### D. ISOLATION OF MESENCHYMAL CELLS USING METHODS FOR OBTAINING SKIN-DERIVED PRECURSORS OR NEURAL CREST CELLS FROM SKIN

Human mesenchymal cells are isolated using similar methods as skin-derived precursors. (Biernaskie, J.A. et al., Isolation of skin-derived precursors (SKPs) and differentiation and enrichment of their Schwann cell progeny, Nature protocols 1(6): 2803-2812 (2006)). Briefly, human skin samples or skin tumors are washed in HBSS, cut into small pieces measuring 3-5 mm² and digested in a 100 mm (10 cm) plastic tissue culture dishes filled with 25 ml of Blendzyme solution (Roche) for 24-48 hours at 4°C. The epidermis is peeled away from the underlying dermis using fine forceps, and the isolated dermal tissue is minced into small, 1-2 mm² pieces using a razor blade. These small pieces of human dermis are collected into 15-ml conical tubes containing 5-10 ml of fresh Blendzyme solution. DNasel (one 400 µl aliquot) can also be added to the suspension to reduce aggregation of cells. For most efficient digestion of the tissue, the sample may be gently agitated for 1-2 h at 37°C. Upon completion of the digestion, 20 ml wash medium plus 10% FBS is added to inactivate the Blendzyme. The tissue samples are centrifuged at 1,200 r.p.m. for 6-8 min to pellet all cells and skin pieces. The supernatant, which contains the medium plus enzyme, is discarded. Fresh wash medium (3-5 ml) is added, and the pellet is dissociated using a 10 ml disposable plastic pipette. The suspension is centrifuged for 20 seconds to pellet large pieces of skin at 1,200 r.p.m. The supernatant is collected into a 50 ml collection tube and kept on ice. The tissue pellet is dissociated in fresh medium for repeating the trituration step until the tissue pieces become thin and cells can no longer be liberated. The dissociated cell suspension is passed through a 70 µm cell strainer into a 50 ml conical tube and centrifuged at 1,200 r.p.m. for 7 min. The cell pellet is resuspended in wash medium plus 2% B27 supplement (Invitrogen). Resuspension volumes range from 5 to 20 ml of medium depending on the size of the pellet, and can be adjusted to simplify quantification of cell yield. The dissociated dermal cells are diluted into 30 ml of proliferation medium (DMEM/F12 (3:1) containing 0.1% penicillin/streptomycin, 40 µg/ml fungizone, 40 ng/ml FGF2, 20 ng/ml EGF, 2% B27 supplement) for a 75 cm² flask and 10 ml for a 25 cm² flask. The cells are cultured for 7-14 days without passaging for the formation of spherical colonies, and the medium is changed every 4-5 days.

### EXAMPLE 4: GENERATION OF MODIFIED MESENCHYMAL CELLS

### A. GENE KNOCKDOWN

To knockdown gene expression, for example, of TSC1, TSC2, CYLD, LKB1, FLCN, MEN1, NF1, PTEN, PRAS40, 4E-BP1, GSK3, or Deptor, lentiviral particles from custom cloned short hairpin RNA (shRNA) or non-target shRNA control in pLKO.1-puro-CMV-tGFP vector (Sigma) may be used according to the manufacturer's instructions. For a pilot experiment, cells are plated in 6-well plates (2 X10⁵ cells/well) and cultured in 10% FBS/DMEM for 24 hours. The medium is replaced by 2 ml of fresh 10% FBS/DMEM containing shRNA for the indicated gene or control shRNA lentiviral particles (0, 1, 2, 5, 10 or 20 MOI) plus 8 µg/ml of hexadimethrine bromide and incubated overnight. The medium containing the viral particles is removed, and the cells are cultured in fresh complete medium for 24 hours before selecting with puromycin for 10-14 days (the titration may be done before use by treating 1 X10⁴ cells in 96 well plates with 0.5-10 µg/ml of puromycin). The medium with puromycin is replaced every 3 days. The puromycin resistant cell colonies are collected and cultured for further analysis. Gene expression is measured by qRT-PCR or Western blot. To evaluate the cells following gene knockdown, the transduced cells are pooled after puromycin selection. The levels of target protein in the transduced cells are measured by western blot and compared to control shRNA cells at passage 1, 10, 20, 30, and 40.

Alternatively, or in addition, gene therapy methods may be used to knockdown gene expression. For example, zinc finger nucleases may be used to generate targeted double-strand breaks in the TSC1 or TSC2 genes, or in the genes encoding proteins that stimulate TSC1/TSC2 function. (See, e.g., Lee et al., Genome Res., 20:81-89 (2010); Händel et al., Curr. Gene Ther., 11:28-37 (2011); Holt et al., Nat. Biotechnol., 28:839-47 (2010); and Ledford, Nature, 471:16 (2011).) Briefly, isolated cells may be treated with CompoZr® Zinc Finger Nucleases (ZFNs) (Sigma Aldrich) (or other suitable nucleases) that typically target the first 2/3 of the coding region of the gene of interest. ZFNs are designed in silico and tested in a cellular assay to identify ZFNs that cleave the target site, and a pair of ZFNs is selected for use. ZFNs may be delivered to the cells using nucleofection, electroporation, or lipid-based transfection of ZFN plasmids or mRNA transcripts. ZFNs may also be delivered using a viral vector such as lentivirus. For nucleofection, 5 x 10⁶ to 10 x 10⁶ cells at about 80% confluency are trypsinized and transfected with about 5 µg of each ZFN-encoding plasmid using Nucleofector kits (Amaxa Biosystems) according to the manufacturer's instructions. After transfection, cells are maintained in media such as DMEM with 10% FBS. A mismatch-specific cleavage assay (such as the Surveyor endonuclease assay (Cel-1; Transgenomics) in which Cel-1 cleaves heteroduplexes of wild-type and mutated DNA strands following denaturation-renaturation) may be used to determine the proportion of cells with the knockout. To obtain pure or enriched populations of cells with the knockout, the cells may be cloned. Alternatively, a gene such as GFP or a puromycin plasmid may be inserted by homologous recombination at the time of TSC1 or TSC2 knockout with ZFNs, allowing the cells to be sorted using FACS or enriched using antibiotic selection. The levels of target protein in the treated cells may be measured by western blot and compared to control untreated cells.

The transduced cells may also be analyzed for the effect of the knockdown on mTORC1 signaling. This may be accomplished by measuring phospho-S6 expression in the transduced cells by western blot and comparing to control shRNA cells at passage 1, 10, 20, 30, and 40. Since Wnt signaling is active during hair morphogenesis, the effect of the knockdowns on Wnt signaling may also be evaluated. This may be done by measuring the level of beta-catenin and GSK3 by western blot with specific antibodies (Cell Signaling Technology, Inc). The WNT network is active in the epidermal placode during development, and WNT proteins are thought to be part of the signal that triggers the dermal condensate to form. (Kishimoto, J. et al., Wnt Signaling Maintains the Hair-Inducing Activity of the Dermal Papilla, Genes & Development 14 (10):1181-1185 (2000); Shimizu, H. et al., Wnt Signaling Through the Beta-Catenin Pathway is Sufficient to Maintain, but Not Restore, Anagen-Phase Characteristics of Dermal Papilla Cells, The Journal of Investigative Dermatology 122 (2):239-245 (2004).)

### B. GENE INDUCTION

Human mesenchymal cells may be transfected for stable expression of mTOR network activating or hair follicle related genes (*e.g*., Ras, Raf, Mek, Erk, Rsk1, PI3K, Akt1, Akt2, Akt3, Rheb, mTOR, Raptor, Rictor, mLST8, S6K1, ribosomal protein S6, SKAR, SREBP1, elF4e, IKKbeta, Myc, Runx1, or p27) under the control of a constitutively active promoter using standard procedures. (Ortiz-Urda, S. et al., Injection of Genetically Engineered Fibroblasts Corrects Regenerated Human Epidermolysis Bullosa Skin Tissue, The Journal of Clinical Investigation 111(2): 251-255 (2003).) Briefly, the genes may be introduced into *Streptomyces* phage φC31 integrase-assisted stable integration plasmid with CMV *IE* promoter by inserting the 285-bp φC31 attB sequence as a BglII fragment into the BglII sites of the backbone vector pcDNA3.1/zeo creating the plasmid pcDNAattB. IRES and blastocidin resistance sequences may be removed from a pWZL Blast vector as a blunted SnaBI-NheI fragment, which is inserted into the EcoRV/XbaI sites of pcDNAattB, creating the plasmid pcDNAattB-IB. Subsequently, one of the indicated genes is amplified and cloned with a lacZ gene as an EcoRI, HindIII/EcoRI, and EcoRI (blunt)/BamHI fragments into the EcoRI, HindIII/EcoRI, and HindIII (blunt)/BamHI sites, respectively, of pcDNAattB-IB. This procedure creates the transfer plasmids comprising the gene-of-interest-attB and placZ-attB. The constructed vectors are then cotransfected with a φC31 integrase-encoding plasmid into human mesenchymal cells. Briefly, human mesenchymal cells are transfected with pInt and the gene-of-interest -attB and placZ-attB using a modified polybrene shock. Primary human mesenchymal cells are cultured in 35-mm plates to 70-80% confluence then transfected by modified polybrene shock. For polybrene transfection, 760 ml of growth media is mixed with the plasmid to be transfected and this mixture is vortexed vigorously. 3.8 ml of 1 mg/ml hexadimetherine bromide (Aldrich Chemical Co., Milwaukee, WI) in HBSS are added and again vortexed. This mixture is overlaid on the cells for 6 hours. A 28% DMSO (Sigma Chemical Co., St. Louis, MO) in growth media mix is applied to the cells after the media has been aspirated. The cells are incubated for 90 seconds before the DMSO is aspirated and replaced with PBS containing 10% bovine calf serum. The plates are rinsed twice and the cells are incubated with fresh growth medium overnight at 37°C. For selection, 3 days after transfection cells are subjected to 10 day of blasticidin (4 µg/ml) in culture media. Efficiency of gene transfer is verified by immunofluorescence microscopy and immunoblot analysis. After 10 day selection, mesenchymal cells colonies are trypsinized and subcloned at limiting dilution to obtain highly proliferative clones.

### C. PROTEIN DELIVERY TO CELLS IN VITRO

The mTOR network activating or hair follicle related proteins may be delivered into human mesenchymal cells using methods as described. (Weill, C.O. et al., A Practical Approach for Intracellular Protein Delivery, Cytotechnology 56 (1), 41-48 (2008).) Cells are plated in order to reach approximately 70-80% confluency the day of protein delivery. For one well of a 24-well plate, 0.5-8 µg of purified protein is diluted in 100 µl of Hepes buffer (20 mM, pH 7.4) in a 1.5 ml microcentrifuge tube, under sterile conditions. In each tube, 1-8 µl of protein delivery reagent PULSin™ (Illkirch, France) are added to the protein solution. After a brief homogenization with a vortex, the protein/reagent mix is incubated for 15 min at room temperature to allow complex formation. The cells are washed with 1 ml of PBS, and 900 µl of culture medium without serum is added to each well. After addition of the complexes into each well, the plate is gently mixed and further incubated at 37°C. After 4 hours, the incubation medium is removed and replaced with 1 ml of fresh complete medium (containing serum). Protein delivery is analyzed immediately or at later time points by immunocytochemistry.

### EXAMPLE 5: ENRICHMENT OF CELLS WITH HAIR INDUCTIVE PROPERTIES

### A. SEPARATION BASED ON CELL MARKERS

The skin tissue is prepared as described in either protocol in Example 3B. Cells are harvested after 7 days using a solution containing 0.25% trypsin and 5 mM EDTA (Sigma) and enriched for hair inductive cells based on cell marker, CD-10. FITC labeled anti CD-10 antibody (eBioscience) is incubated with the fibroblast for 30 min at 4°C. The cells are sorted using BD Biosciences FACSAria Cell Sorter after washing the cells with PBS with 0.1 % BSA.

Alternatively, the cells are labeled with anti-CD10/RPE antibody (10 ml for 1 X 10⁶ cells; DAKO, Glostrup, Denmark; clone SS2/36) for 30 min at room temperature. Labeled cells are washed with PBS, 2% bovine serum albumin, incubated with anti-PE micro beads (10 ml/10⁶ cells; Miltenyi Biotec, Bergisch Gladbach, Germany) for 30 min at room temperature and separated by MACS columns placed in a MiniMACS Separator (Miltenyi Biotec) according to manufacturer's protocol.

### B. SEPARATION BASED ON ENHANCING GROWTH OF DESIRED CELLS OR STUNTING/KILLING UNDESIRABLE CELLS

Growth factors such as BMP2, 4, 5, or 6, Wnt-3a, Wnt-10b, FGF2, KGF, or others may be added to the growth medium to maintain and enrich the hair inductive cells including dermal papilla cells. Dermal papilla cells are cultured in the presence of an increased level of WNT protein or an agent that mimics the effects of WNT-promoted signal transduction. This method is based upon the discovery, discussed above, that WNT signaling is active during hair morphogenesis. (Kishimoto, J. et al., Wnt Signaling Maintains the Hair-Inducing Activity of the Dermal Papilla, Genes & Development 14 (10):1181-1185 (2000); Shimizu, H. et al., Wnt Signaling Through the Beta-Catenin Pathway is Sufficient to Maintain, but Not Restore, Anagen-Phase Characteristics of Dermal Papilla Cells, The Journal of Investigative Dermatology 122 (2):239-245 (2004).)

An alternative approach is to prepare conditioned medium containing human Wnt-3a protein. Mouse L cells are cultured in a 1:1 mixture of DMEM and HAM F12 medium supplemented with 10% FCS and antibiotics at 37°C. For establishment of L cells transfected with Wnt-3a cDNA, pGKWnt-3a may be constructed by inserting the human Wnt-3a cDNA, whose expression is driven by a promoter of rat phosphoglycerokinase gene (PGK promoter) and terminated at a transcriptional terminator sequence of the bovine growth hormone gene, into pGKneo, containing the neomycin phosphotransferase gene (neo) driven by the PGK promoter. pGKWnt-3a is introduced by the calcium phosphate method into L cells, which are plated in 60 mm culture dishes at a density of 1.5 X 10⁶ cells/plate 1 day before the DNA addition. To these cultures, 400 mg/mL of G418 are added 2 days after transfection. Stably transfected clones are then selected and sub-cultured. To collect the conditioned medium (CM) from cultures of Wnt-3a-producing L cells, these cells are seeded at a density of 1 X 10⁶ cells in a 100 mm dish containing a 1:1 mixture of DMEM and HAM F12 supplemented with 10% FCS, and cultured for 4 days. The conditioned medium is harvested, centrifuged at 1000 g for 10 min, and filtered through a nitrocellulose membrane. As a control, conditioned medium may be prepared from L cells transfected only with pGKneo and cultured under the same conditions as above. The conditioned medium may be used to obtain Wnt-enhanced hair inductive cells. Briefly, 100-1000 of skin mesenchymal cells are plated on 100 mm dishes in DMEM plus 10% FBS and cultured for 24 hours. In the next day, the medium is replaced by L cell conditioned medium containing Wnt2a protein and cultured for 2 weeks with medium changes every 3 days. After 2 weeks, the cell clones are collected for further analysis or injection to human skin.

### EXAMPLE 6: MAINTENANCE OF HAIR INDUCTIVE PROPERTIES DURING PROPAGATION USING SPECIALIZED MEDIA OR GROWTH FACTORS

The hair follicle inductive potential of human DP cells may be maintained in one of following media:

Chang medium (Chang H. C. et al., "Human amniotic fluid cells grown in a hormone-supplemented medium: suitability for prenatal diagnosis," Proc Natl Acad Sci U S A 79(15): 4795-9 (1982)): Briefly, the basic culture medium [serum free (SF) medium] is a 1:1 mixture of Dulbecco-Vogt modified Eagle's medium (DVME medium) and Ham's F12 medium (F12 medium) supplemented with 15 mM Hepes and 1.2 g of NaHCO3, 40 mg of penicillin, 8 mg of ampicillin, and 90 mg of streptomycin per liter. The SF medium plus 10 growth-promoting factors is termed H medium (supplemented medium). The growth promoting factors added are: transferrin (5 µg/ml), selenium (20 nM), insulin (10 µg/ml), triiodothyronine (0.1 nM), glucagon (1 µg/ml), fibroblast growth factor (10 ng/ml), hydrocortisone (1 nM), testosterone (1 nM), estradiol (1 nM), and progesterone (1 nM).

Keratinocyte-conditioned medium (KCM): To collect KCM from keratinocyte culture, 10⁶ of the cells are plated on 100-mm dish and cultured for 3-5 days in 10 ml of medium (50% DMEM plus 50% KSFM in the absence of FBS or growth supplements). The conditioned medium is collected and used for culture of skin mesenchymal cells for 2-4 weeks before injection to human skin.

Application of commercially available medium or growth factors: Mesenchymal stem cell medium (Invitrogen), or human follicle DP cell growth medium (PromoCell) is used for culture of human DP cells. Other growth factors, such as BMP6 (10 ng/ml), FGF-2 (10 ng/ml, BioVision) or leptin (0, 10, or100 ng/ml, Sigma-Aldrich) may be used for DP cell culture.

Use of small molecule inhibitors: To maintain the hair inductive mesenchymal cells, GSK-3 inhibitor, BIO (Calbiochem, La Jolla, CA) is added to culture medium at 1.5 µM in 100-mm of dish. The cells are sub-cultured and passaged for more than 2 weeks before further analysis or injection to human skin.

### EXAMPLE 7: ISOLATION OF EPIDERMAL CELLS

Epidermal cells may be isolated from the following sources: patient skin or mucosa (autologous), donor skin or mucosa (allogeneic), epidermal cell lines, epidermal cells derived from stem cells, and primary or passaged epidermal cells.

To isolate keratinocytes, human neonatal foreskin or adult skin tissues are treated with dispase at 4°C for overnight. The epidermal sheet is separated from dermal sheet and subsequently digested with 0.05% trypsin, 0.53 mM EDTA at 37°C for 20 min. The cells are collected and plated on tissue culture dishes in keratinocyte serum-free media supplemented with bovine pituitary extract and recombinant epidermal growth factor. This method parallels the cell dissociation method discussed above for mesenchymal cells (*i.e*., the dermal section is used for the mesenchymal cells, and the epithelial section is used for the epidermal cells).

Alternatively, stem cells may be used to generate epidermal cells by inducing the stem cells to differentiate into epidermal cells using the following protocol. Previous studies indicated that when stem cells are plated onto BM-coated dishes, they give rise to epithelial sheets that are capable of differentiating into keratin 14 (K14)-positive cells. These studies also suggested that such cultures contain epidermal progenitor cells that are maintained in secondary cultures called epithelial progenitor cells (EPCs). When cultured at high density, EPCs progress along the hair follicle differentiation pathway to express hair keratins, as determined by indirect immunofluorescence with antibodies. To induce stem cells to differentiate into epidermal cells, stem cells growing on 35-mm tissue culture dishes are coated with Matrigel (1 mL/35-mm dish, approx 0.1 mg) for 30 min at room temperature, then the Matrigel is gently replaced with 15% DMEM. On day 4, the cells are treated with 0.25% trypsin-EDTA for 2 to 3 min in the incubator. The cells are transferred into a 15-mL Falcon tube and an aliquot is removed to count the total available cells. The cells are centrifuged at 700xg for 2 to 3 min for pellet formation. While spinning, the cells are counted using a Coulter Counter. The pellet is resuspended with 15% DMEM and diluted appropriately in order to plate 10⁶ cells/35-mm dish. For immunofluorescence, the cells may be plated on glass 22 × 22 mm coverslips.

### EXAMPLE 8: ENRICHMENT OF EPIDERMAL CELLS WITH ABILITY TO DIFFERENTIATE INTO HAIR FOLLICLES

### A. CELL ADHESION

The stem cell populations of neonatal or adult human skin can be enriched by rapid adherence according to previously reported methods. Briefly, 100mm bacteriological plastic dishes are coated overnight with 100 µg/ml of type IV collagen, incubated with 0.5 mg/ml heat denatured BSA at 37°C for 1 hour, and washed in serum-free medium. Keratinocytes are resuspended in serum-free medium at a density of 1- 5 X 10³ cells/ml. Ten ml of cell suspension is added to type IV collagen coated dishes, and the dishes are returned to the incubator for 5 microscope. Rapidly adherent cells are harvested and re-plated at 1 X10⁵ cells for further culture in FAD (DMEM/F12 3:1, v/v, Gibco) supplemented with 0.4 mg/ml hydrocortisone (Sigma), 5 mg/ml transferrin (Sigma), 5 mg/ml insulin (Sigma), 100 IU/ ml penicillin, 100 mg/ml streptomycin, 10% FCS, 10 ng/ml epidermal growth factor (EGF) (Sigma), and 10 ng/ml basic fibroblast growth factor (bFGF) (Gibco). The dishes are placed in an incubator at 37°C, 100% humidity, and 5% CO₂, and the medium is changed every 2-3 days. Epidermal stem cells that are more adherent to the culture dish coated with extracellular matrix have more potential ability to form into hair follicles.

### B. CELL SORTING

Bulge cells may be isolated with magnetic beads systems. Two magnetic beads systems are combined to isolate bulge ORS cells from the mid-follicle suspension. First, hair follicle cells are stained with the cocktail of PE-conjugated anti-human CD24, CD34, CD71, and CD146 antibodies (BNC) for 20 min. at 4°C. After washing, follicle cells are incubated with anti-PE microbeads (Miltenyi Biotec) for 25 minutes at 4°C. Then, PE-positive non-bulge cells are removed with the magnetic separations using mini-MACS MS columns (Miltenyi Biotec). The removal procedures are repeated 3-5 times to ensure maximum depletion. Next, mid-follicle cells are incubated with purified anti-human CD200 mouse mAb at 4°C for 20 minutes, washed, and incubated with Dynabeads M-450 sheep anti-mouse IgG magnetic beads (Dynal Biotech) at 4°C for 30 minutes with tilting. Then, positive selection is performed with a MPC-L magnetic particle concentrator (Dynal Biotech) to obtain CD200-positive cells. CD59-positive cells may be similarly collected as a positive selection control. It is expected that preparations of epidermal cells that are enriched for bulge cells will have greater capacity to form hair follicles.

### EXAMPLE 9: PREPARATION OF CELLS FOR GRAFTING

### A. SKIN SUBSTITUTES

Three-dimensional *in vitro* constructs are prepared for grafting using established methods modified as described herein. Briefly, mesenchymal cells are mixed with 1 mg/ml type I collagen (rat or bovine, as described below) in 10% FBS/DMEM, and added to 6 well transwell plates (Corning Incorporated, Corning, NY) at a density of 15 x 10⁶ cells per mm² (1.5 x 10⁵ cells per cm²). The dermal equivalents are cultured in 10% FBS/DMEM for 4 days before aliquoting 1 X 10⁶ keratinocytes on top. The constructs are cultured submerged for 2 days in a mixture of DMEM and Ham's F12 (3:1) (GIBCO/Invitrogen, Grand Island, NY) containing 0.1% FBS, after which the keratinocytes are brought to the air-liquid interface and cultured in a mixture of DMEM and Ham's F12 (3:1) containing 1% FBS for another 2 days before grafting.

### B. CELL CLUSTERS

Cell aggregates for injection may be formed using the hanging droplet method. (Qiao J. et al., "Hair follicle neogenesis induced by cultured human scalp dermal papilla cells," Regen Med 4(5): 667-76 (2009).) Briefly, a mixture of human mesenchymal cells and keratinocytes (10:1, 5:1, 1:1, 1:5 or 1:10) is suspended in Chang medium containing 0.24% methylcellulose. The cells are applied in 20-µl droplets (each droplet contains 4 × 10⁴ cells) in the bottom of a 100-mm petri dish. The petri dish is inverted such that the droplets are hanging upside down. The suspended droplets are incubated in a 37°C, 5% CO₂ incubator. Aggregate formation is completed within 18-20 h. Upon formation, aggregates are transferred individually to wells of a 96-well round-bottom assay plate containing 150 µl Chang medium. The wells are precoated with 0.24% methylcellulose medium to prevent adherence of proto-hairs. The culture medium is changed every 2-3 days.

### C. MICROSPHERES

Biodegradable microspheres for injection are fabricated from 75:25 PLGA (molecular weight = 100,000 Da, Birmingham Polymers, Birmingham, AL) using a conventional oil/water emulsion and solvent evaporation/extraction method. In brief, 600 mg PLGA is dissolved in 12 ml of methylene chloride, added to 400 ml aqueous solution of 0.5% (w/v) polyvinyl alcohol (molecular weight = 30,000-70,000 Da, Sigma), and stirred vigorously at room temperature overnight. The microspheres are collected by centrifugation, washed three times with distilled water, and strained to a size of 50-200 µm in diameter. The microspheres are lyophilized and sterilized with ultraviolet light for 6 hours. Human mesenchymal cells (2.5 × 10⁷ cells) and keratinocytes (6 × 10⁶ cells) are placed with PLGA microspheres (1 µg microspheres/10⁵ cells) in a spinner flask (Bellco Glass Inc., Vineland, NJ) containing 30 ml of serum-free KGM containing 10 ng/ml of EGF for keratinocytes, or DMEM/F12 containing 10% (v/v) FBS for mesenchymal cells, and cultured at 50 rpm for 2 weeks. The medium is exchanged every other day. Cell aggregates are allowed to settle down, 16 ml of the culture supernatant is collected and centrifuged, 15 ml of the supernatant is removed, and 15 ml of fresh medium is added to the centrifuged cells in 1 ml of remaining supernatant. The cells in fresh medium are transferred to the spinner flasks. Alternatively, clusters of cells may be formed by suspending the cells in sodium alginate and then forming spherical droplets using a high-voltage electric droplet generator as described in Lin C.M. et al., "Microencapsulated human hair dermal papilla cells: a substitute for dermal papilla?," Arch Dermatol Res. 300(9):531-5 (2008).

### EXAMPLE 10: EVALUATION OF SKIN SUBSTITUTES OF THE INVENTION FOR BIOMECHANICAL PROPERTIES, WOUND HEALING, AND LONG TERM HAIR FOLLICLE REGENERATION

The skin substitutes of the invention may be tested for biomechanical properties, wound healing, and long term hair follicle regeneration.

Biomechanical properties include skin barrier function, sebum secretion, skin tensile strength, transepidermal water loss, and skin electrical capacitance. Skin capacitance may be measured using a Corneometer CM 825 PC (Courage & Khazaka Electronic GmbH, Cologne, Germany). Transepidermal water loss may be measured by a Tewaeter TM 300 (Courage & Khazaka Electronic GmbH, Cologne, Germany). To assess the activity of sebaceous glands, one may measure the expression of human sebum lipid and proteins using oil red O staining and real-time PCR of laser microdissected material. Total RNA may be isolated from laser-microdissected sebaceous glands and the mRNA reverse transcribed. To measure skin tensile strength, a small strip of graft obtained after sacrificing the animal may be placed in a tensiometer (Instron 5542 tensiometer, Insron, Canton, MA) and peak breaking force measured. Briefly, a small portion of the tissue strip (approximately 0.5 cm of incision) is oriented in the jaws of the tensiometer perpendicular to the line of the incision. Peak breaking forces are measured and converted to tensile strength values (kilogram force per square centimeter) by dividing the breaking force by the cross-sectional area of the tissue that broke. The same procedure may be used to measure the tensile strength of plucked hairs.

To assess wound healing, wounds may be created in the grafts four to six weeks after grafting. The wound may be bandaged, and the rate of wound healing determined by serial photography every 1-2 days with a scale to measure wound contraction and reepitheliazation. Sections of grafts may be harvested and stained with Masson's trichome and evaluated histologically for wound and scar area, dermal thickness, and epidermal thickness by sectioning through the center of the wound. Histomorphometric measurements of the wounds may be performed, and qualitative assessments made of inflammatory cell infiltrate, fibroblast proliferation, collagen formation, and angiogenesis. Immunohistochemistry may be used to identify human cells, cell proliferation (Ki-67), and numbers of myofibroblasts.

Hair cycling may be documented by repeated observations throughout the hair cycle. However, since the hair cycle takes about 100 days for non-scalp skin and up to a few years for the scalp, the experimental progress may be speeded by hair plucking (*e.g.,* using wax). Hair plucking is a well-proven method for inducing hairs to re-enter anagen. The hair-plucking assay may be combined with an assessment of the epidermal stem-cell compartment for the presence of label-retaining cells in the epidermis and bulge region of the hair follicle. BrdU may be injected intraperitoneally twice daily for 6 days beginning at the completion of follicular neogenesis. At 10-14 weeks, the hairs on one half of the graft may be plucked. These studies will allow determination of the presence and location of label-retaining epidermal stem cells and their response to plucking skin with or without hair follicles.

### EXAMPLE 11: GRAFTING PROCESS

### A. PLACEMENT OF COMPOSITE

Mice are grafted in a horizontal laminar flow hood using 6-8 week old female Cr:NIH(S)-nu/nu mice (FCRDC, Frederick, MD) anesthetized using inhalant anesthesia with a mixture of O₂ and isoflurane (2-4%). The grafting area on back of the mouse is carefully estimated, and skin is removed using curved scissors after washing with povidine and 70% ethanol. Constructs are placed on the graft bed in correct anatomical orientation, covered with sterile petroleum jelly gauze, and secured with bandages. The mice are transferred back to the sterile cages after reawakening. The bandages are changed at 2 weeks and removed after 4 weeks. Mice are sacrificed 4-18 weeks after grafting.

### B. INJECTION OF CELLS

Cells are directly injected into human skin using a technique similar to that described in Ortiz-Urda et al. (cited above). For injection of human mesenchymal cells into mouse skin, 6-8 week old female Cr:NIH(S)-nu/nu mice are injected intradermally with 10⁶ cells resuspended in 100 µl PBS using a 30-gauge needle. The injection is performed by first piercing the skin, then directing the needle back upward toward the surface and injecting the cells as superficially as possible. This leads to formation of a well-demarcated papule in the center of the injected area. Eight to 16 weeks after injection, biopsies and analyses are performed on the mouse skin.

### C. IMPLANTATION OF CELLS

After anesthetizing, small incisions approximately 0.5-1.0 mm in width and length are made using a 27-gauge needle. A single cultured aggregate (proto-hair) is inserted at a shallow position within each incision. Following insertion, incisions are left to heal.

After the animal or patient is anesthetized, full-thickness skin wounds (1.5 × 1.5 cm² rectangular shape) are created on the transplantation area. To minimize the migration of host skin cells from the wound margins and spontaneous wound contraction, the skin at the wound margins is burned using a cautery and fixed to adjacent muscle layers with nonresorbable 5-0 nylon sutures (AILEE Co., Pusan, Korea). Mesenchymal cells (approximately 10⁸ cells/wound) and keratinocytes (approximately 7.5 × 10⁶ cells/wound) cultured on PLGA microspheres are transplanted to the wounds using a 1-mL syringe without a needle. After transplantation, the wounds are dressed with dressing materials, Tegaderm (3M Health Care, St. Paul, MN) and sterile cotton gauze, and firmly fixed using Coban, a self-adhesive wrap (3M Health Care). For mice, an antibiotic (Cefazolin, 0.1 mg/mouse, Yuhan Co., Seoul, Korea) and an analgesic (Buprenorphine, 0.1 mg/kg, Hanlim Pharm Co., Seoul, Korea) are administered intramuscularly and subcutaneously, respectively, for 5 days after transplantation. The mice are housed singly after surgery and receive humane care in compliance with the guidelines for the care and use of laboratory animals of NIH.

### EXAMPLE 12: APPLICATION OF SKIN SUBSTITUTE TO A PATIENT WOUND

Patients exhibiting full- or partial-thickness skin loss, wounds, burns, scars, and full- or partial-hair loss are given a standard preoperative assessment to determine surgical risk. The site for application of the skin substitute should have a good blood supply, such as dermis, fascia, muscle, granulation tissue, periosteum, perichondrium, peritenon, and perineurium, but not cartilage, tendon, or nerve. The wound must be free of necrotic tissue. The wound should be relatively uncontaminated by bacteria, with bacterial counts of less than 100,000 per square centimeter. An adequate wound bed may require debridement, dressing changes, and systemic or topical antibiotics. Antimicrobial, antifungal, and antiviral agents, administered topically or systemically, may be used during a period of time (such as a week) prior to and following administration of the skin substitute to reduce the risk of infection. Wound vacuum-assisted closure may be used to improve wound bed characteristics prior to grafting, and may also be used after grafting.

The patient is anesthetized using local, regional, or general anesthesia, and the graft site is washed with water, an antibiotic wash, or an alcohol solution (such as an alcohol swab). The existing skin tissue, devitalized tissue, eschar, wound or ulcer edges, or scar tissue is removed using standard techniques in the art. Debridement may extend to healthy, viable, bleeding tissue. Prior to debridement thoroughly cleanse the wound with sterile saline to remove loose debris and necrotic tissue. Using tissue nippers, a surgical blade, or curette remove hyperkeratotic and/or necrotic tissue and debris from the wound surface. Ulcer margins may be debrided to have a saucer effect. After debridement, cleanse the wound thoroughly with sterile saline solution and gently dry with gauze. Oozing or bleeding resulting from debridement or revision of wound edges may be stopped through the use of gentle pressure. Other options include ligation of vessels, electrocautery, chemical cautery, or laser cautery, but these approaches may produce devitalized tissue and their use should be minimized. Heavy exudation may displace a skin substitute and reduce adherence. Exudation may be minimized by appropriate clinical treatment. For example, sterile air at room temperature or up to 42°C may be blown over the wound until the wound is sticky. If exudation persists, the skin substitute may be made permeable to exudate by perforating the skin substitute to allow for drainage.

Skin substitutes may be applied to a clean, debrided skin surface after thoroughly irrigating the wound with a non-cytotoxic solution. Before applying the skin substitute, the practitioner can review the expiration date of the skin substitute, check the pH, and visually observe and smell the skin substitute to ensure that there are no contaminants, such as bacterial contaminants or particulate matter. The skin substitute may be stored in a polyethylene bag at controlled temperature 68°F-73°F (20°C-23°C) until immediately prior to use. The practitioner may cut open the sealed polyethylene bag, and if the skin substitute is provided in a cell culture dish or plastic tray, it may be transferred to the sterile field with aseptic technique. If present, a tray or cell culture dish lid may be lifted off, and the practitioner may note the epidermal and dermal layer orientation of the skin substitute. Using a sterile atraumatic instrument, a practitioner may gently dislodge approximately 0.5 inch of the skin substitute away from the wall of the tray or cell culture dish. When lifting the skin substitute, a practitioner may be careful not to perforate or lift any membrane beneath the skin substitute, which, if present, should remain in the tray. With sterile gloved hands, a practitioner may insert one index finger under the released section of the skin substitute and use the other index finger to grasp the skin substitute in a second spot along the edge of the device. Holding the skin substitute in two places, the practitioner may lift the entire skin substitute out of the tray or cell culture dish using a smooth, even motion. If excessive folding occurs, the skin substitute can be floated (epidermal surface up) onto warm sterile saline solution in a sterile tray. The skin substitute may be placed so that the dermal layer (the glossy layer closest to the medium) is in direct contact with the site for the skin substitute. Using a saline moistened cotton applicator, the practitioner may smooth the skin substitute onto the site so there are no air bubbles or wrinkled edges. If the skin substitute is larger than the site for application, the excess skin substitute may be trimmed away to prevent it from adhering to the dressing. If the skin substitute is smaller than the site for application, multiple skin substitutes may be applied adjacent to each other until the defect is filled.

The skin substitute may be secured with any appropriate clinical dressing. It is preferable to use a nonadherent, semiocclusive, absorbent dressing material. It should provide uniform pressure over the entire grafted area. Sutures or staples are not required but may be used in some instances to anchor the graft to the graft bed (tacking sutures). Absorbable sutures, such as 5-0 fast absorbing gut, are preferable because they do not require removal. Dressings may be used to assure contact of the skin substitute to the site for application and to prevent movement. Therapeutic compression may be applied to the graft site. In some cases it may be necessary to immobilize the grafted limb to minimize shearing forces between the skin substitute and the application site. Bolster dressings are useful in areas where motion is difficult to avoid and in wounds with irregular contours. Dressings may be changed once a week or more frequently if necessary. Pain, odor, discharge, or other signs of complications are indications for dressing removal and inspection of the application site.

Additional applications of skin substitutes may be necessary in certain instances. Prior to additional applications, non-adherent remnants of a prior skin graft or skin substitute should be gently removed. Healing tissue or adherent skin substitutes may be left in place. The site may be cleansed with a non-cytotoxic solution prior to additional applications of skin substitute. In one embodiment, an additional skin substitute may be applied to the areas where the prior skin substitute is not adherent.

## Claims

1. A skin substitute comprising epithelial cells and modified mesenchymal cells, wherein, compared to wild type mesenchymal cells, the modified mesenchymal cells have:
(a) a decreased level, function, and/or activity of a TSC1/TSC2 complex; and/or
(b) an increased mTORC1 function, a decreased mTORC2 function, or both.

2. The skin substitute of claim 1, wherein the modified mesenchymal cells comprise:
(a) a downregulated TSC1;
(b) upregulation of at least one of Ras, Raf, Mek, Erk, Rsk1, PI3K, Akt1, Akt2, Akt3, Rheb, mTOR, Raptor, Rictor, mLST8, S6K1, ribosomal protein S6, SKAR, SREBP1, elF4e, IKKbeta, Myc, Runx1, or p27;
(c) downregulation of at least one of TSC1, TSC2, a TSC1/TSC2 complex, CYLD, LKB1, FLCN, MEN1, NF1, PTEN, PRAS40, 4E-BP1, GSK3, or Deptor;
(d) a transfected growth factor gene, wherein the growth factor is selected from insulin, EGF, HGF, IGF and KGF;
(e) a downregulated TSC2;
(f) a downregulated FLCN; or
(g) downregulated TSC2 and FLCN.

3. The skin substitute of claim 2, wherein the downregulation of at least one of TSC1, TSC2, the TSC1/TSC2 complex, CYLD, LKB1, FLCN, MEN1, NF1, PTEN, PRAS40, 4E-BP1, GSK3, or Deptor is accomplished by treating wild type mesenchymal cells with an siRNA, shRNA, or RNAi.

4. The skin substitute of claim 1, wherein
(a) the modified mesenchymal cells are from tumors associated with Birt-Hogg-Dube syndrome, Brooke-Spiegler syndrome, Cowden syndrome, multiple endocrine neoplasia type 1, neurofibromatosis, or tuberous sclerosis complex;
(b) the modified mesenchymal cells are from angiofibromas, fibrofolliculomas, fibrous papules, forehead plaques, hair follicle nevi, infundibulomas, isthmicomas, perifollicular fibromas, sebaceous nevi, organoid nevi, syringomas, shagreen patches, trichodiscomas, trichoepitheliomas, trichoblastomas, trichilemmomas, trichoadenomas, poromas, or ungual fibromas; or
(c) the wild type mesenchymal cells are dermal fibroblasts, dermal papilla cells, dermal sheath cells, induced pluripotent stem cells, or mesenchymal stem cells.

5. The skin substitute of claim 1, wherein the modified mesenchymal cells are wild type mesenchymal cells comprising an expression vector comprising a nucleic acid sequence encoding a protein that inhibits TSC1, TSC2, or the TSC1/TSC2 complex under the control of a constitutive promoter;
optionally wherein the wild type mesenchymal cells are dermal fibroblasts, dermal papilla cells, dermal sheath cells, induced pluripotent stem cells, or mesenchymal stem cells.

6. The skin substitute of claim 1, wherein the modified mesenchymal cells are provided with a matrix, optionally wherein the matrix is a collagen matrix, a ground substance matrix, or a type I collagen matrix.

7. The skin substitute of claim 1, wherein
the epithelial cells are from two different sources; or
the epithelial cells are keratinocytes or keratinocyte-like cells, optionally wherein the keratinocytes are neonatal foreskin keratinocytes.

8. The skin substitute of claim 1, for use in transplanting to a patient cells capable of inducing human hair follicles, optionally wherein the patient has partial-thickness skin loss, full-thickness skin loss, a wound, a burn, a scar, or hair loss.

9. The skin substitute for use according to claim 8, wherein at least one of the epithelial cells and the modified mesenchymal cells is derived from the patient; or
both the epithelial cells and the modified mesenchymal cells are derived from the patient.

10. The skin substitute for use according to claim 8, wherein the skin substitute induces eccrine glands, or the skin substitute induces sebaceous glands.

11. Modified mesenchymal cells, for use in treating a condition requiring growth of hair follicles, eccrine glands, and/or sebaceous glands, wherein the use comprises subdermally or intradermally delivering to a patient the modified mesenchymal cells, wherein, compared to wild type mesenchymal cells, the modified mesenchymal cells have:
(a) a decreased level, function, and/or activity of a TSC1/TSC2 complex; and/or
(b) an increased mTORC1 function, a decreased mTORC2 function, or both.

12. The modified mesenchymal cells for use according to claim 11, wherein the modified mesenchymal cells comprise
(a) a downregulated TSC1;
(b) downregulation of at least one of TSC1, TSC2, a TSC1/TSC2 complex, CYLD, LKB1, FLCN, MEN1, NF1, PTEN, PRAS40, 4E-BP1, GSK3, or Deptor;
(c) upregulation of at least one of Ras, Raf, Mek, Erk, Rsk1, PI3K, Akt1, Akt2, Akt3, Rheb, mTOR, Raptor, Rictor, mLST8, S6K1, ribosomal protein S6, SKAR, SREBP1, elF4e, IKKbeta, Myc, Runx1, or p27;
(d) a transfected growth factor gene, wherein the growth factor is selected from insulin, EGF, HGF, IGF and KGF;
(e) a downregulated TSC2;
(f) a downregulated FLCN; or
(g) downregulated TSC2 and FLCN.

13. The modified mesenchymal cells for use according to claim 11, wherein
the modified mesenchymal cells are delivered in a microsphere, optionally wherein the microsphere is formed by mixing about 30,000 cells each of neonatal foreskin fibroblasts and neonatal foreskin keratinocytes in a 1:1 mixture of dermal papilla medium and keratinocyte serum free medium, and incubating the clusters for about four weeks; or
the modified mesenchymal cells are delivered with a matrix, optionally wherein the matrix is a collagen matrix or a ground substance matrix, optionally wherein the matrix is a type I collagen matrix.

14. The modified mesenchymal cells for use according to claim 12, wherein the downregulation of at least one of TSC1, TSC2, a TSC1/TSC2 complex, CYLD, LKB1, FLCN, MEN1, NF1, PTEN, PRAS40, 4E-BP1, GSK3, or Deptor is accomplished by treating wild type mesenchymal cells with an siRNA, shRNA, or RNAi.

15. The modified mesenchymal cells for use according to claim 11, wherein
the modified mesenchymal cells are delivered with epithelial cells;
the epithelial cells are keratinocytes or keratinocyte-like cells, optionally wherein the keratinocytes are neonatal foreskin keratinocytes; or
the epithelial cells are from two different sources; optionally
wherein the epithelial cells and the modified mesenchymal cells are derived from the same donor, optionally wherein the donor is the patient; or
wherein the epithelial cells and the modified mesenchymal cells are derived from different donors, optionally wherein at least one donor is the patient.

16. The modified mesenchymal cells for use according to claim 11, wherein
(a) the modified mesenchymal cells are from tumors associated with Birt-Hogg-Dube syndrome, Brooke-Spiegler syndrome, Cowden syndrome, multiple endocrine neoplasia type 1, neurofibromatosis, or tuberous sclerosis complex;
(b) the modified mesenchymal cells are from angiofibromas, fibrofolliculomas, fibrous papules, forehead plaques, hair follicle nevi, infundibulomas, isthmicomas, perifollicular fibromas, sebaceous nevi, organoid nevi, syringomas, shagreen patches, trichodiscomas, trichoepitheliomas, trichoblastomas, trichilemmomas, trichoadenomas, poromas, or ungual fibromas; or
(c) the wild type mesenchymal cells are dermal fibroblasts, dermal papilla cells, dermal sheath cells, induced pluripotent stem cells, or mesenchymal stem cells.

17. The modified mesenchymal cells for use according to claim 11, wherein the modified mesenchymal cells are wild type mesenchymal cells comprising an expression vector comprising a gene encoding a protein that inhibits TSC1, TSC2, or the TSC1/TSC2 complex under the control of a constitutive promoter;
optionally wherein the wild type mesenchymal cells are dermal fibroblasts, dermal papilla cells, dermal sheath cells, induced pluripotent stem cells, or mesenchymal stem cells, optionally wherein the wild type mesenchymal cells are dermal fibroblasts.

18. The modified mesenchymal cells for use according to claim 11, wherein
the patient has partial-thickness skin loss, full-thickness skin loss, a wound, a burn, a scar, or hair loss;
the composition induces eccrine glands or the composition induces sebaceous glands; or
the mesenchymal cells are derived from the patient.

## Patentansprüche

1. Hautersatz, umfassend Epithelzellen und modifizierte Mesenchymzellen, wobei die modifizierten Mesenchymzellen im Vergleich zu Wildtyp-Mesenchymzellen Folgendes aufweisen:
(a) ein verringerter Spiegel, Funktion und/oder Aktivität eines TSC1/TSC2-Komplexes; und/oder
(b) eine erhöhte mTORC1-Funktion, eine verringerte mTORC2-Funktion oder beides.

2. Hautersatz nach Anspruch 1, wobei die modifizierten Mesenchymzellen umfassen:
(a) ein hinunterreguliertes TSC1;
(b) eine Hinaufregulierung von wenigstens einem aus Ras, Raf, Mek, Erk, Rsk1, PI3K, Akt1, Akt2, Akt3, Rheb, mTOR, Raptor, Rictor, mLST8, S6K1, ribosomalem Protein S6, SKAR, SREBP1, eIF4e, IKKbeta, Myc, Runx1 oder p27;
(c) eine Hinunterregulierung von wenigstens einem aus TSC1, TSC2, einem TSC1/TSC2-Komplex, CYLD, LKB1, FLCN, MEN1, NF1, PTEN, PRAS40, 4E-BP1, GSK3 oder Deptor;
(d) ein transfiziertes Wachstumsfaktorgen, wobei der Wachstumsfaktor aus Insulin, EGF, HGF, IGF und KGF ausgewählt ist;
(e) ein hinunterreguliertes TSC2;
(f) ein hinunterreguliertes FLCN; oder
(g) hinunterreguliertes TSC2 und FLCN.

3. Hautersatz nach Anspruch 2, wobei die Hinunterregulierung von wenigstens einem aus TSC1, TSC2, dem TSC1/TSC2-Komplex, CYLD, LKB1, FLCN, MEN1, NF1, PTEN, PRAS40, 4E-BP1, GSK3 oder Deptor erzielt wird, indem Wildtyp-Mesenchymzellen mit siRNA, shRNA oder RNAi behandelt werden.

4. Hautersatz nach Anspruch 1, wobei
(a) die modifizierten Mesenchymzellen von Tumoren stammen, die mit dem Birt-Hogg-Dube-Syndrom, dem Brooke-Spiegler-Syndrom, dem Cowden-Syndrom, multipler endokriner Neoplasie des Typs 1, Neurofibromatose oder dem Tuberöse-Sklerose-Komplex assoziiert sind;
(b) die modifizierten Mesenchymzellen von Angiofibromen, Fibrofollikulomen, fibrösen Papeln, Stirn-Plaques, Haarfollikel-Nävi, Infundibulomen, Isthmicomen, perifollikulären Fibromen, Talgdrüsen-Nävi, organoiden Nävi, Syringomen, Chagrin-Haut, Trichodiscomen, Trichoepitheliomen, Trichoblastomen, Trichilemmomen, Trichoadenomen, Poromen oder ungualen Fibromen stammen; oder
(c) die Wildtyp-Mesenchymzellen dermale Fibroblasten, dermale Papillenzellen, dermale Hüllzellen, induzierte pluripotente Stammzellen oder mesenchymale Stammzellen sind.

5. Hautersatz nach Anspruch 1, wobei die modifizierten Mesenchymzellen Wildtyp-Mesenchymzellen sind, die einen Expressionsvektor umfassen, der eine Nukleinsäuresequenz umfasst, die ein Protein codiert, das TSC1, TSC2 oder den TSC1/TSC2 Komplex unter der Kontrolle eines konstitutiven Promoters hemmt;
wobei die Wildtyp-Mesenchymzellen optional dermale Fibroblasten, dermale Papillenzellen, dermale Hüllzellen, induzierte pluripotente Stammzellen oder mesenchymale Stammzellen sind.

6. Hautersatz nach Anspruch 1, wobei die modifizierten Mesenchymzellen mit einer Matrix versehen sind, wobei die Matrix optional eine Collagen-Matrix, eine Grundsubstanz-Matrix oder eine Typ-I-Collagen-Matrix ist.

7. Hautersatz nach Anspruch 1, wobei
die Epithelzellen von zwei verschiedenen Quellen stammen; oder
die Epithelzellen Keratinozyten oder keratinozytenähnliche Zellen sind, wobei die Keratinozyten optional neonatale Vorhaut-Keratinozyten sind.

8. Hautersatz nach Anspruch 1 zur Verwendung bei der Transplantation auf einen Patienten von Zellen, die imstande sind, humane Haarfollikel zu induzieren, wobei der Patient optional Teildicken-Hautverlust, Volldicken-Hautverlust, eine Wunde, eine Verbrennung, eine Narbe oder Haarverlust aufweist.

9. Hautersatz zur Verwendung nach Anspruch 8, wobei wenigstens eines von den Epithelzellen und den modifizierten Mesenchymzellen von dem Patienten abgeleitet ist; oder
beide von den Epithelzellen und den modifizierten Mesenchymzellen von dem Patienten abgeleitet sind.

10. Hautersatz zur Verwendung nach Anspruch 8, wobei der Hautersatz ekkrine Drüsen induziert oder der Hautersatz Talgdrüsen induziert.

11. Modifizierte Mesenchymzellen zur Verwendung bei der Behandlung eines Zustands, der das Wachstum von Haarfollikeln, ekkrinen Drüsen und/oder Talgdrüsen erfordert, wobei die Verwendung die subdermale oder intradermale Abgabe der modifizierten Mesenchymzellen an einen Patienten umfasst, wobei die modifizierten Mesenchymzellen im Vergleich zu den Wildtyp-Mesenchymzellen Folgendes aufweisen:
(a) ein verringerter Spiegel, Funktion und/oder Aktivität eines TSC1/TSC2-Komplexes; und/oder
(b) eine erhöhte mTORC1-Funktion, eine verringerte mTORC2-Funktion oder beides.

12. Modifizierte Mesenchymzellen zur Verwendung nach Anspruch 11, wobei die modifizierten Mesenchymzellen umfassen:
(a) ein hinunterreguliertes TSC1;
(b) Hinunterregulierung von wenigstens einem aus TSC1, TSC2, einem TSC1/TSC2-Komplex, CYLD, LKB1, FLCN, MEN1, NF1, PTEN, PRAS40, 4E-BP1, GSK3 oder Deptor;
(c) eine Hinaufregulierung von wenigstens einem aus Ras, Raf, Mek, Erk, Rsk1, PI3K, Akt1, Akt2, Akt3, Rheb, mTOR, Raptor, Rictor, mLST8, S6K1, ribosomalem Protein S6, SKAR, SREBP1, eIF4e, IKKbeta, Myc, Runx1 oder p27;
(d) ein transfiziertes Wachstumsfaktorgen, wobei der Wachstumsfaktor aus Insulin, EGF, HGF, IGF und KGF ausgewählt ist;
(e) ein hinunterreguliertes TSC2;
(f) ein hinunterreguliertes FLCN; oder
(g) hinunterreguliertes TSC2 und FLCN.

13. Modifizierte Mesenchymzellen zur Verwendung nach Anspruch 11, wobei
die modifizierten Mesenchymzellen in einem Mikrokügelchen abgegeben werden, wobei das Mikrokügelchen optional gebildet wird, indem etwa 30.000 Zellen von jedem von neonatalen Vorhaut-Fibroblasten und neonatalen Vorhaut-Keratinozyten in einem 1:1-Gemisch aus Medium für dermale Papillen und serumfreiem Medium für Keratinozyten gemischt werden und die Verbände während etwa vier Wochen inkubiert werden; oder
die modifizierten Mesenchymzellen mit einer Matrix abgegeben werden, wobei die Matrix optional eine Collagen-Matrix oder eine Grundsubstanz-Matrix ist, wobei die Matrix optional eine Typ-I-Collagen-Matrix ist.

14. Modifizierte Mesenchymzellen zur Verwendung nach Anspruch 12, wobei die Hinunterregulierung von wenigstens einem aus TSC1, TSC2, einem TSC1/TSC2-Komplex, CYLD, LKB1, FLCN, MEN1, NF1, PTEN, PRAS40, 4E-BP1, GSK3 oder Deptor erzielt wird, indem Wildtyp-Mesenchymzellen mit siRNA, shRNA oder RNAi behandelt werden.

15. Modifizierte Mesenchymzellen zur Verwendung nach Anspruch 11, wobei
die modifizierten Mesenchymzellen mit Epithelzellen abgegeben werden;
die Epithelzellen Keratinozyten oder keratinozytenähnliche Zellen sind, wobei die Keratinozyten optional neonatale Vorhaut-Keratinozyten sind; oder
die Epithelzellen von zwei verschiedenen Quellen stammen; wobei optional
die Epithelzellen und die modifizierten Mesenchymzellen von demselben Spender abgeleitet sind, wobei optional der Spender der Patienten ist; oder
die Epithelzellen und die modifizierten Mesenchymzellen von verschiedenen Spendern abgeleitet sind, wobei optional wenigstens ein Spender der Patient ist.

16. Modifizierte Mesenchymzellen zur Verwendung nach Anspruch 11, wobei
(a) die modifizierten Mesenchymzellen von Tumoren stammen, die mit dem Birt-Hogg-Dube-Syndrom, dem Brooke-Spiegler-Syndrom, dem Cowden-Syndrom, multipler endokriner Neoplasie des Typs 1, Neurofibromatose oder dem Tuberöse-Sklerose-Komplex assoziiert sind;
(b) die modifizierten Mesenchymzellen von Angiofibromen, Fibrofollikulomen, fibrösen Papeln, Stirn-Plaques, Haarfollikel-Nävi, Infundibulomen, Isthmicomen, perifollikulären Fibromen, Talgdrüsen-Nävi, organoiden Nävi, Syringomen, Chagrin-Haut, Trichodiscomen, Trichoepitheliomen, Trichoblastomen, Trichilemmomen, Trichoadenomen, Poromen oder ungualen Fibromen stammen; oder
(c) die Wildtyp-Mesenchymzellen dermale Fibroblasten, dermale Papillenzellen, dermale Hüllzellen, induzierte pluripotente Stammzellen oder mesenchymale Stammzellen sind.

17. Modifizierte Mesenchymzellen zur Verwendung nach Anspruch 11, wobei die modifizierten Mesenchymzellen Wildtyp-Mesenchymzellen sind, die einen Expressionsvektor umfassen, der ein Gen umfasst, das ein Protein codiert, das TSC1, TSC2 oder den TSC1/TSC2 Komplex unter der Kontrolle eines konstitutiven Promoters hemmt;
wobei die Wildtyp-Mesenchymzellen optional dermale Fibroblasten, dermale Papillenzellen, dermale Hüllzellen, induzierte pluripotente Stammzellen oder mesenchymale Stammzellen sind, wobei die Wildtyp-Mesenchymzellen optional dermale Fibroblasten sind.

18. Modifizierte Mesenchymzellen zur Verwendung nach Anspruch 11, wobei
der Patient Teildicken-Hautverlust, Volldicken-Hautverlust, eine Wunde, eine Verbrennung, eine Narbe oder Haarverlust aufweist;
die Zusammensetzung ekkrine Drüsen induziert oder die Zusammensetzung Talgdrüsen induziert; oder
die Mesenchymzellen von dem Patienten abgeleitet sind.

## Revendications

1. Substitut de peau comprenant des cellules épithéliales et des cellules mésenchymateuses modifiées, dans lequel, comparé à des cellules mésenchymateuses de type sauvage, les cellules mésenchymateuses modifiées ont :
(a) un niveau, une fonction, et/ou une activité diminués d'un complexe TSC1/TCS2 ; et/ou
(b) une fonction mTORC1 accrue, une fonction mTORC2 diminuée, ou les deux.

2. Substitut de peau selon la revendication 1, dans lequel les cellules mésenchymateuses modifiées comprennent :
(a) un TSC1 régulé à la baisse ;
(b) une régulation à la hausse d'au moins l'un de Ras, Raf, Mek, Erk, Rsk1, PI3K, Akt1, Akt2, Akt3, Rheb, mTOR, Raptor, Rictor, mLST8, S6K1, la protéine ribosomale S6, SKAR, SREBP1, eIF4e, IKKbêta, Myc, Runx1, ou p27 ;
(c) la régulation à la baisse d'au moins l'un de TSC1, TSC2, un complexe TSC1/TSC2, CYLD, LKB1, FLCN, MEN1, NF1, PTEN, PRAS40, 4E-BP1, GSK3, ou Deptor ;
(d) un gène de facteur de croissance transfecté, dans lequel le facteur de croissance est sélectionné parmi l'insuline, l'EGF, le HGF, l'IGF et le KGF ;
(e) un TSC2 régulé à la baisse ;
(f) un FLCN régulé à la baisse ; ou
(g) TSC2 et FLCN régulés à la baisse.

3. Substitut de peau selon la revendication 2, dans lequel la régulation à la baisse d'au moins l'un de TSC1, TSC2, du complexe TSC1/TSC2, CYLD, LKB1, FLCN, MEN1, NF1, PTEN, PRAS40, 4E-BP1, GSK3, ou Deptor est réalisée par le traitement de cellules mésenchymateuses de type sauvage avec un ARNsi, un ARNsh, ou un ARNi.

4. Substitut de peau selon la revendication 1, dans lequel
(a) les cellules mésenchymateuses modifiées proviennent de tumeurs associées au syndrome de Birt-Hogg-Dube, au syndrome de Brooke-Spiegler, au syndrome de Cowden, à une néoplasie endocrinienne multiple de type 1, à la neurofibromatose, ou à la sclérose tubéreuse complexe ;
(b) les cellules mésenchymateuses modifiées proviennent d'angiofibromes, de fibrofolliculomes, de papules fibreuses, de plaques sur le front, de naevi du follicule pileux, d'infundibulomes, de tumeurs de l'infundibulum folliculaire, de fibromes périfolliculaires, de naevi sébacés, de naevi organoïdes, de syringomes, de plaques peau de chagrin, de trichodiscomes, de trichoépithéliomes, de trichoblastomes, de trichilemmomes, de trichoadénomas, de poromes, ou de fibromes unguéaux ; ou
(c) les cellules mésenchymateuses de type sauvage sont des fibroblastes dermiques, des cellules papillaires dermiques, des cellules de gaine dermique, des cellules souches pluripotentes induites, ou des cellules souches mésenchymateuses.

5. Substitut de peau selon la revendication 1, dans lequel les cellules mésenchymateuses modifiées sont des cellules mésenchymateuses de type sauvage comprenant un vecteur d'expression comprenant une séquence d'acide nucléique codant pour une protéine qui inhibe TSC1, TSC2, ou le complexe TSC1/TSC2 sous le contrôle d'un promoteur constitutif ;
facultativement dans lequel les cellules mésenchymateuses de type sauvage sont des fibroblastes dermiques, des cellules papillaires dermiques, des cellules de gaine dermique, des cellules souches pluripotentes induites, ou des cellules souches mésenchymateuses.

6. Substitut de peau selon la revendication 1, dans lequel les cellules mésenchymateuses modifiées sont dotées d'une matrice, facultativement dans lequel la matrice est une matrice de collagène, une matrice de substance broyée, ou une matrice de collagène de type I.

7. Substitut de peau selon la revendication 1, dans lequel
les cellules épithéliales proviennent de deux sources différentes ; ou
les cellules épithéliales sont des kératinocytes ou des cellules de type kératinocytes, facultativement dans lequel les kératinocytes sont des kératinocytes de prépuce de nouveau-né.

8. Substitut de peau selon la revendication 1, pour son utilisation dans la transplantation à des cellules d'un patient capables d'induire des follicules pileux humains, facultativement dans lequel le patient souffre d'une perte de peau d'épaisseur partielle, d'une perte de peau d'épaisseur totale, d'une plaie, d'une brûlure, d'une cicatrice, ou d'une perte de cheveux.

9. Substitut de peau pour son utilisation selon la revendication 8, dans lequel au moins les unes des cellules épithéliales et des cellules mésenchymateuses modifiées proviennent du patient ; ou
les cellules épithéliales et les cellules mésenchymateuses modifiées proviennent du patient.

10. Substitut de peau pour son utilisation selon la revendication 8, dans lequel le substitut de peau induit des glandes eccrines, ou le substitut de peau induit des glandes sébacées.

11. Cellules mésenchymateuses modifiées, pour leur utilisation dans le traitement d'une affection nécessitant la croissance des follicules pileux, des glandes eccrines, et/ou des glandes sébacées, dans lesquelles l'utilisation comprend l'administration sous-dermique ou intradermique des cellules mésenchymateuses modifiées à un patient, dans lesquelles, comparé à des cellules mésenchymateuses de type sauvage, les cellules mésenchymateuses modifiées ont :
(a) un niveau, une fonction, et/ou une activité diminués d'un complexe TSC1/TCS2 ; et/ou
(b) une fonction mTORC1 accrue, une fonction mTORC2 diminuée, ou les deux.

12. Cellules mésenchymateuses modifiées pour leur utilisation selon la revendication 11, dans lesquelles les cellules mésenchymateuses modifiées comprennent :
(a) un TSC1 régulé à la baisse ;
(b) la régulation à la baisse d'au moins l'un de TSC1, TSC2, un complexe TSC1/TSC2, CYLD, LKB1, FLCN, MEN1, NF1, PTEN, PRAS40, 4E-BP1, GSK3, ou Deptor ;
(c) la régulation à la hausse d'au moins l'un de Ras, Raf, Mek, Erk, Rsk1, PI3K, Akt1, Akt2, Akt3, Rheb, mTOR, Raptor, Rictor, mLST8, S6K1, la protéine ribosomale S6, SKAR, SREBP1, eIF4e, IKKbêta, Myc, Runx1, ou p27 ;
(d) un gène de facteur de croissance transfecté, dans lesquelles le facteur de croissance est sélectionné parmi l'insuline, l'EGF, le HGF, l'IGF et le KGF ;
(e) un TSC2 régulé à la baisse ;
(f) un FLCN régulé à la baisse ; ou
(g) TSC2 et FLCN régulés à la baisse.

13. Cellules mésenchymateuses modifiées pour leur utilisation selon la revendication 11, dans lesquelles
les cellules mésenchymateuses modifiées sont administrées dans une microsphère, facultativement dans lesquelles la microsphère est formée par mélange d'environ 30 000 cellules de chacun de fibroblastes de prépuce de nouveau-né et de kératinocytes de prépuce de nouveau-né en un mélange 1/1 de milieu pour papilles dermiques et de milieu pour kératinocytes dépourvu de sérum, et incubation des agrégats pendant environ quatre semaines ; ou
les cellules mésenchymateuses modifiées sont administrées avec une matrice, facultativement dans lesquelles la matrice est une matrice de collagène ou une matrice de substance broyée, facultativement, dans lesquelles la matrice est une matrice de collagène de type I.

14. Cellules mésenchymateuses modifiées pour leur utilisation selon la revendication 12, dans lesquelles la régulation à la baisse d'au moins l'un de TSC1, TSC2, un complexe TSC1/TSC2, CYLD, LKB1, FLCN, MEN1, NF1, PTEN, PRAS40, 4E-BP1, GSK3, ou Deptor est réalisée par le traitement de cellules mésenchymateuses de type sauvage avec un ARNsi, un ARNsh, ou un ARNi.

15. Cellules mésenchymateuses modifiées pour leur utilisation selon la revendication 11, dans lesquelles
les cellules mésenchymateuses modifiées sont administrées avec des cellules épithéliales ;
les cellules épithéliales sont des kératinocytes ou des cellules de type kératinocytes, facultativement dans lesquelles les kératinocytes sont des kératinocytes de prépuce de nouveau-né ; ou
les cellules épithéliales proviennent de deux sources différentes ; facultativement
dans lesquelles les cellules épithéliales et les cellules mésenchymateuses modifiées proviennent du même donneur, facultativement dans lesquelles le donneur est le patient ; ou
dans lesquelles les cellules épithéliales et les cellules mésenchymateuses modifiées proviennent de donneurs différents, facultativement dans lesquelles au moins un donneur est le patient.

16. Cellules mésenchymateuses modifiées pour leur utilisation selon la revendication 11, dans lesquelles
(a) les cellules mésenchymateuses modifiées proviennent de tumeurs associées au syndrome de Birt-Hogg-Dube, au syndrome de Brooke-Spiegler, au syndrome de Cowden, à une néoplasie endocrinienne multiple de type 1, à la neurofibromatose, ou à la sclérose tubéreuse complexe ;
(b) les cellules mésenchymateuses modifiées proviennent d'angiofibromes, de fibrofolliculomes, de papules fibreuses, de plaques sur le front, de naevi du follicule pileux, d'infundibulomes, de tumeurs de l'infundibulum folliculaire, de fibromes périfolliculaires, de naevi sébacés, de naevi organoïdes, de syringomes, de plaques peau de chagrin, de trichodiscomes, de trichoépithéliomes, de trichoblastomes, de trichilemmomes, de trichoadénomas, de poromes, ou de fibromes unguéaux ; ou
(c) les cellules mésenchymateuses de type sauvage sont des fibroblastes dermiques, des cellules papillaires dermiques, des cellules de gaine dermique, des cellules souches pluripotentes induites, ou des cellules souches mésenchymateuses.

17. Cellules mésenchymateuses modifiées pour leur utilisation selon la revendication 11, dans lesquelles
les cellules mésenchymateuses modifiées sont des cellules mésenchymateuses de type sauvage comprenant un vecteur d'expression comprenant un gène codant pour une protéine qui inhibe TSC1, TSC2, ou le complexe TSC1/TSC2 sous le contrôle d'un promoteur constitutif ;
facultativement dans lesquelles les cellules mésenchymateuses de type sauvage sont des fibroblastes dermiques, des cellules papillaires dermiques, des cellules de gaine dermique, des cellules souches pluripotentes induites, ou des cellules souches mésenchymateuses, facultativement dans lesquelles les cellules mésenchymateuses de type sauvage sont des fibroblastes dermiques.

18. Cellules mésenchymateuses modifiées pour leur utilisation selon la revendication 11, dans lesquelles
le patient souffre d'une perte de peau d'épaisseur partielle, d'une perte de peau d'épaisseur totale, d'une plaie, d'une brûlure, d'une cicatrice, ou de perte de cheveux ;
la composition induit des glandes eccrines ou la composition induit des glandes sébacées ; ou
les cellules mésenchymateuses proviennent du patient.
